(19) Europäisches Patentamt
European Patent Office
Office européen des brevets

(11) EP 1 150 970 B1

(12) # FASCICULE DE BREVET EUROPEEN

(45) Date de publication et mention de la délivrance du brevet:
**22.08.2007 Bulletin 2007/34**

(21) Numéro de dépôt: **00903744.1**

(22) Date de dépôt: **08.02.2000**

(51) Int Cl.:
***C07D 401/06*** *(2006.01)* ***A61K 31/445*** *(2006.01)*
***A61P 25/00*** *(2006.01)*

(86) Numéro de dépôt international:
**PCT/FR2000/000284**

(87) Numéro de publication internationale:
**WO 2000/047572 (17.08.2000 Gazette 2000/33)**

(54) **DERIVES DE (1-PHENACY-3-PHENYL-3-PIPERIDYLETHYL)PIPERIDINE, PROCEDE POUR LEUR OBTENTION ET COMPOSITIONS PHARMACEUTIQUES LES CONTENANT**

(1-PHENACYL-3-PHENYL-3-PIPERIDYLETHYL)PIPERIDINDERIVATE, VERFAHREN ZU DEREN HERSTELLUNG UND SIE ENTHALTENDE PHARMAZEUTISCHE ZUSAMMENSETZUNGEN

(1-PHENACY-3-PHENYL-3-PIPERIDYLETHYL)PIPERIDINE DERIVATIVES, METHOD FOR THE PRODUCTION THEREOF AND PHARMACEUTICAL COMPOSITIONS CONTAINING THE SAME

(84) Etats contractants désignés:
**AT BE CH CY DE DK ES FI FR GB GR IE IT LI LU MC NL PT SE**
Etats d'extension désignés:
**AL LT LV MK RO SI**

(30) Priorité: **10.02.1999 FR 9901593**
**07.04.1999 FR 9904429**

(43) Date de publication de la demande:
**07.11.2001 Bulletin 2001/45**

(73) Titulaire: **Sanofi-Aventis**
**75013 Paris (FR)**

(72) Inventeurs:
- **DUCOUX, Jean, Philippe**
**F-34090 Montpellier (FR)**
- **EMONDS-ALT, Xavier**
**F-34980 Combaillaux (FR)**
- **GUEULE, Patrick**
**F-34820 Teyran (FR)**
- **PROIETTO, Vincenzo**
**F-34680 Saint-Georges-d'Orques (FR)**

(74) Mandataire: **Ludwig, Jacques et al**
**Sanofi-Aventis**
**174 avenue de France**
**75013 Paris (FR)**

(56) Documents cités:
**EP-A- 0 512 901** **EP-A- 0 714 891**
**WO-A-96/06094**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 1 150 970 B1

## Description

La présente invention a pour objet de nouveaux dérivés de pipéridine, un procédé pour leur préparation et les compositions pharmaceutiques en contenant en tant que principe actif.

Plus particulièrement, la présente invention concerne de nouveaux dérivés de pipéridine à usage thérapeutique dans les phénomènes pathologiques qui impliquent le système des tachykinines comme par exemple de manière non limitative : la douleur (L. Urban et al., TINS, 1994, 17, 432-438 ; L. Seguin et al., Pain, 1995, 61, 325-343 ; S. H. Buck, 1994, The Tachykinin Receptors, Humana Press, Totowa, New-Jersey), l'allergie et l'inflammation (S. H. Buck, 1994, The Tachykinin Receptors, Humana Press, Totowa, New-Jersey), les troubles gastro-intestinaux (P. Holzer and U. Holzer-Petsche, Phamacol. Ther., 1997, 73, 173-217 et 219-263), les troubles respiratoires (J. Mizrahi et al., Pharmacology, 1982, 25, 39-50 ; C. Advenier et al., Eur. Respir. J., 1997, 10, 1892-1906 ; C. Advenier and X. Emonds-Alt, Pulmonary Pharmacol., 1996, 9, 329-333), les troubles urinaires (S. H. Buck, 1994, The Tachykinin Receptors, Humana Press, Totowa, New-Jersey ; C. A. Maggi, Progress in Neurobiology, 1995, 45, 1-98), les troubles neurologiques, les troubles neuropsychiatriques (C. A. Maggi et al., J. Autonomic Pharmacol., 1993, 13, 23-93 ; M. Otsuka and K. Yoshioka, Physiol. Rev. 1993, 73, 229-308).

Dans les années récentes de nombreux travaux de recherche ont été effectués sur les tachykinines et leurs récepteurs. Les tachykinines sont distribuées à la fois dans le système nerveux central et dans le système nerveux périphérique. Les récepteurs aux tachykinines ont été reconnus et sont classés en trois types : $NK_1$, $NK_2$, $NK_3$. La substance P (SP) est le ligand endogène des récepteurs $NK_1$, la neurokinine A ($NK_A$) celui des récepteurs $NK_2$ et la neurokinine B ($NK_B$), celui des récepteurs $NK_3$.

Les récepteurs $NK_1$, $NK_2$, $NK_3$ ont été mis en évidence chez différentes espèces.

Une revue de C. A. Maggi et al. (J. Autonomic Pharmacol., 1993, 13, 23-93) et une revue de D. Regoli et al. (Pharmacol. Rev., 1994, 46, 551-599) font le point sur les récepteurs aux tachykinines et leurs antagonistes et exposent les études pharmacologiques et les applications en thérapeutique humaine.

De nombreux brevets ou demandes de brevet décrivent des composés actifs sur les récepteurs de tachykinines. Ainsi la demande de brevet européenne 0 512 901 concerne les composés de formule :

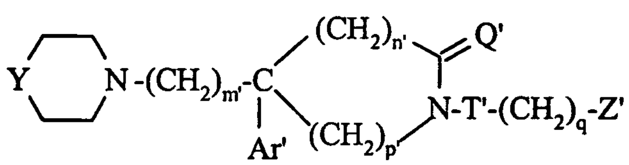

(A)

dans laquelle notamment :

- Q' représente un atome d'oxygène ou deux atomes d'hydrogène,
- T' = -C(O)- ou $-CH_2-$, et
- Y, Ar', Z', m', n', p' et q ont différentes valeurs.

La demande de brevet EP-0 714 891 concerne les composés de formule :

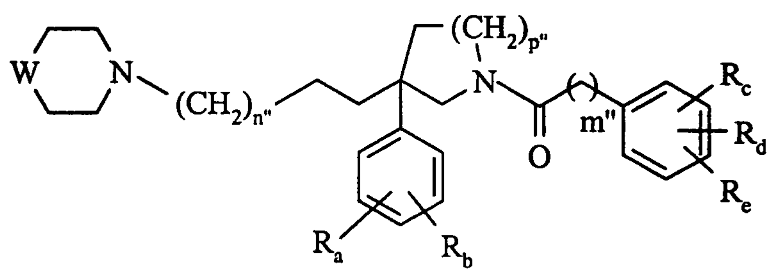

(B)

dans laquelle :

- p" est 1, 2 ou 3 ;
- m" et n" sont indépendamment 0 à 6 ;
- W, $R_a$, $R_b$, $R_c$, $R_d$ et $R_e$ ont différentes valeurs.

On a maintenant trouvé de nouveaux composés qui présentent une très forte affinité et une grande sélectivité pour les récepteurs $NK_1$ humains de la substance P et qui sont des antagonistes desdits récepteurs.

De plus, les composés selon la présente invention présentent une bonne biodisponibilité lorsqu'ils sont administrés par la voie orale.

Ces composés peuvent être utilisés pour la préparation de médicaments utiles dans le traitement de toute pathologie où la substance P et les récepteurs $NK_1$ sont impliqués, notamment dans le traitement des pathologies des systèmes respiratoire, gastro-intestinal, urinaire, immunitaire, cardiovasculaire, nerveux central ainsi que dans le traitement de la douleur, de la migraine, des inflammations, des nausées et des vomissements, des maladies de la peau.

Ainsi, selon un de ses aspects, la présente invention a pour objet des composés de formule :

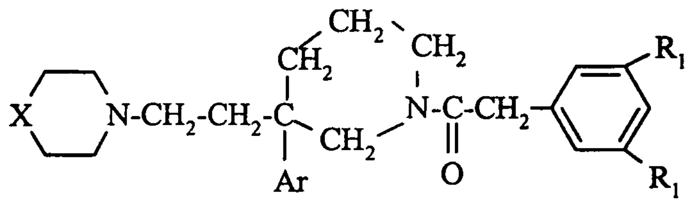

(I)

dans laquelle :

- Ar représente un phényle monosubstitué ou disubstitué par un atome d'halogène ; un $(C_1-C_3)$alkyle ;
- X représente un groupe

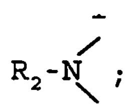

; un groupe

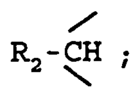

- $R_1$ représente un atome de chlore, un atome de brome, un $(C_1-C_3)$alkyle ou un trifluorométhyle ;
- $R_2$ représente un groupe $-CR_3R_4CONR_5R_6$ ;
- $R_3$ et $R_4$ représentent le même radical choisi parmi un méthyle, un éthyle, un n-propyle ou un n-butyle ;
- ou bien $R_3$ et $R_4$ ensemble avec l'atome de carbone auquel ils sont liés constituent un $(C_3-C_6)$cycloalkyle;
- $R_5$ et $R_6$ représentent chacun indépendamment un hydrogène ; un $(C_1-C_3)$alkyle ;
- ou bien $R_5$ et $R_6$ ensemble avec l'atome d'azote auquel ils sont liés constituent un radical hétérocyclique choisi parmi l'azétidin-1-yle, la pyrrolidin-1-yle, la pipéridin-1-yle, la morpholin-4-yle, la thiomorpholin-4-yle ou la perhydroazépin-1-yle ;

  ainsi que leurs sels éventuels avec des acides minéraux

ou organiques, leurs solvats et/ou leurs hydrates.

Les composés de formule (I) selon l'invention comprennent aussi bien les isomères optiquement purs que leurs mélanges en proportions quelconques.

On peut former des sels des composés de formule (I). Ces sels comprennent aussi bien ceux avec des acides minéraux ou organiques qui permettent une séparation ou une cristallisation convenable des composés de formule (I), tels que l'acide picrique ou l'acide oxalique ou un acide optiquement actif, par exemple un acide mandélique ou camphosulfonique, que ceux qui forment des sels pharmaceutiquement acceptables, tels que le chlorhydrate, le bromhydrate, le sulfate, l'hydrogénosulfate, le dihydrogénophosphate, le méthanesulfonate, le méthylsulfate, l'oxalate, le maléate, le fumarate, le succinate, le naphtalène-2 sulfonate, le gluconate, le citrate, le benzènesulfonate, le paratoluènesulfonate.

Par halogène, on entend un atome de chlore, de brome, de fluor ou d'iode.

Dans la présente description les groupes alkyles sont droits ou ramifiés.

Selon la présente invention, on préfère les composés de formule :

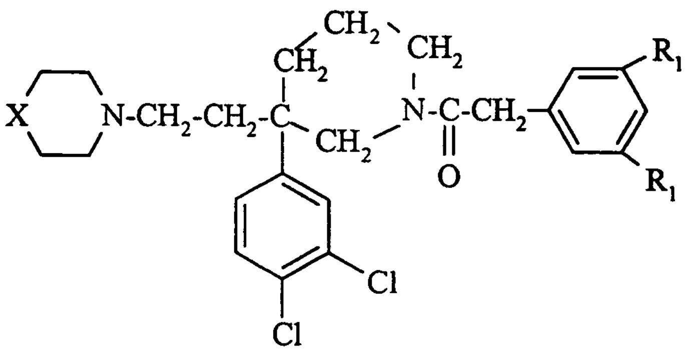

(Ia)

dans laquelle X et $R_1$ sont tels que définis pour un composé de formule (I), ainsi que leurs sels avec des acides minéraux ou organiques, leurs solvats et/ou leurs hydrates.

Selon la présente invention, on préfère les composés de formule (I) dans laquelle Ar représente un 3,4-dichlorophényle ou un 3,4-diméthylphényle.

Selon la présente invention, on préfère les composés de formule (I) dans laquelle les substituants $R_1$ représentent un atome de chlore, un méthyle, un éthyle ou un trifluorométhyle.

Selon la présente invention, on préfère les composés de formule (I) dans laquelle X représente un groupe

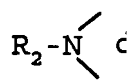

dans lequel $R_2$ représente un groupe $-CR_3R_4CONR_5R_6$.

Particulièrement, on préfère les composés dans lesquels $R_3$ et $R_4$ représentent chacun un méthyle ou bien ensemble avec l'atome de carbone auquel ils sont liés, constituent un cyclohexyle. Particulièrement, on préfère également les composés dans lesquels $R_5$ et $R_6$ représentent chacun l'hydrogène ou un méthyle.

Selon la présente invention, on préfère les composés de formule (I) dans laquelle X représente un groupe

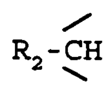

dans lequel $R_2$ représente un groupe $-CR_3R_4CONR_5R_6$. Particulièrement, on préfére les composés dans lesquels $R_3$ et $R_4$ représentent chacun un méthyle ou bien, ensemble avec l'atome de carbone auquel ils sont liés, constituent un cyclopropyle ou un cyclohexyle. Particulièrement, on préfère également les composés dans lesquels $R_5$ et $R_6$ représentent chacun l'hydrogène ou un méthyle.

Selon la présente invention, on préfère les composés de formule :

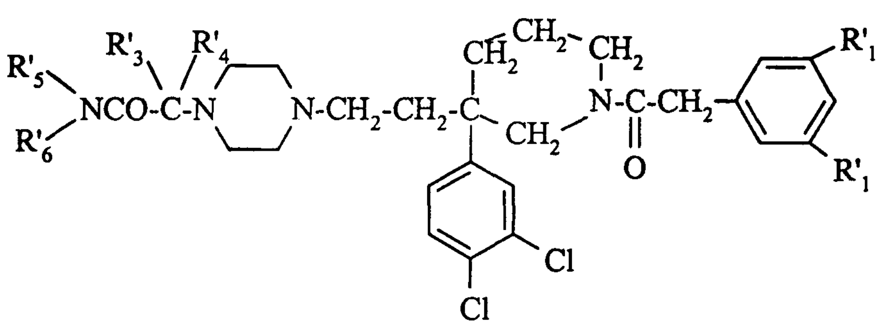

(I')

dans laquelle :

- $R'_1$ représente un atome de chlore, un méthyle, un éthyle ou un trifluorométhyle ;
- $R'_3$ et $R'_4$ représentent chacun un méthyle ou bien, ensemble avec l'atome de carbone auquel ils sont liés constituent un cyclohexyle ;
- $R'_5$ et $R'_6$ représentent chacun l'hydrogène ou un méthyle ;

ainsi que leurs sels avec des acides minéraux ou organiques, leurs solvats et/ou leurs hydrates.

Selon la présente invention, on préfère les composés de formule :

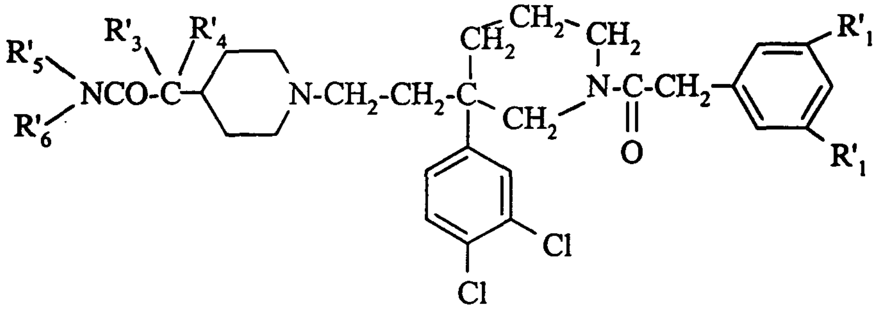

(I")

dans laquelle :

- $R'_1$ représente un atome de chlore, un méthyle, un éthyle ou un trifluorométhyle ;
- $R'_3$ et $R'_4$ représentent chacun un méthyle ou bien, ensemble avec l'atome de carbone auquel ils sont liés, constituent un cyclohexyle ou un cyclopropyle ;
- $R'_5$ et $R'_6$ représentent chacun l'hydrogène ou un méthyle ; ainsi que leurs sels avec des acides minéraux ou organiques, leurs solvats et/ou leurs hydrates.

Selon la présente invention, on préfère les composés de formule (I), (I'), (I") sous forme optiquement pure.

Les composés suivants :

- la 3-[2-[4-(1-carbamoyl-1-méthyléthyl)pipéridin-1-yl]éthyl]-3-(3,4-dichlorophényl)-1-[2-(3,5-diméthylphényl)acétyl] pipéridine, isomère (-) ;
- la 3- [2- [4- (1-carbamoyl-1-méthyléthyl)pipérazin-1-yl]éthyl]-3-(3,4-dichlorophényl)-1-[2-(3,5-diméthylphényl)acétyl] pipéridine, isomère (-) ;
- la 3- [2- [4- (1-N,N-diméthylcarbamoyl-1-méthyléthyl)pipérazin-1-yl]éthyl]-3-(3,4-dichlorophényl)-1-[2-(3,5-diméthylphényl)acétyl]pipéridine, isomère (-) ;
- la 3- [2- [4- (1-carbamoyl-1-méthyléthyl) pipéridin-1-yl]éthyl]-3-(3,4-dichlorophényl)-1-[2-(3,5-dichlorophényl)acétyl] pipéridine, isomère (-) ;
- la 3-[2-[4-(1-carbamoyl-1-méthyléthyl)pipéridin-1-yl]éthyl]-3-(3,4-dichlorophényl)-1-[2-[3,5-bis(trifluorométhyl)phényl]acétyl]pipéridine, isomère (+) ;
- la 3-[2-[4-(1-carbamoylcyclohexyl)pipéridin-1-yl]éthyl]-3-(3,4-dichlorophényl)-l-[2-(3,5-diméthylphényl)acétyl]pipéridine, isomère (-) ;
- la 3-[2-[4-(1-carbamoylcyclohexyl)pipérazin-1-yl]éthyl]-3-(3,4-dichlorophényl)-1-[2-(3,5-diméthylphényl)acétyl]pipéridine, isomère (-) ;
- la 3-[2-[4-(1-carbamoylcyclohexyl)pipéridin-1-yl]éthyl]-3-(3,4-dichlorophényl)-1-[2-(3,5-dichlorophényl)acétyl]pipéridine, isomère (-) ;
- la 3-[2-[4-(1-carbamoyl-1-méthyléthyl)pipérazin-1-yl]éthyl]-3-(3,4-dichlorophényl)-1-[2-(3,5-dichlorophényl)acétyl] pipéridine, isomère (+) ;
- la 3-[2-[4-(1-N,N-diméthylcarbamoyl-1-méthyléthyl)pipérazin-1-yl]éthyl]-3-(3,4-dichlorophényl) -1- [2- (3,5-dichlorophényl)acétyl]pipéridine, isomère (+) ;
- la 3-[2-[4-(1-carbamoylcyclohexyl)pipérazin-1-yl]éthyl]-3-(3,4-dichlorophényl)-1-[2-(3,5-dichlorophényl)acétyl]pipéridine, isomère (+) ;
- la 3-[2-[4-(1-carbamoylcyclohexyl)pipéridin-1-yl]éthyl]-3-(3,4-dichlorophényl)-1-[2-[3,5-bis(trifluorométhyl)phényl] acétyl]pipéridine, isomère (+) ;
- la 3-[2-[4-(1-carbamoylcyclohexyl)pipérazin-1-yl]éthyl]-3-(3,4-dichlorophényl)-1-[2-[3,5-bis(trifluorométhyl)phényl] acétyl]pipéridine, isomère (+) ;
- la 3-[2-[4-(1-N,N-diméthylcarbamoyl-1-méthyléthyl)pipéridin-1-yl]éthyl]-3-(3,4-dichlorophényl)-1-[2-(3,5-diméthylphényl)acétyl]pipéridine, isomère (-) ;

- la 3-[2-[4-(1-carbamoyl-1-méthyléthyl)pipéridin-1-yl]éthyl]-3-(3,4-dichlorophényl)-1-[2-(3,5-diéthylphényl)acétyl]pipéridine, isomère (-) ;
- la 3-[2-[4-(1-carbamoylcyclopropyl)pipéridin-1-yl]éthyl]-3-(3,4-dichlorophényl)-1-[2-(3,5-diméthylphényl)acétyl]pipéridine, isomère (-) ;
- la 3-[2-[4-(1-carbamoylcyclopropyl)pipéridin-1-yl]éthyl]-3-(3,4-dichlorophényl)-1-[2-[3,5-bis(trifluorométhyl)phényl] acétyl]pipéridine, isomère (+) ;
- la 3- [2- [4- (1-carbamoylcyclopropyl) pipéridin-1-yl]éthyl]-3-(3,4-dichlorophényl)-1-[2-(3,5-dichlorophényl)acétyl]pipéridine, isomère (+) ;
- la 3-[2-[4-(1-carbamoyl-1-méthyléthyl)pipérazin-1-yl]éthyl]-3-(3,4-dichlorophényl)-1-[2-(3,5-diéthylphényl)acétyl]pipéridine, isomère (-) ;
- la 3- [2- [4- (1-carbamoyl-1-méthyléthyl) pipéridin-1-yl]éthyl]-3-(3,4-diméthylphényl)-1-[2-(3,5-dichlorophényl)acétyl] pipéridine ;
- la 3- 12- [4- (1-carbamoyl-1-méthyléthyl)pipérazin-1-yl]éthyl]-3-(3,4-diméthylphényl)-1-[2-(3,5-dichlorophényl)acétyl]pipéridine ;

ainsi que leurs sels,leurs solvats et/ou leurs hydrates sont plus particulièrement préférés.

Selon un autre de ses aspects, la présente invention concerne un procédé pour la préparation des composés de formule (I), de leurs sels, leurs solvats et/ou leurs hydrates, caractérisé en ce que :

1a) on traite un composé de formule :

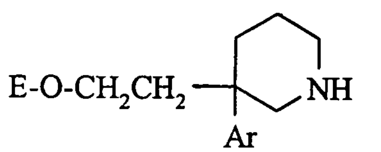

(II)

dans laquelle Ar est tel que défini pour un composé de formule (I) et E représente l'hydrogène ou un groupe O-protecteur, avec un dérivé fonctionnel d'un acide de formule :

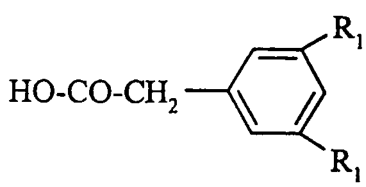

(III)

dans laquelle $R_1$ est tel que défini pour un composé de formule (I), pour obtenir un composé de formule :

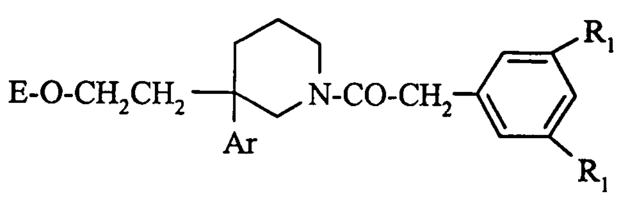

(IV) ;

2a) éventuellement, lorsque E représente un groupe protecteur, on l'élimine par action d'un acide ou d'une base, pour obtenir l'alcool de formule :

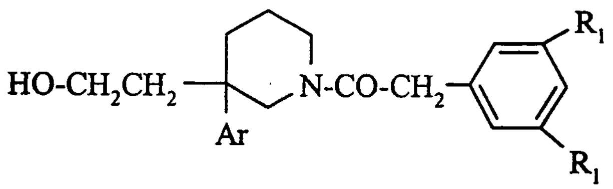

(IV, E = H) ;

3a) on traite l'alcool obtenu à l'étape 1a) ou à l'étape 2a) de formule (IV, E = H) avec un composé de formule :

$Y\text{-}SO_2\text{-}Cl$ (V)

dans laquelle Y représente un groupe méthyle, phényle, tolyle, trifluorométhyle, pour obtenir un composé de formule :

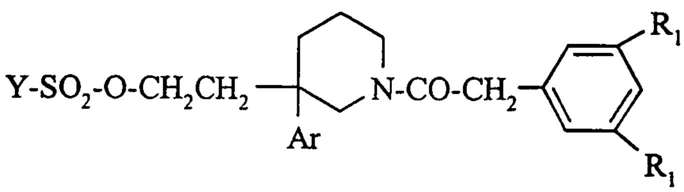

(VI) ;

4a) on fait réagir le composé de formule (VI) avec un composé de formule :

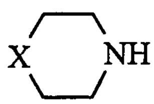

(VII)

dans laquelle X est tel que défini pour un composé de formule (I) ;
5a) et, éventuellement, on transforme le composé ainsi obtenu en l'un de ses sels avec un acide minéral ou organique.

Lorsque E représente un groupe O-protecteur, celui-ci est choisi parmi les groupes O-protecteurs classiques bien connus de l'homme de l'art, tels que, par exemple, le tétrahydropyran-2-yle, le benzoyle ou un $(C_1\text{-}C_4)$alkylcarbonyle.

Dans l'étape 1a), comme dérivé fonctionnel de l'acide (III), on utilise l'acide lui-même, ou bien un des dérivés fonctionnels qui réagissent avec les amines, par exemple un anhydride, un anhydride mixte, le chlorure d'acide, ou un ester activé, comme l'ester de paranitrophényle.

Lorsqu'on met en oeuvre l'acide de formule (III) lui-même, on opère en présence d'un agent de couplage utilisé en chimie peptidique tel que le 1,3-dicyclohexylcarbodiimide ou l'hexafluorophosphate de benzotriazol-1-yloxytris (diméthylamino)phosphonium en présence d'une base telle que la triéthylamine ou la N,N-diisopropyléthylamine, dans un solvant inerte tel que le dichlorométhane ou le N,N-diméthylformamide à une température comprise entre 0°C et la température ambiante.

Lorsqu'on utilise un chlorure d'acide, la réaction s'effectue dans un solvant inerte tel que le dichlorométhane ou le benzène, en présence d'une base telle que la triéthylamine ou la N-méthylmorpholine et à une température comprise entre -60°C et la température ambiante.

Le composé de formule (IV) ainsi obtenu est éventuellement déprotégé à l'étape 2a) selon les méthodes connues de l'homme de l'art. Par exemple, lorsque E représente un groupe tétrahydropyran-2-yle, la déprotection s'effectue par hydrolyse acide en utilisant l'acide chlorhydrique dans un solvant tel que l'éther, le méthanol ou le mélange de ces solvants, ou en utilisant le p-toluènesulfonate de pyridinium dans un solvant tel que le méthanol ou encore, en utilisant une résine Amberlyst® dans un solvant tel que le méthanol. La réaction s'effectue à une température comprise entre la température ambiante et la température de reflux du solvant. Lorsque E représente un groupe benzoyle ou un groupe $(C_1\text{-}C_4)$alkylcarbonyle, la déprotection s'effectue par hydrolyse en milieu alcalin en utilisant par exemple un hydroxyde de métal alcalin tel que l'hydroxyde de sodium, l'hydroxyde de potassium ou l'hydroxyde de lithium, dans un solvant inerte tel que l'eau, le méthanol, l'éthanol, le dioxane ou un mélange de ces solvants, à une température comprise entre 0°C et la température de reflux du solvant.

A l'étape 3a) la réaction de l'alcool de formule (IV, E = H) avec un chlorure de sulfonyle de formule (V) s'effectue en présence d'une base telle que la triéthylamine, la pyridine, la N,N-diisopropyléthylamine ou la N-méthylmorpholine,

dans un solvant inerte tel que le dichlorométhane, le benzène ou le toluène, à une température comprise entre -20˚C et la température de reflux du solvant.

Le composé de formule (VI) ainsi obtenu est mis en réaction à l'étape 4a), avec un composé de formule (VII). La réaction s'effectue dans un solvant inerte tel que le N,N-diméthylformamide, l'acétonitrile, le chlorure de méthylène, le toluène ou l'isopropanol et en présence ou en l'absence d'une base. Lorsqu'on utilise une base, celle-ci est choisie parmi les bases organiques telles que la triéthylamine, la N,N-diisopropyléthylamine ou la N-méthylmorpholine ou parmi les carbonates ou bicarbonates de métal alcalin tels que le carbonate de potassium, le carbonate de sodium ou le bicarbonate de sodium. En l'absence de base, la réaction s'effectue en utilisant un excès du composé de formule (VII) et en présence d'un iodure de métal alcalin tel que l'iodure de potassium ou l'iodure de sodium. La réaction s'effectue à une température comprise entre la température ambiante et 100˚C.

Selon une variante du procédé :

1b) on procède comme à l'étape 1a) et éventuellement à l'étape 2a) ;

2b) on oxyde le composé de formule (IV, E = H) ainsi obtenu pour préparer un composé de formule :

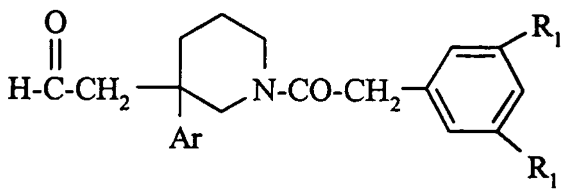

(VIII) ;

3b) on fait réagir le composé de formule (VIII) avec un composé de formule (VII) tel que défini précédemment, en présence d'un acide, puis on réduit le sel d'imminium formé intermédiairement au moyen d'un agent réducteur ;

4b) et, éventuellement, on transforme le composé ainsi obtenu en un de ses sels avec un acide minéral ou organique.

Selon la variante du procédé, à l'étape 2b) on soumet un alcool de formule (IV, E = H) à une oxydation pour obtenir un aldéhyde de formule (VIII). La réaction d'oxydation s'effectue en utilisant par exemple le chlorure d'oxalyle, le diméthylsulfoxyde et la triéthylamine dans un solvant tel que de dichlorométhane et à une température comprise entre - 78˚C et la température ambiante.

Puis à l'étape 3b), on fait réagir le composé de formule (VII) avec un aldéhyde de formule (VIII) en présence d'un acide tel que l'acide acétique, dans un solvant inerte tel que le méthanol ou le dichlorométhane, pour former *in-situ* une imine intermédiaire qui est réduite chimiquement en utilisant par exemple le cyanoborohydrure de sodium ou le triacétoxyborohydrure de sodium, ou catalytiquement en utilisant l'hydrogène et un catalyseur tel que le palladium sur charbon ou le nickel de Raney®.

On obtient finalement les composés de formule (I) selon l'invention.

Les composés de formule (I) ainsi obtenus sont isolés sous forme de base libre ou de sel, selon les techniques classiques.

Lorsque les composés de formule (I) sont obtenus sous forme de base libre, la salification est effectuée par traitement avec l'acide choisi dans un solvant organique. Par traitement de la base libre, dissoute par exemple dans un éther tel que l'éther diéthylique ou dans un alcool tel que le propan-2-ol ou dans l'acétone ou dans le dichlorométhane, ou dans l'acétate d'éthyle avec une solution de l'acide choisi dans un des solvants précités, on obtient le sel correspondant qui est isolé selon les techniques classiques.

Ainsi, on prépare par exemple le chlorhydrate, le bromhydrate, le sulfate, l'hydrogénosulfate, le dihydrogénophosphate, le méthanesulfonate, le méthylsulfate, l'oxalate, le maléate, le succinate, le fumarate, le naphtalène-2-sulfonate, le benzènesulfonate, le para-toluènesulfonate, le gluconate.

A la fin de la réaction, les composés de formule (I) peuvent être isolés sous forme d'un de leurs sels, par exemple le chlorhydrate, ou l'oxalate ; dans ce cas, s'il est nécessaire, la base libre peut être préparée par neutralisation dudit sel avec une base minérale ou organique, telle que l'hydroxyde de sodium ou la triéthylamine ou avec un carbonate ou bicarbonate alcalin, tel que le carbonate ou bicarbonate de sodium ou de potassium.

Les composés de formule (II) se prépare par des méthodes connues en particulier celles qui sont décrites dans les demandes EP-A-0 512 901, EP-A-0 591 040 ou EP-A-0 714 891.

Les composés de formule (III) sont commerciaux ou préparés selon des méthodes connues.

Ainsi, par exemple, on prépare les composés de formule (III) selon le SCHEMA 1 ci-après.

SCHEMA 1

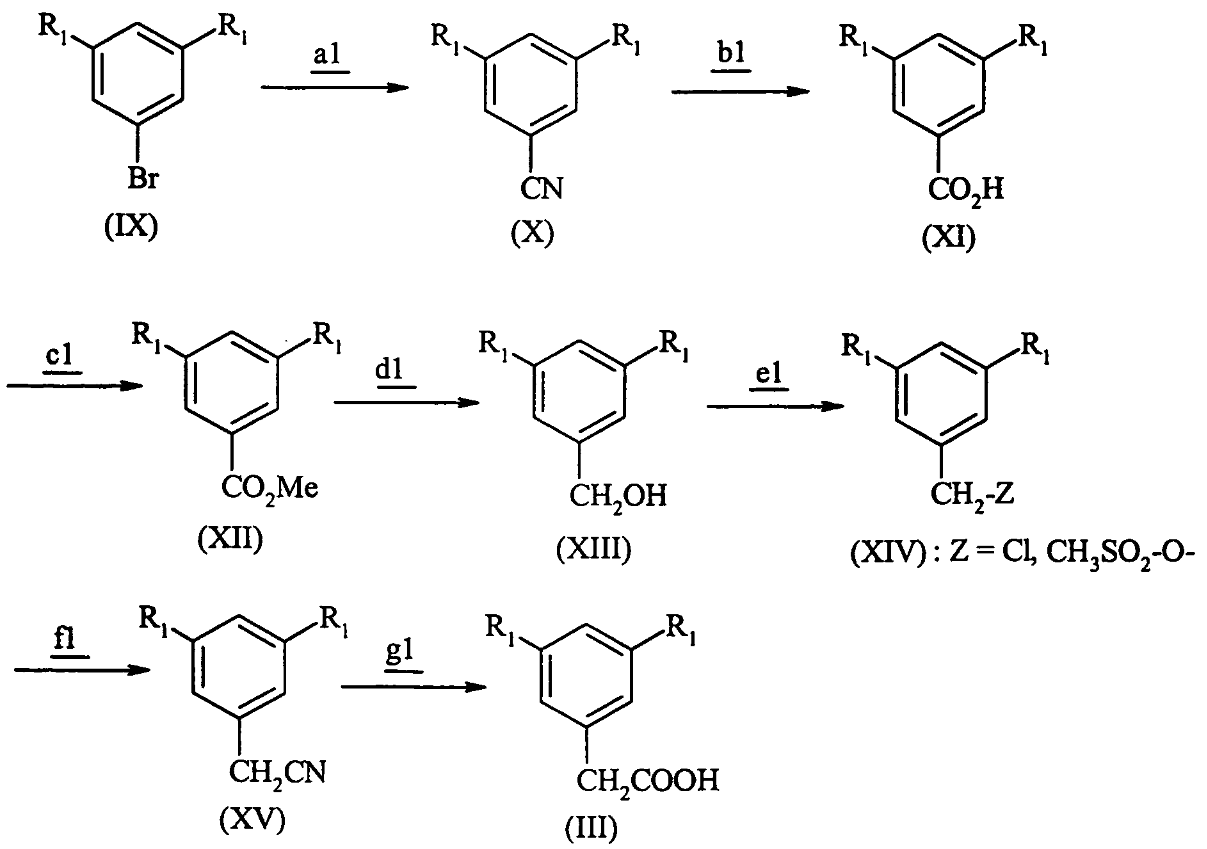


(IX) (X) (XI) (XII) (XIII) (XIV) : Z = Cl, $CH_3SO_2$-O- (XV) (III)

Les étapes <u>a1</u> et <u>b1</u> du SCHEMA 1 s'effectuent selon les méthodes décrites dans J. Am. Chem. Soc., 1941, 63, 3280-3282.

A l'étape c1 on prépare un ester de formule (XII) à partir d'un acide de formule (XI) selon les méthodes connues de l'homme de l'art.

L'ester (XII) ainsi obtenu est réduit à l'étape d1 en alcool de formule (XIII) selon les méthodes connues de l'homme de l'art.

Les étapes e1 et f1 s'effectuent selon les méthodes décrites dans J. Med. Chem., 1973, 16, 684-687.

Les dérivés phénylacétonitrile de formule (XV) ainsi obtenus sont hydrolysés à l'étape g1 en composés de formule (III) selon les méthodes décrites dans J. Org. Chem., 1968, 33, 4288 ou dans EP-A-O 714 891.

Les dérivés bromés de formule (IX) sont connus ou préparés selon des méthodes connues telles que celles décrites dans J. Org. Chem., 1971, 36(1), 193-196, ou dans J. Am. Chem. Soc., 1941, 63, 3280-3282.

Les composés de formule (VII) dans laquelle X représente un groupe

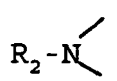

dans lequel $R_2$ représente un groupe $-CR_3R_4CONR_5R_6$ se préparent selon le SCHEMA 2 ci-après :

SCHEMA 2

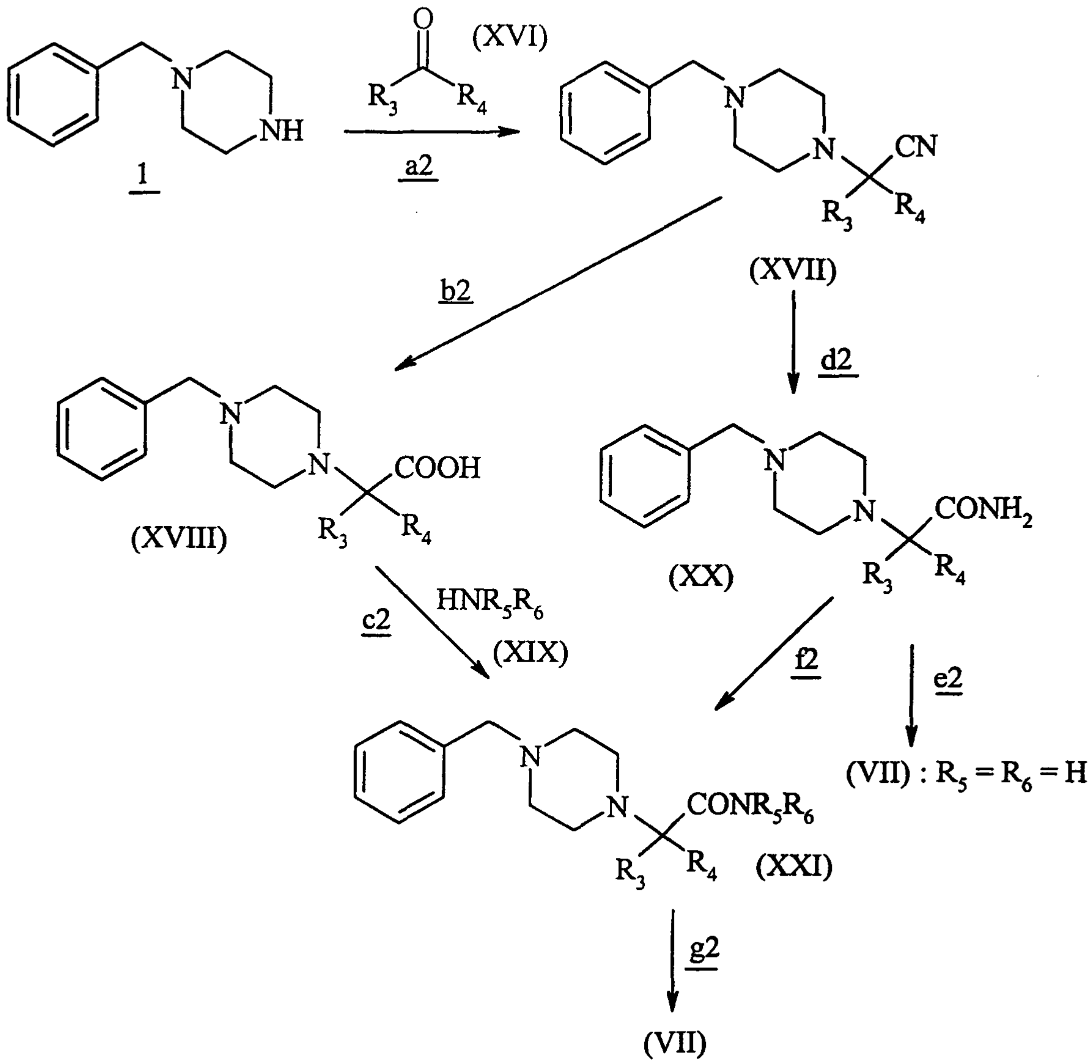


A l' étape a2 du SCHEMA 2, on fait réagir le composé 1 avec une cétone de formule (XVI), en présence de 2-hydroxyisobutyronitrile, selon la méthode décrite dans Eur. J. Med. Chem., 1990, 25, 609-615.

Le dérivé nitrile de formule (XVII) ainsi obtenu est hydrolysé à l'étape b2 selon les méthodes connues de l'homme de l'art pour donner un dérivé acide de formule (XVIII) .

L'acide (XVIII) est mis en réaction à l'étape c2 avec une amine de formule (XIX) selon les méthodes classiques du couplage peptidique pour donner le dérivé (XXI).

Alternativement, on hydrolyse, à l'étape d2, le dérivé nitrile de formule (XVII) selon les méthodes connues pour obtenir le dérivé carboxamide de formule (XX), qui est éventuellement déprotégé à l'étape e2, selon les méthodes classiques, pour obtenir le composé (VII) dans lequel $R_5 = R_6 = H$.

A l'étape f2, par réaction du composé de formule (XX), en présence d'une base forte, respectivement, avec un halogènure de $(C_1-C_3)$alkyle, ou successivement avec deux halogénures de $(C_1-C_3)$alkyle, ou avec un dihalogénure de formule Hal-$R_5$-$R_6$-Hal, selon les méthodes classiques d'alkylation, on prépare un composé de formule (XXI) dans laquelle respectivement $R_5$ représente un $(C_1-C_3)$alkyle et $R_6$ = H, ou $R_5$ et $R_6$ représentent chacun indépendamment un $(C_1-C_3)$alkyle, ou $R_5$ et $R_6$ ensemble avec l'atome d'azote auquel ils sont liés constituent un hétérocycle.

Le composé (XXI) ainsi obtenu est déprotégé à l'étape g2, selon les méthodes connues, pour donner le composé (VII) attendu.

Les composés de formule (VII) dans laquelle X représente un groupe

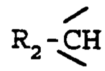

dans lequel $R_2$ représente un groupe $-CR_3R_4CONR_5R_6$ se préparent selon le SCHEMA 3 ci-après.

SCHEMA 3

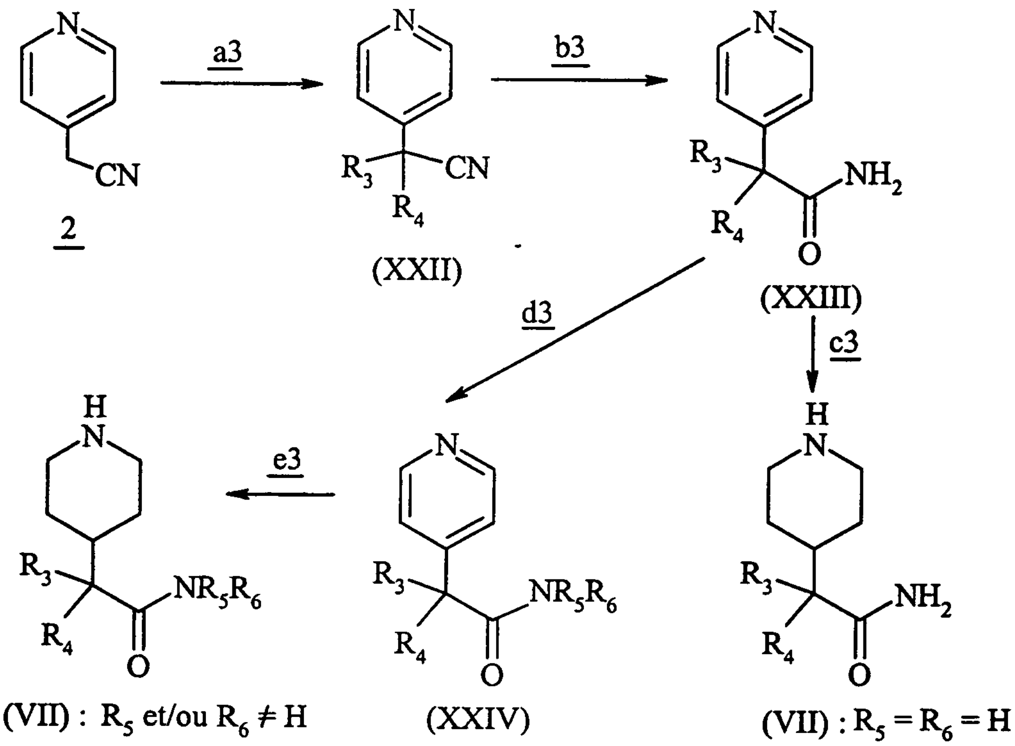


2 (XXII) (XXIII) (XXIV)

(VII) : $R_5$ et/ou $R_6 \neq H$ (XXIV) (VII) : $R_5 = R_6 = H$

A l'étape a3 du SCHEMA 3, la réaction du composé 2, en présence d'une base forte telle que l'hydrure de sodium ou l'amidure de sodium, avec respectivement un halogénure de ($C_1$-$C_4$)alkyle linéaire, ou avec un dihalogénure de formule Hal$(CH_2)_m$-Hal dans laquelle m = 2 à 5 et Hal représente un atome d'halogène, dans un solvant inerte tel que le N,N-diméthylformamide ou le dichlorométhane et à une température comprise entre 0°C et la température ambiante, selon les méthodes classiques d'alkylation, permet d'obtenir le composé de formule (XXII) dans laquelle, respectivement $R_3$ et $R_4$ représentent chacun un ($C_1$-$C_4$)alkyle linéaire ou, ensemble avec l'atome de carbone auquel ils sont liés constituent un ($C_3$-$C_6$)cycloalkyle.

Le dérivé nitrile (XXII) ainsi obtenu est hydrolysé à l'étape b3 selon les méthodes connues de l'homme de l'art pour donner le dérivé carboxamide (XXIII).

Eventuellement, à l'étape c3, on hydrogène, en présence d'un catalyseur tel que l'oxyde de platine, le cycle pyridine selon les méthodes classiques, pour obtenir un composé de formule (VII) dans laquelle $R_5$ et $R_6$ = H.

A l'étape d5, par réaction d'alkylation, selon les méthodes classiques précédemment décrites, du composé de formule (XXIII), puis réduction, par hydrogénation catalytique classique, du composé (XXIV) ainsi obtenu, on obtient un composé de formule (VII) dans laquelle $R_5$ et/ou $R_6 \neq$ H.

On peut également obtenir les composés de formule (VII) dans laquelle X représente un groupe

$$-CH-CR_3R_4CONR_5R_6$$

selon le SCHEMA 4 ci-après.

SCHEMA 4

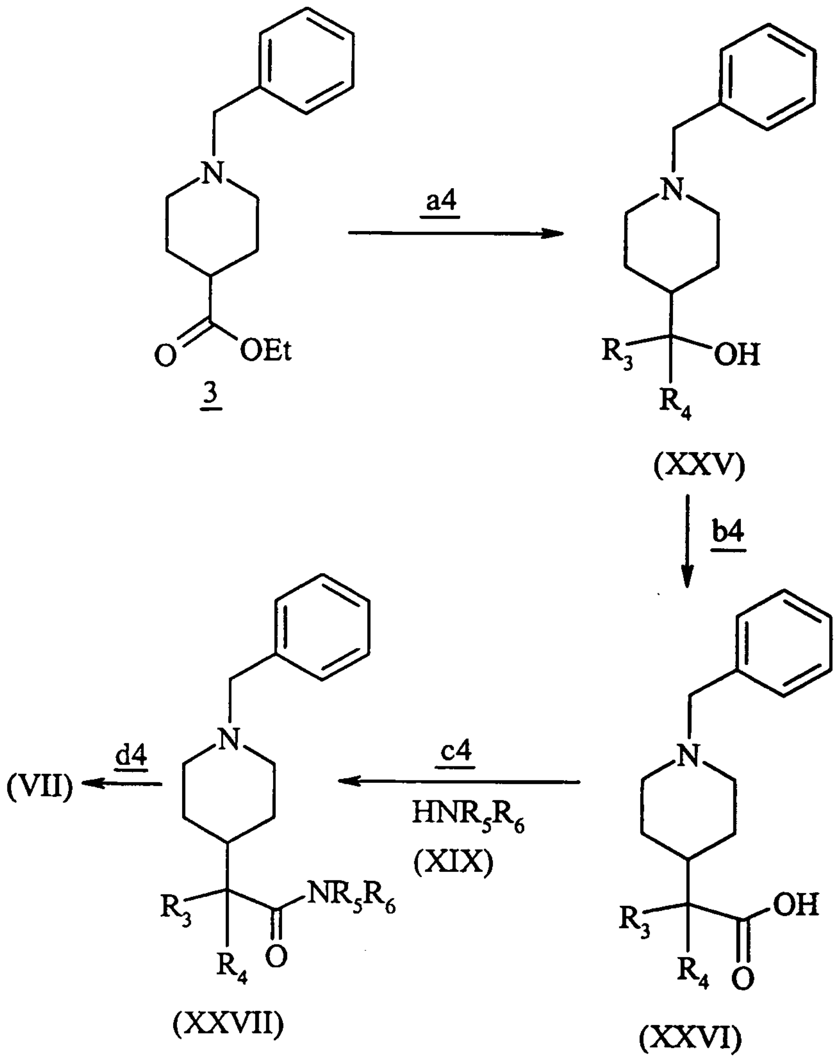


A l'étape a4 du SCHEMA 4, la réaction du composé 3 avec un dérivé organolithien ou organomagnésien approprié tel que, par exemple, le méthyllithium, le chlorure d'éthylmagnésium, le chlorure de propylmagnésium ou le pentane-1,5-di (magnésium chlorure), selon les méthodes décrites dans EP-A-0 625 509, permet d'obtenir l'alcool de formule (XXV).

L'alcool (XXV) ainsi obtenu est oxydé à l'étape b4 en acide de formule (XXVI) selon la méthode décrite dans Helvetica Chimica Acta, 1972, 55 (7), 2439.

L'acide (XXVI) est mis en réaction à l'étape c4 avec une amine de formule (XIX) selon les méthodes classiques du couplage peptidique pour donner le composé (XXVII).

Le composé (XXVII) est déprotégé à l'étape d4, selon les méthodes connues, pour donner le composé (VII) attendu.

Le composé 3 se prépare par réaction de l'isonipécotate d'éthyle avec du bromure de benzyle, en présence d'une base, selon les méthodes classiques d'alkylation.

Les composés de formule (VII) sont nouveaux et font partie de l'invention.

Ainsi, selon un autre de ses aspects, l'invention a pour objet un composé de formule :

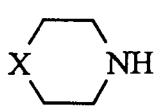 (VII)

dans laquelle :

- X représente un groupe

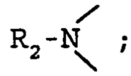

un groupe

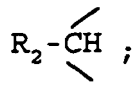

- $R_2$ représente un groupe $-CR_3R_4CONR_5R_6$ ;
- $R_3$ et $R_4$ représentent le même radical choisi parmi un méthyle, un éthyle, un n-propyle ou un n-butyle ;
- ou bien $R_3$ et $R_4$ ensemble avec l'atome de carbone auquel ils sont liés constituent un $(C_3-C_6)$cycloalkyle;
- $R_5$ et $R_6$ représentent chacun indépendamment un hydrogène ; un $(C_1-C_3)$alkyle ;
- ou bien $R_5$ et $R_6$ ensemble avec l'atome d'azote auquel ils sont liés constituent un radical hétérocyclique choisi parmi l'azétidin-1-yle, la pyrrolidin-1-yle, la pipéridin-1-yle, la morpholin-4-yle, la thiomorpholin-4-yle ou la perhydroazépin-1-yle ;

et ses sels avec des acides minéraux ou organiques.

La résolution des mélanges racémiques des composés de formule (I) permet d'isoler les énantiomères de formule :

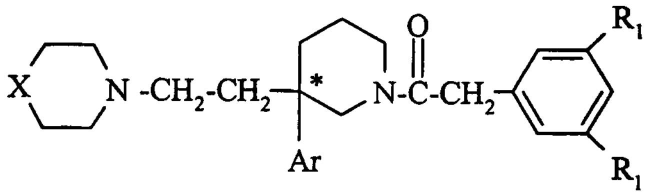

(I*)

dans laquelle :

- "*" signifie que l'atome de carbone ainsi marquée à la configuration absolue (S) ou (R) déterminée ;
- X, Ar et $R_1$ sont tels que définis pour un composé de formule (I) ; ainsi que leurs sels éventuels avec des acides minéraux

ou organiques, leurs solvats et/ou leurs hydrates.

Il est cependant préférable d'effectuer le dédoublement des mélanges racémiques à partir du composé intermédiaire de formule (II, E=H) utile pour la préparation du composé de formule (I) tel que décrit dans les demandes de brevet : EP-A-0512901, EP-A-0612716 et EP-A-0591040.

Selon un autre de ses aspects, la présente invention concerne un procédé stéréospécifique pour la préparation des composés de formule (I) ayant la configuration (S), de leurs sels, de leurs solvats et/ou hydrates, caractérisé en ce que :

1d) on traite l'isomère (S) d'un composé de formule :

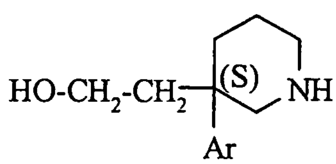

(II*, E=H)

dans laquelle Ar est tel que défini pour un composé de formule (I), avec un dérivé fonctionnel de l'acide de formule :

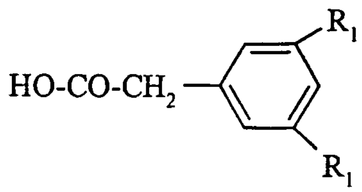

(III)

dans laquelle $R_1$ est tel que défini pour un composé de formule (I), pour obtenir un composé de formule :

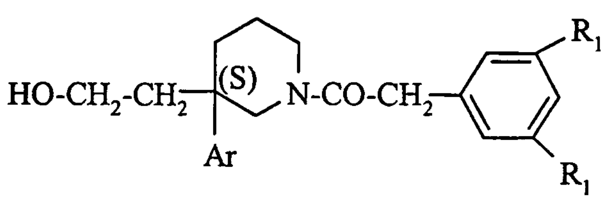

(IV*, E=H) ;

2d) on oxyde le composé de formule (IV*) pour obtenir un composé de formule :

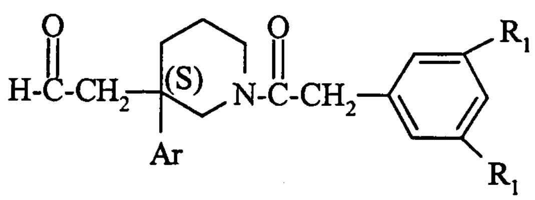

(VIII*) ;

3d) on fait réagir le composé de formule (VIII*) avec un composé de formule :

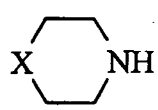

(VII) ;

dans laquelle X est tel que défini pour un composé de formule (I),
en présence d'un acide, puis on réduit le sel d'iminium formé intermédiairement au moyen d'un agent réducteur ;
4d) et, éventuellement on transforme le composé ainsi obtenu en l'un de ses sels avec un acide minéral ou organique.

Les composés de formule (I) ci-dessus comprennent également ceux dans lesquels un ou plusieurs atomes d'hydrogène, ou de carbone ont été remplacés par leur isotope radioactif par exemple le tritium, ou le carbone-14. De tels composés marqués sont utiles dans des travaux de recherche, de métabolisme ou de pharmacocinétique, dans des essais biochimiques en tant que ligand de récepteurs.

Les composés selon l'invention ont fait l'objet d'essais biochimiques.

L'affinité des composés pour les récepteurs aux tachykinines a été évaluée in vitro par plusieurs essais biochimiques utilisant des radioligands :

1) La liaison de $[^{125}I]$ BH-SP (Substance P marquée à l'iode-125 à l'aide du réactif de Bolton-Hunter) aux récepteurs $NK_1$ des cellules lymphoblastiques humaines (D. G. Payan et al., J. Immunol., 1984, 133, 3260-3265).
2) La liaison $[^{125}I]$ His-$NK_A$ aux récepteurs clonés $NK_2$ humains exprimés par des cellules CHO (Y. Takeda et al., J. Neurochem., 1992, 59, 740-745).
3) La liaison $[^{125}I]$ His $[MePhe^7]$ $NK_B$ aux récepteurs $NK_3$ du cortex cérébral de rat, du cortex cérébral de cobaye et du cortex cérébral de gerbille ainsi qu'aux récepteurs clonés $NK_3$ humains exprimés par des cellules CHO (Buell et al., FEBS Letters, 1992, 299, 90-95).

Les essais ont été effectués selon X. Emonds-Alt et al. (Eur. J. Pharmacol., 1993, 250, 403-413 ; Life Sci., 1995, 56, PL 27-32).

Les composés selon l'invention inhibent fortement la liaison de la substance P aux récepteurs $NK_1$ des cellules lymphoblastiques humaines IM9. La constante d'inhibition Ki pour les récepteurs des cellules lymphoblastiques humaines est de l'ordre de $10^{-11}$M.

Les constantes d'inhibition Ki pour les récepteurs clonés $NK_2$ humains sont de l'ordre de $10^{-8}$M et les constantes d'inhibition Ki pour les récepteurs clonés $NK_3$ humains sont supérieures à $10^{-7}$M.

Les composés de formule (I) sont des antagonistes, puissants et sélectifs, des récepteurs $NK_1$ humains de la substance P.

Ainsi les composés de formule (I) ont également été évalué in vivo sur des modèles animaux.

Chez le cobaye, au niveau du striatum, l'application locale d'un agoniste spécifique des récepteurs $NK_1$, par exemple [$Sar^9$, Met $(O_2)$ $^{11}$] substance P, augmente la libération d'acétylcholine. Cette libération est inhibée par l'administration orale ou intrapéritonéale des composés selon la présente invention. Ce test a été adapté de la méthode décrite par R. Steinberg et al., J. Neurochemistry, 1995, 65, 2543-2548.

Ces résultats montrent que les composés de formule (I) sont actifs par voie orale, qu'ils passent la barrière hématoméningée et qu'ils sont susceptibles de bloquer, au niveau du système nerveux central, l'action propre aux récepteurs $NK_1$.

Les composés de formule (I) ont été évalués dans le test de bronchoconstriction chez le cobaye, selon la méthode décrite par X. Emonds-Alt et al., European Journal of Pharmacology, 1993, 250, 403-413. Les composés de formule (I) administrés par voie intraveineuse antagonisent fortement la bronchoconstriction induite par administration intraveineuse de septide chez le cobaye dans ces conditions expérimentales.

L'activité pharmacologique in vivo des composés de formule (I) a également été évaluée dans le modèle d'hypotension chez le chien, selon la méthode décrite par X. Emonds-Alt et al., Eur. J. Pharmacol., 1993, 250, 403-413. Les composés de formule (I) administrés par voie intraveineuse inhibent fortement l'hypotension induite par administration intraveineuse de [$Sar^9$, Met $(O_2)$ $^{11}$] substance P chez le chien anesthésié dans ces conditions expérimentales.

Ces résultats montrent que les composés de formule (I) bloquent au niveau du système nerveux périphérique, l'action propre aux récepteurs $NK_1$.

Les composés de la présente invention sont notamment des principes actifs de compositions pharmaceutiques, dont la toxicité est compatible avec leur utilisation en tant que médicaments.

Les composés de formule (I) ci-dessus peuvent être utilisés à des doses journalières de 0,01 à 100 mg par kilo de poids corporel du mammifère à traiter, de préférence à des doses journalières de 0,1 à 50 mg/kg. Chez l'être humain, la dose peut varier de préférence de 0,1 à 4000 mg par jour, plus particulièrement de 0,5 à 1000 mg selon l'âge du sujet à traiter ou le type de traitement : prophylactique ou curatif.

Pour leur utilisation comme médicaments, les composés de formule (I) sont généralement administrés en unités de dosage. Lesdites unités de dosage sont de préférence formulées dans des compositions pharmaceutiques dans lesquelles le principe actif est mélangé avec un ou plusieurs excipients pharmaceutiques.

Ainsi, selon un autre de ses aspects, la présente invention concerne des compositions pharmaceutiques renfermant, en tant que principe actif, un composé de formule (I) ou l'un de ses sels, solvats et/ou hydrates pharmaceutiquement acceptables.

Dans les compositions pharmaceutiques de la présente invention pour l'administration par voie orale, sublinguale, inhalée, sous-cutanée, intramusculaire, intraveineuse, transdermique, locale ou rectale, les principes actifs peuvent être administrés sous formes unitaires d'administration, en mélange avec des supports pharmaceutiques classiques, aux animaux et aux êtres humains. Les formes unitaires d'administration appropriées comprennent les formes par voie orale telles que les comprimés, les gélules, les poudres, les granules et les solutions ou suspensions orales, les formes d'administration sublinguale et buccale, les aérosols, les formes d'administration topique, les implants, les formes d'administration sous-cutanée, intramusculaire, intraveineuse, intranasale ou intraoculaire et les formes d'administration rectale.

Lorsque l'on prépare une composition solide sous forme de comprimés ou de gélules, on ajoute au principe actif, micronisé ou non, un mélange d'excipients pharmaceutiques qui peut être composé de diluants comme par exemple le lactose, la cellulose microcristalline, l'amidon, le phosphate dicalcique, de liants comme par exemple la polyvinylpyrrolidone, l'hydroxypropylméthyl cellulose, des délitants comme la polyvinylpyrrolidone réticulée, la carboxyméthylcellulose réticulée, des agents d'écoulement comme la silice, le talc, des lubrifiants comme le stéarate de magnésium, l'acide stéarique, le tribéhénate de glycérol, le stéarylfumarate de sodium.

Des agents mouillants ou tensioactifs tels que le laurylsulfate de sodium, le polysorbate 80, le poloxamer 188 peuvent être ajoutés à la formulation.

Les comprimés peuvent être réalisés par différentes techniques, compression directe, granulation sèche, granulation humide, fusion à chaud (hot-melt).

Les comprimés peuvent être nus ou dragéifiés (par du saccharose par exemple) ou enrobés avec divers

polymères ou autres matières appropriés.

Les comprimés peuvent avoir une libération flash, retardée ou prolongée en réalisant des matrices polymériques ou en utilisant des polymères spécifiques au niveau du pelliculage.

Les gélules peuvent être molles ou dures, pelliculées ou non de manière à avoir une activité flash, prolongée ou retardée (par exemple par une forme entérique).

Elles peuvent contenir non seulement une formulation solide formulée comme précédemment pour les comprimés mais aussi des liquides ou des semi-solides.

Une préparation sous forme de sirop ou d'élixir peut contenir le principe actif conjointement avec un édulcorant, acalorique de préférence, du méthylparaben et du propylparaben comme antiseptique, ainsi qu'un agent donnant du goût et un colorant approprié.

Les poudres ou les granules dispersibles dans l'eau peuvent contenir le principe actif en mélange avec des agents de dispersion, des agents mouillants ou des agents de mise en suspension, comme la polyvinylpyrrolidone, de même qu'avec des édulcorants ou des correcteurs du goût.

Pour une administration rectale, on recourt à des suppositoires qui sont préparés avec des liants fondant à la température rectale, par exemple du beurre de cacao ou des polyéthylèneglycols.

Pour une administration parentérale, intranasale ou intraoculaire, on utilise des suspensions aqueuses, des solutions salines isotoniques ou des solutions stériles et injectables qui contiennent des agents de dispersion et/ou des agents solubilisants pharmacologiquement compatibles, par exemple le propylèneglycol.

Ainsi, pour préparer une solution aqueuse injectable par voie intraveineuse on peut utiliser un cosolvant comme par exemple un alcool tel que l'éthanol ou un glycol tel que le polyéthylèneglycol ou le propylèneglycol, et un tensioactif hydrophile tel que le polysorbate 80 ou le poloxamer 188. Pour préparer une solution huileuse injectable par voie intramusculaire, on peut solubiliser le principe actif par un triglycéride ou un ester de glycérol.

Pour l'administration locale on peut utiliser des crèmes, des pommades, des gels, des collyres, des sprays.

Pour l'administration transdermique, on peut utiliser des patches sous forme multilaminée ou à réservoir dans lequel le principe actif peut être en solution alcoolique, des sprays.

Pour une administration par inhalation on utilise un aérosol contenant par exemple du trioléate de sorbitane ou de l'acide oléique ainsi que du trichlorofluorométhane, du dichlorofluorométhane, du dichlorotétrafluoroéthane, des substituts des fréons ou tout autre gaz propulseur biologiquement compatible ; on peut également utiliser un système contenant le principe actif seul ou associé à un excipient, sous forme de poudre.

Le principe actif peut être également présenté sous forme de complexe avec une cyclodextrine, par exemple, α, β, γ-cyclodextrine, 2-hydroxypropyl-β-cyclodextrine.

Le principe actif peut être formulé également sous forme de microcapsules ou microsphères, éventuellement avec un ou plusieurs supports ou additifs.

Parmi les formes à libération prolongée utiles dans le cas de traitements chroniques, on peut utiliser les implants. Ceux-ci peuvent être préparés sous forme de suspension huileuse ou sous forme de suspension de microsphères dans un milieu isotonique.

Dans chaque unité de dosage le principe actif de formule (I) est présent dans les quantités adaptées aux doses journalières envisagées. En général chaque unité de dosage est convenablement ajustée selon le dosage et le type d'administration prévu, par exemple comprimés, gélules et similaires, sachets, ampoules, sirops et similaires, gouttes de façon à ce qu'une telle unité de dosage contienne de 0,1 à 1000 mg de principe actif, de préférence de 0,5 à 250 mg devant être administrés une à quatre fois par jour.

Bien que ces dosages soient des exemples de situations moyennes, il peut y avoir des cas particuliers où des dosages plus élevés ou plus faibles sont appropriés, de tels dosages appartiennent également à l'invention. Selon la pratique habituelle, le dosage approprié à chaque patient est déterminé par le médecin selon le mode d'administration, l'âge, le poids et la réponse dudit patient.

Selon un autre de ses aspects, la présente invention concerne l'utilisation des composés de formule (I), ou de l'un de leurs sels, solvats et/ou hydrates pharmaceutiquement acceptables pour la préparation de médicaments destinés à traiter toute pathologie où la substance P et les récepteurs NK1 humains sont impliqués.

Selon un autre de ses aspects, la présente invention concerne l'utilisation des composés de formule (I) ou de l'un de leurs sels, solvats et/ou hydrates pharmaceutiquement acceptables pour la préparation de médicaments destinés à traiter les pathologies du système respiratoire, gastro-intestinal, urinaire, immunitaire, cardiovasculaire et du système nerveux central ainsi que la douleur, la migraine, les inflammations, les nausées et les vomissements, les maladies de la peau.

Par exemple et de manière non limitative, les composés de formule (I) sont utiles :

- comme analgésique, en particulier dans le traitement de douleurs traumatiques telles que les douleurs post-opératoires ; de la névralgie du plexus brachial ; de douleurs chroniques telles que les douleurs arthritiques due à l'ostéoarthrite, l'arthrite rhumatoïde ou l'arthrite psoriatique ; de douleurs neuropathiques telles que la névralgie

post-herpétique, la névralgie du trijumeau, la névralgie segmentale ou intercostale, la fibromyalgie, la causalgie, la neuropathie périphérique, la neuropathie diabétique, les neuropathies induites par une chimiothérapie, les neuropathies liées au SIDA, la névralgie occipitale, la névralgie géniculée, la névralgie glossopharyngienne ; de douleurs de l'illusion des amputés ; de diverses formes de mal de tête telle que la migraine chronique ou aiguë, de douleur temporomandibulaire, de douleur du sinus maxillaire, du névralgisme facial, d'odontalgie ; de douleur du cancéreux ; de douleur d'origine viscérale ; de douleur gastro-intestinale ; de douleur due à la compression nerveuse, de douleurs dues à la pratique intensive d'un sport ; de la dysménorrhée ; de douleur menstruelle ; de douleur due à une méningite, à une arachnoïdite ; de douleur muscosquelettique ; de douleurs du bas du dos dues à une sténose spinale, à un disque prolabé, à une sciatique ; de douleur de l'angineux ; de douleurs dues à une spondylite ankylosante ; de douleurs liées à la goutte ; de douleurs liées aux brûlures, à la cicatrisation, à une dermatose prurigineuse ; de douleur thalamique ;

- comme antiinflammatoire en particulier pour traiter les inflammations dans l'asthme, l'influenza, les bronchites chroniques (en particulier la bronchite chronique obstructive, COPD de l'anglais chronic obstructive pulmonary disease), les toux, les allergies, le bronchospasme et l'arthrite rhumatoide ; les maladies inflammatoires du système gastro-intestinal par exemple la maladie de Crohn, les colites ulcératives, les pancréatites, les gastrites, l'inflammation des intestins, les troubles causés par les antiinflammatoires non-stéroidiens, les effets inflammatoires et secrétoires dûs aux infections bactériennes par exemple dûe au Clostridium difficile ; les maladies inflammatoires de la peau, par exemple l'herpès et l'eczéma ; les maladies inflammatoires de la vessie telles que la cystite et l'incontinence urinaire ; les inflammations ophtalmiques telles que la conjonctivite, la vitréorétinopathie ; les inflammations dentaires telles que gingivite et périodontite ;
- dans le traitement des maladies allergiques en particulier de la peau comme l'urticaire, les dermatites de contact, les dermatites atopiques et des maladies respiratoires comme les rhinites ;
- dans le traitement des maladies du système nerveux central en particulier des psychoses telles que la schizophrénie, la manie et la démence ; des désordres cognitifs telle que la maladie d'Alzheimer, l'anxiété, la démence liée au SIDA ; des neuropathies diabétiques ; de la dépression ; de la maladie de Parkinson ; de la dépendance aux drogues; des abus de substances ; des troubles de la vigilance, du sommeil, du rythme circadien, de l'humeur, de l'épilepsie ; du syndrôme de Down ; de la chorée d'Huntington ; des désordres somatiques liés au stress ; des maladies neurodégénératives telles que la maladie de Pick, la maladie de Creutzfeldt-Jacob ; des désordres liés à la panique, à la phobie, au stress ;
- dans le traitement des modifications de la perméabilité de la barrière hémato-encéphalique durant les processus inflammatoires et auto-immuns du système nerveux central, par exemple lors d'infections liées au SIDA ;
- comme myorelaxant et antispasmodique ;
- dans le traitement des nausées et vomissements aigus ou retardés et anticipés, par exemple les nausées et vomissements induits par des drogues comme les agents utilisés en chimiothérapie lors d'un cancer ; par thérapies par rayons lors d'irradiations du thorax ou de l'abdomen dans le traitement du cancer, de carcinoïdose ; par ingestion de poison ; par des toxines dues à des désordres métaboliques ou infectieux comme les gastrites, ou produites lors d'infection gastro-intestinale bactérienne ou virale ; lors d'une grossesse ; lors de troubles vestibulaires telles que le mal des transports, les vertiges, le syndrome de Ménière ; lors des maladies post-opératoires ; les nausées et vomissements induits par dialyse, par les prostaglandines ; par obstructions gastro-intestinales ; lors de motilité gastro-intestinale réduite ; lors de douleurs viscérales par infarctus du myocarde ou péritonite ; lors de migraine ; lors du mal d'altitude ; par ingestion d'analgésiques opiacés comme la morphine ; lors de reflux gastro-oesophagien ; lors d'indigestion acide ou de surconsommation de nourriture ou de boisson, lors d'acidité ou aigreur gastrique, régurgitation, brûlure d'estomac, par exemple épisodiques, nocturnes ou induites par un repas et dyspepsie ;
- dans le traitement des maladies du système gastro-intestinal telles que le syndrome du colon irritable, les ulcères gastriques et duodénaux, les ulcères oesophagiques, les diarrhées, les hypersécrétions, les lymphomes, les gastrites, les reflux gastro-oesophagiens, l'incontinence fécale, la maladie de Hirschsprung, les allergies alimentaires ;
- dans le traitement des maladies de la peau telles que le psoriasis, les prurits, les brûlures, notamment les coups de soleil ;
- dans le traitement des maladies du système cardiovasculaire telles que l'hypertension, les aspects vasculaires de la migraine, les oedèmes, la thrombose, l'angine de poitrine, les spasmes vasculaires, les maladies circulatoires dûes à une vasodilatation, la maladie de Raynaud, les fibroses, les maladies du collagène, l'athérosclérose ;
- dans le traitement des cancers du poumon à petites cellules ; des tumeurs cérébrales, des adéno-carcinomes de la sphère uro-génitale ;
- les maladies de démyelination telles que la sclérose en plaques ou la sclérose latérale amyotrophique ;
- dans le traitement des maladies du système immunitaire liées à la suppression ou à la stimulation des fonctions des cellules immunes par exemple l'arthrite rhumatoide, le psoriasis, la maladie de Crohn, le diabète, le lupus, les réactions de rejet après transplantation ;
- dans le traitement des troubles de la miction en particulier la pollakiurie ;

- dans le traitement de la réticulose histiocytaire comme dans les tissus lymphatiques ;
- comme anorexigène ;
- dans le traitement de l'emphysème ; du syndrome de Reiter ; des hémorroïdes ;
- dans le traitement des troubles oculaires telles que le glaucome, l'hypertension oculaire, le myosis, l'excès de larmes ;
- dans le traitement ou la prévention d'une attaque, de l'épilepsie, des traumatismes de la tête, des traumatismes du cordon spinal, des lésions ischémiques cérébrales dûe à une attaque ou à une occlusion vasculaire ;
- dans le traitement des troubles de la fréquence et du rythme cardiaque, en particulier ceux qui sont occasionnés par la douleur ou le stress ;
- dans le traitement des peaux sensibles et pour prévenir ou combattre les irritations de la peau ou des muqueuses, les pellicules, les érythèmes ou les prurits ;
- dans le traitement des troubles neurologiques de la peau tels que lichens, prurigos, toxidermies prurigineuses, prurits sévères d'origine neurogène ;
- dans le traitement des ulcères et de toutes maladies causées par Helicobacter Pylori ou une bactérie uréase positive gram négative ;
- dans le traitement des maladies causées par l'angiogénèse ou dont l'angiogénèse est un symtôme ;
- dans le traitement des algies oculaires et/ou palbébrales et/ou les dysesthésies oculaires ou palpébrales ;
- comme antiperspirant.

La présente invention inclut aussi une méthode pour traiter lesdites affections aux doses indiquées ci-dessus.
Les compositions pharmaceutiques selon la présente invention peuvent également renfermer d'autres produits actifs utiles pour traiter les maladies ou troubles indiqués ci-dessus, par exemple, des bronchodilatateurs, des antitussifs, des antihistaminiques, des antiinflammatoires, des antiémétiques, des agents de chimiothérapie.
Dans les Préparations et dans les Exemples on utilise les abréviations suivantes :

DMF : diméthylformamide
DMSO : diméthylsulfoxyde
DCM : dichlorométhane
THF : tétrahydrofurane
éther : éther diéthylique
éther chlorhydrique : solution saturée d'acide chlorhydrique dans l'éther diéthylique
BOP : benzotriazol-1-yloxytris(diméthylamino) phosphonium hexafluorophosphate
F : point de fusion
Teb : température d'ébullition
TA : température ambiante
silice H : gel de silice 60H commercialisé par Merck (DARMSTAD)

Les spectres de résonance magnétique nucléaire du proton (RMN $^1H$) sont enregistrés à 200 MHz dans DMSO-$d_6$, en utilisant le pic du DMSO-$d_6$ comme référence. Les déplacements chimiques δ sont indiqués en parties par million (ppm). Les signaux observés sont exprimés ainsi : s : singulet ; se : singulet élargi ; t : triplet ; qd : quadruplet ; m : massif.

PREPARATION 1.1

3-(3,4-Dichlorophényl)-3-(2-hydroxyéthyl)pipéridine, isomère (-),

(II*) : E = H ; Ar = 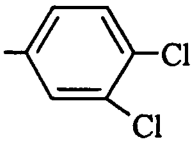

La préparation de ce composé est décrite dans la demande de brevet EP-A-0 591 040.

PREPARATION 1.2

3-(3,4-Diméthylphényl)-3-[2-(tétrahydropyran-2-yloxy)éthyl]pipéridine.

(II) : E = 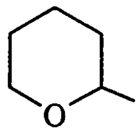 ; Ar = 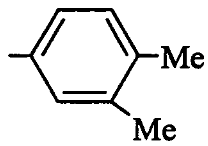

A) 2-(3,4-Diméthylphényl)-4-(tétrahydropyran-2-yloxy)butanenitrile.

A une solution de 20 g de 3,4-diméthylphényl acétonitrile dans 100 ml de THF anhydre, on ajoute, à TA et par portions, 6,6 g d'hydrure de sodium à 60 % dans l'huile et laisse 2 heures sous agitation à TA. On ajoute ensuite, en goutte à goutte, 29 g de 1-bromo-2-(tétrahydropyran-2-yloxy)éthane et laisse 2 jours sous agitation à TA. On verse le mélange réactionnel sur de la glace, extrait à l'AcOEt, lave la phase organique à l'eau, par une solution saturée de NaCl, sèche sur $Na_2SO_4$ et évapore sous vide le solvant. On chromatographie le résidu sur gel de silice en éluant par le toluène puis par le gradient du mélange toluène/AcOEt de (99/1 ; v/v) à (90/10 ; v/v). On obtient 17 g du produit attendu.

B) 4-Cyano-4-(3,4-diméthylphényl)-6-(tétrahydropyran-2-yloxy)hexanoate de méthyle.

A un mélange de 17 g du composé obtenu à l'étape précédente et 11 ml d'acrylate de méthyle dans 30 ml de dioxane, on ajoute 0,3 ml d'une solution à 40 % d'hydroxide de benzyltriméthylammonium (Triton®B) dans le MeOH et laisse 48 heures sous agitation à TA. On concentre sous vide le mélange réactionnel, reprend le résidu par une solution aqueuse d'HCl 0,5N, extrait à l'éther, lave la phase organique par une solution aqueuse de $Na_2CO_3$ à 10 %, sèche sur $Na_2SO_4$ et évapore sous vide le solvant. On obtient 23 g du produit attendu.

C) 5-(3,4-Diméthylphényl)-5-[2-(tétrahydropyran-2-yloxy)éthyl]pipérid-2-one.

A une solution de 23 g du composé obtenu à l'étape précédente dans 250 ml d'EtOH à 95 % on ajoute 40 ml d'une solution à 20 % d'ammoniaque puis on introduit le nickel de Raney® . On hydrogène ensuite pendant 24 heures à 40°C et sous une pression de 16 bars. On filtre le catalyseur sur Célite® et concentre sous vide le filtrat. On obtient 22 g du produit attendu.

D) 3-(3,4-Diméthylphényl)-3-[2-(tétrahydropyran-2-yloxy)éthyl]pipéridine.

A une suspension de 10 g d'hydrure d'aluminium et de lithium dans 200 ml de THF, on ajoute 22 g du composé obtenu à l'étape précédente et chauffe à reflux pendant 2 heures. Après refroidissement à TA, on ajoute 10 ml d'eau et 80 ml de THF, puis 10 ml de NaOH 4N et 30 ml d'eau. On filtre les sels minéraux sur Célite® et concentre sous vide le filtrat. On obtient 15 g du produit attendu.

PREPARATION 2.1

Acide 3,5-dichlorophénylacétique.
(III) : $R_1$ = Cl.

A) Chlorure de 3,5-dichlorobenzyle.

A une solution de 14,5 g d'alcool 3,5-dichlorobenzylique dans 150 ml de chloroforme, on ajoute goutte à goutte à TA une solution de 12,5 g de chlorure de thionyle dans 20 ml de chloroforme, puis chauffe à 40-50°C pendant 8 heures et laisse une nuit sous agitation à TA. On concentre sous vide et obtient 16 g du produit attendu que l'on utilise tel quel.

B) 3,5-Dichlorophénylacétonitrile.

A une solution de 16 g du composé obtenu à l'étape précédente dans 50 ml d'EtOH, on ajoute une solution

de 6,5 g de cyanure de potassium dans 50 ml d'eau et chauffe à reflux pendant 4 heures. On concentre sous vide, reprend le résidu à l'eau, extrait à l'éther, lave la phase organique à l'eau, sèche sur $Na_2SO_4$ et évapore sous vide le solvant. On chromatographie le résidu sur silice H en éluant par le mélange heptane/toluène (50/50 ; v/v) puis par du toluène. On obtient 7 g du produit attendu que l'on utilise tel quel.

C) Acide 3,5-dichlorophénylacétique.

A une solution de 7 g du composé obtenu à l'étape précédente dans 50 ml d'EtOH on ajoute une solution de 8,4 g de KOH dans 10 ml d'eau, puis chauffe à reflux pendant 5 heures. On concentre sous vide, reprend le résidu à l'eau, lave la phase aqueuse à l'éther, acidifie la phase aqueuse à pH = 1 par ajout d'HCl concentré et laisse une nuit sous agitation à TA. On essore le produit cristallise formé, le lave à l'eau et le sèche sous vide à 60°C. On obtient 7 g du produit attendu, F = 112-114,5°C.

PREPARATION 2.2

Acide 3,5-diéthylphénylacétique.
(III) : $R_1$ = Et.

A) 3,5-Diéthylbromobenzène.

On refroidit à -5°C un mélange de 20 g de 4-bromo-2,6-diéthylaniline, 160 ml d'acide acétique, 100 ml d'une solution d'HCl concentrée, 30 ml d'eau et 100 ml d'EtOH, ajoute goutte à goutte une solution de 6,6 g de nitrite de sodium dans 25 ml d'eau et laisse 30 minutes sous agitation à TA. On verse le mélange réactionnel sur 170 ml d'$H_3PO_2$ à 50 % refroidi à 0°C, laisse 2 heures sous agitation à 0°C puis 48 heures à TA. On extrait le mélange réactionnel à l'éther, lave la phase organique à l'eau, par une solution de NaOH 1N, à l'eau, sèche sur $Na_2SO_4$ et évapore sous vide le solvant. On chromatographie le résidu sur gel de silice en éluant au cyclohexane. On obtient 18 g du produit attendu.

B) 3,5-Diéthylbenzonitrile.

On chauffe pendant 15 heures à reflux un mélange de 24,7 g du composé obtenu à l'étape précédente et 12 g de cyanure cuivreux dans 70 ml de DMF. Après refroidissement à TA, on verse le mélange réactionnel sur 50 ml d'eau et laisse sous agitation à TA jusqu'à formation d'une gomme. On refroidit le mélange au bain de glace, ajoute 150 ml d'éthylènediamine et laisse 2 heures sous agitation à TA. On extrait le mélange à l'AcOEt, lave la phase organique à l'eau, sèche sur $Na_2SO_4$ et évapore sous vide le solvant. On chromatographie le résidu sur gel de silice en éluant par le mélange cyclohexane/AcOEt (95/5 ; v/v). On obtient 12 g du produit attendu.

C) Acide 3, 5-diéthylbenzoïque.

A une solution de 12 g du composé obtenu à l'étape précédente dans 60 ml d'EtOH on ajoute une solution de 22 g de KOH dans 15 ml d'eau et chauffe à reflux pendant 24 heures. On concentre sous vide le mélange réactionnel, extrait le résidu à l'eau, lave la phase aqueuse à l'éther, acidifie la phase aqueuse à pH = 2 par ajout d'HCl concentrée, essore le précipité formé, le lave à l'eau et le sèche sous vide. On obtient 13 g du produit attendu.

D) Ester méthylique de l'acide 3,5-diéthylbenzoique.

On chauffe à reflux pendant 48 heures un mélange de 13 g du composé obtenu à l'étape précédente dans 90 ml de MeOH et 10 gouttes d'$H_2SO_4$. On concentre sous vide le mélange réactionnel, reprend le résidu à l'eau, neutralise par ajout d'une solution à 10 % de $NaHCO_3$ extrait à l'éther, lave la phase organique par une solution de $NaHCO_3$ à 10 %, à l'eau, sèche sur $Na_2SO_4$ et évapore sous vide le solvant. On obtient 12 g du produit attendu.

E) Alcool 3,5-diéthylbenzylique.

On refroidit à 0°C une suspension de 2,5 g d'hydrure d'aluminium et de lithium dans 50 ml de THF, ajoute goutte à goutte une solution de 12 g du composé obtenu à l'étape précédente dans 50 ml de THF et laisse 30 minutes sous agitation. On hydrolyse le mélange réactionnel par ajout de 2,5 ml d'eau, 2,5 ml de NaOH 4N et 7,5 ml d'eau. On filtre les sels minéraux et concentre sous vide le filtrat. On obtient 10,9 g du produit attendu qui est utilisé tel quel.

F) Méthanesulfonate de 3,5-diéthylbenzyle.

A une solution de 10,9 g du composé obtenu à l'étape précédente et 7,4 g de triéthylamine dans 100 ml de DCM, on ajoute goutte à goutte, à TA, une solution de 8,4 g de chlorure de méthanesulfonyle dans 50 ml de DCM et laisse 30 minutes sous agitation. On concentre sous vide le mélange réactionnel, reprend le résidu à l'eau, extrait à l'éther, lave la phase organique à l'eau, sèche sur $Na_2SO_4$ et évapore sous vide le solvant. On obtient 16 g du produit attendu que l'on utilise tel quel.

G) 3,5-Diéthylphénylacétonitrile.

A une solution de 16 g du composé obtenu à l'étape précédente dans 100 ml de DMF, on ajoute une solution de 5,15 g de cyanure de potassium dans 20 ml d'eau et chauffe à 80˚C pendant 1 heure. On concentre sous vide le mélange réactionnel, reprend le résidu à l'eau, extrait à l'éther, lave la phase organique à l'eau, sèche sur $Na_2SO_4$ et évapore sous vide le solvant. On chromatographie le résidu sur gel de silice H en éluant au DCM. On obtient 3 g du produit attendu.

H) Acide 3, 5 -diéthylphénylacétique.

A une solution de 3 g du composé obtenu à l'étape précédente dans 50 ml d'EtOH on ajoute une solution de 7,8 g de KOH dans 10 ml d'eau, puis chauffe à reflux pendant 5 heures. On concentre sous vide, reprend le résidu à l'eau, lave la phase aqueuse à l'éther, acidifie la phase aqueuse à pH = 1 par ajout d'HCl concentré et laisse une nuit sous agitation à TA. On essore le produit cristallisé formé, le lave à l'eau et le sèche sous vide. On obtient 2,5 g du produit attendu.

RMN $^1H$ δ (ppm) : 1,1 : t : 6H ; 2,4 : qd : 4H ; 3,4 : s : 2H ; 6,8 : m : 3H ; 12,2 : se : 1H.

PREPARATION 3.1

Chlorhydrate de 2-(pipéridin-4-yl)isobutyramide.

$$(VII), HCl : X = -\underset{|}{C}H-C(CH_3)_2-CONH_2.$$

A) 2-Méthyl-2-(pyridin-4-yl)propionitrile.

On refroidit à 0˚C un mélange de 3 g de chlorhydrate de pyridin-4-ylacétonitrile dans 50 ml de DMF, ajoute par petites portions 2,6 g d'hydrure de sodium à 60 % dans l'huile, et laisse 2 heures sous agitation à TA. On refroidit le mélange réactionnel au bain de glace, ajoute goutte à goutte 6 g d'iodure de méthyle et laisse une nuit sous agitation à TA. On verse le mélange réactionnel sur un mélange eau/glace, extrait à l'éther, lave la phase organique par une solution saturée de NaCl, sèche sur $MgSO_4$, filtre et évapore sous vide le solvant. On chromatographie le résidu sur gel de silice H en éluant au DCM puis par le mélange DCM/MeOH (98/2 ; v/v). On obtient 2,39 g du produit attendu sous forme d'huile qui cristallise.

B) Chlorhydrate de 2-(pyridin-4-yl)isobutyramide.

On chauffe à 100˚C pendant 15 minutes un mélange de 2,39 g du composé obtenu à l'étape précédente et 10 ml d'une solution d'$H_2SO_4$ concentré. On refroidit le mélange réactionnel à TA, ajoute 50 g de glace, alcalinise à pH = 14 par ajout d'une solution de NaOH concentrée, filtre les sels minéraux, extrait le filtrat à l'AcOEt puis au DCM, sèche les phases organiques jointes sur $MgSO_4$, filtre et évapore sous vide les solvants (F = 134˚C, base). On dissout le produit obtenu dans l'acétone, acidifie à pH = 1 par ajout d'éther chlorhydrique et essore le précipité formé. On obtient 2,9 g du produit attendu.

C) Chlorhydrate de 2-(pipéridin-4-yl)isobutyramide.

On hydrogène pendant 3 jours, à 60˚C, sous une pression de 60 bars un mélange de 2,9 g du composé obtenu

à l'étape précédente, 1 g de $PtO_2$ et 50 ml de MeOH. On filtre le catalyseur sur Célite®, lave au MeOH et concentre sous vide le filtrat. On reprend le résidu dans l'acétonitrile, essore le précipité formé, le lave à l'acétonitrile puis à l'éther. On obtient 2,5 g du produit attendu, F > 260°C.

PREPARATION 3.2

Dichlorhydrate de 2-(pipérazin-1-yl)isobutyramide.

(VII), 2HCl : X = 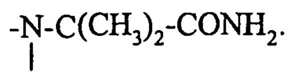

A) 2-(4-Benzylpipérazin-1-yl)-2-méthylpropionitrile.

On mélange 4,5 ml d'acétone, 20 g de $MgSO_4$ sec, 10 g de N,N-diméthylacétamide, 10 g de 1-benzylpipérazine et 9,5 ml de 2-hydroxyisobutyronitrile et chauffe à 45°C pendant 48 heures sous forte agitation. On verse le mélange réactionnel sur de la glace et laisse 30 minutes sous agitation. On extrait le mélange à l'éther, lave plusieurs fois la phase organique à l'eau, sèche sur $Na_2SO_4$ et évapore sous vide le solvant. On obtient 13 g du produit attendu.

B) Dichlorhydrate de 2-(4-benzylpipérazin-1-yl)isobutyramide.

On chauffe rapidement à 110°C pendant 30 minutes un mélange de 13 g du composé obtenu à l'étape précédente et 130 ml d'une solution d'$H_2SO_4$ à 90 %. Après refroidissement à TA, on verse le mélange réactionnel sur de la glace, alcalinise à pH = 10 par ajout d'une solution de $NH_4OH$ concentrée et essore le produit cristallisé formé. On dissout le produit dans du DCM, sèche la phase organique sur $MgSO_4$ et évapore sous vide le solvant. On reprend le produit dans l'éther chlorhydrique et essore le précipité formé. On obtient 9,5 g du produit attendu.

C) Dichlorhydrate de 2-(pipérazin-1-yl)isobutyramide.

On hydrogène pendant une nuit, à TA et à pression atmosphérique, un mélange de 1,3 g du composé obtenu à l'étape précédente et 0,18 g de Palladium sur charbon à 10 % dans 30 ml d'EtOH 95. On filtre le catalyseur sur Célite® et concentre sous vide le filtrat. On obtient 0,6 g du produit attendu.

PREPARATION 3.3

Dichlorhydrate de 1-(pipérazin-1-yl)cyclohexane carboxamide.

(VII), 2HCl : X = 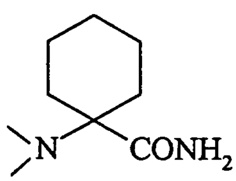

A) 1-(4-Benzylpipérazin-1-yl)cyclohexanecarbonitrile.

On mélange 5,7 g de cyclohexanone, 20 g de $MgSO_4$ sec, 10 g de N,N-diméthylacétamide, 10 g de 1-benzylpipérazine et 9,5 ml de 2-hydroxyisobutyronitrile et chauffe à 45°C pendant 48 heures sous forte agitation. On verse le mélange réactionnel sur de la glace et laisse 30 minutes sous agitation. On extrait le mélange à l'éther, lave plusieurs fois la phase organique à l'eau, sèche sur $Na_2SO_4$ et évapore sous vide le solvant. On obtient 15 g du produit attendu.

B) Dichlorhydrate de 1-(4-benzylpipérazin-1-yl)cyclohexanecarboxamide.

On prépare ce composé selon le mode opératoire décrit à l'étape B de la Préparation 3.2 à partir de 15 g du composé obtenu à l'étape précédente et 50 ml d'une solution d'$H_2SO_4$ à 90 %. On obtient 5,5 g du produit attendu.

C) Dichlorhydrate de 1-(pipérazin-1-yl)cyclohexane carboxamide.

On prépare ce composé selon le mode opératoire décrit à l'étape C de la Préparation 3.2 à partir de 2,3 g du composé obtenu à l'étape précédente, 0,3 g de palladium sur charbon à 10 % dans 30 ml d'EtOH 95. On obtient 1,6 g du produit attendu.

PREPARATION 3.4

Diformate de N,N-diméthyl-2-(pipérazin-1-yl) isobutyramide.

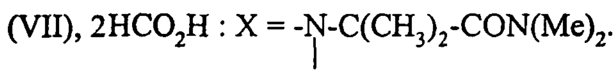

A)N,N-diméthyl-2-(4-benzylpipérazin-1-yl)isoburyramide.

A un mélange de 2,6 g de composé obtenu à l'étape B de la Préparation 3.2 (base libre) dans 50 ml de THF anhydre, on ajoute par portions 1,44 g d'hydrure de sodium à 60 % dans l'huile. On ajoute ensuite, en goutte à goutte, 1,3 ml d'iodure de méthyle et laisse 4 heures sous agitation à TA. On verse le mélange réactionnel dans l'eau, extrait à l'éther, sèche la phase organique sur $MgSO_4$ et évapore sous vide les solvants. On obtient 1,8 g du produit attendu.

B) Diformate de N,N-diméthyl-2-(pipérazin-1-yl)isobutyramide.

A une solution de 1,8 g du composé obtenu à l'étape précédente dans 30 ml de MeOH, on ajoute 2 g de formiate d'ammonium, 0,5 g de palladium sur charbon à 5 % et laisse 4 heures sous agitation à TA. On filtre le catalyseur sur Célite® et concentre sous vide le filtrat. On reprend le résidu dans l'AcOEt, essore le précipité formé, le lave à l'AcOEt et le sèche. On obtient 1,2 g du produit attendu.

PREPARATION 3.5

Chlorhydrate de 1-(pipéridin-4-yl)cyclohexane carboxamide.

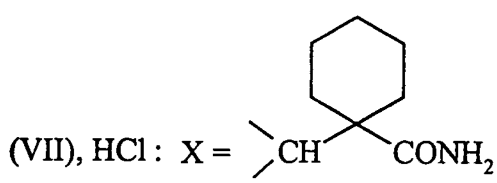

A) 1-(Pyridin-4-yl)cyclohexanecarbonitrile.

On refroidit à 0°C un mélange de 3 g de chlorhydrate de pyridin-4-ylacétonitrile dans 50 ml de DMF, ajoute par petites portions 2,6 g d'hydrure de sodium à 60 % dans l'huile et laisse 1 heure 30 minutes sous agitation à TA. On refroidit le mélange réactionnel au bain de glace, ajoute goutte à goutte 2,7 ml de 1,5-dibromopentane et laisse 48 heures sous agitation à TA. On verse le mélange réactionnel sur une solution saturée de $NH_4Cl$, extrait à l'éther, lave trois fois 1a phase organique à l'eau, sèche sur $MgSO_4$ et évapore sous vide le solvant. On chromatographie le résidu sur gel de silice H en éluant au DCM puis par le mélange DCM/MeOH (98/2 ; v/v). On obtient 2,5 g du produit attendu, F = 79°C.

B) Chlorhydrate de 1- (pyridin-4-yl)cyclohexane carboxamide.

On chauffe à 100˚C pendant 15 minutes un mélange de 2,5 g du composé obtenu à l'étape précédente et 15 ml d'une solution d'$H_2SO_4$ concentrée. On refroidit le mélange réactionnel à TA, le verse sur de la glace, alcalinise à pH = 14 par ajout d'une solution de NaOH concentrée, essore le précipité formé, le lave à l'eau et le sèche. On dissout le produit obtenu dans l'acétone, acidifie à pH = 1 par ajout d'éther chlorhydrique, laisse 30 minutes sous agitation à TA et essore le précipité formé. On obtient 3 g du produit attendu, F = 224˚C (déc.).

C) Chlorhydrate de 1-(pipéridin-4-yl)cyclohexane carboxamide.

On hydrogène pendant 3 jours, à 60˚C, sous une pression de 80 bars, un mélange de 2,9 g du composé obtenu à l'étape précédente, 0,5 g de $PtO_2$ et 50 ml de MeOH. On filtre le catalyseur sur Célite®, et concentre sous vide le filtrat. On reprend le résidu dans l'acétonitrile, laisse 1 heure sous agitation à TA, et essore le précipité formé. On obtient 2,7 g du produit attendu, F = 235˚C.

PREPARATION 3.6

Chlorhydrate de N,N-diméthyl-2-(pipéridin-4-yl) isobutyramide.

$$(VII), HCl : X = -CH-C(CH_3)_2-CON(CH_3)_2$$

A) Ester éthylique de l'acide 1-benzylpipéridine-4-carboxylique.

A un mélange de 25 g d'isonipécotate d'éthyle et 25 g de $K_2CO_3$ dans 125 ml de DMF, on ajoute goutte à goutte 30 g de bromure de benzyle en maintenant la température du mélange réactionnel entre 25 et 30˚C, puis laisse 1 heure sous agitation à TA. On verse le mélange réactionnel sur 1 litre d'eau glacée, extrait deux fois à l'éther, lave la phase organique à l'eau, sèche sur $MgSO_4$ et évapore sous vide le solvant. On distille l'huile résultante obtenue sous pression réduite. On obtient 29,2 g du produit attendu, Teb = 120-122˚C sous 2,7 Pa.

B) 2-(1-Benzylpipéridin-4-yl)propan-2-ol.

A 200 ml d'une solution 1,5 M de méthyllithium, comme complexe avec du bromure de lithium, dans l'éther, on ajoute, sous atmosphère d'argon, en goutte à goutte et en maintenant la température du milieu entre 25 et 30˚C, une solution de 24,73 g du composé obtenu à l'étape précédente dans 100 ml de benzène, puis chauffe à reflux pendant 48 heures. On refroidit le mélange réactionnel à TA, puis le verse sur 400 ml d'une solution saturée de $NH_4Cl$ dans l'eau préalablement refroidie au bain de glace. On extrait le mélange trois fois à l'éther, sèche les phases organiques jointes sur $MgSO_4$ et concentre sous vide le solvant. On dissout le résidu dans 100 ml d'acétone, refroidit à 10˚C, acidifie à pH = 1 par ajout d'éther chlorhydrique, essore le précipité formé et le lave par un mélange acétone/éther (50/50 ; v/v). On obtient 24,5 g du produit attendu sous forme de chlorhydrate, F = 204˚C. Pour libérer la base, on reprend le chlorhydrate dans une solution concentrée de NaOH, extrait à l'éther, sèche sur $MgSO_4$ et évapore sous vide le solvant. On obtient 21 g du produit attendu, F = 66˚C.

C) Acide 2-(1-benzylpipéridin-4-yl)-2-méthylpropionique.

On refroidit à 3˚C un mélange de 5,98 g d'acide sulfurique à 95 % et 4,42 g d'acide sulfurique fumant à 30 % en $SO_3$, ajoute, goutte à goutte et en maintenant la température en dessous de 10˚C, une solution de 2 g du composé obtenu à l'étape précédente dans 1,55 g d'acide formique à 100 %. On laisse 2 heures sous agitation à 3-5˚C, puis laisse remonter la température à TA et abandonne une nuit à TA. On verse le mélange réactionnel sur de la glace, amène à pH = 6,5 par ajout d'une solution concentrée de NaOH et par ajout d'une solution concentrée de $NH_4OH$, extrait trois fois au DCM, sèche les phases organiques jointes sur $MgSO_4$ et concentre sous vide le solvant. On reprend le résidu à l'acétone, essore le précipité et le sèche. On obtient 1,22 g du produit attendu, F = 195˚C.

D) Chlorhydrate de N,N-diméthyl-2-(1-benzylpipéridin-4-yl)isobutyramide.

On laisse 1 heure sous agitation à TA un mélange de 1,2 g du composé obtenu à l'étape précédente, 0,8 ml de triéthylamine, 2,8 ml d'une solution 2 M de diméthylamine dans le THF et 2,5 g de BOP dans 20 ml de DCM. On concentre sous vide le mélange réactionnel, reprend le résidu à l'éther, lave la phase organique à l'eau, par une solution de NaOH 1N, par une solution saturée de NaCl, sèche sur $MgSO_4$ et concentre sous vide le solvant. On chromatographie le résidu sur gel de silice H en éluant au DCM, puis par le gradient du mélange DCM/MeOH de (99/1 ; v/v) à (95/5 ; v/v). On dissout le produit obtenu dans l'acétone, acidifie à pH = 1 par ajout d'éther chlorhydrique, essore le précipité formé et le sèche. On obtient 0,8 g du produit attendu, F = 229°C.

E) Chlorhydrate de N,N-diméthyl-2-(pipéridin-4-yl)isobutyramide.

On hydrogène pendant une nuit, sous pression atmosphérique et à TA, un mélange de 0,8 g du composé obtenu à l'étape précédente et 0,2 g de palladium sur charbon à 10 % dans 20 ml de MeOH. On filtre le catalyseur sur Célite® et concentre sous vide le filtrat. On dissout le résidu dans l'acétonitrile, ajoute de l'éther, essore le précipité formé et le sèche. On obtient 0,51 g du produit attendu, F = 258°C.

PREPARATION 3.7

Chlorhydrate de 1-(pipéridin-4-yl)cyclopropane carboxamide.

(VII), HCl : X = 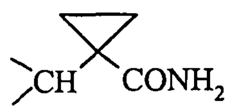

A) 1-(Pyridin-4-yl)cyclopropanecarbonitrile.

A un mélange de 2,5 g d'amidure de sodium dans 80 ml de DCM, on ajoute 3,5 g de pyridin-4-ylacétonitrile puis 2,6 ml de 1,2-dibromoéthane et laisse une nuit sous agitation à TA. On verse le mélange réactionnel dans l'eau, extrait à l'AcOEt, lave la phase organique à l'eau, sèche sur $Na_2SO_4$ et évapore sous vide les solvants. On chromatographie le résidu sur gel de silice en éluant au DCM puis par le mélange DCM/MeOH de (99/1 ; v/v) à (95/5 ; v/v). On obtient 2,5 g du produit attendu.

B) Chlorhydrate de 1-(pyridin-4-yl)cyclopropane carboxamide.

On chauffe rapidement à 100°C un mélange de 2,5 g du composé obtenu à l'étape précédente et 20 ml d'une solution d'$H_2SO_4$ à 96 % et laisse 1 heure sous agitation à 100°C. Après refroidissement à TA, on verse le mélange réactionnel sur de la glace, neutralise à pH = 7 par ajout d'une solution de $NH_4OH$ à 20 %, essore le précipité formé, le lave à l'eau et le sèche. On dissout le précipité dans le DCM, acidifie à pH = 1 par ajout d'éther chlorhydrique et essore le précipité formé. On obtient 1,8 g du produit attendu.

C) Chlorhydrate de 1-(pipéridin-4-yl)cyclopropane carboxamide.

On hydrogène pendant 15 heures, à 80°C et sous une pression de 100 bars, un mélange de 1,8 g du composé obtenu à l'étape précédente et 0,6 g de $PtO_2$ dans 50 ml de MeOH. On filtre le catalyseur sur Célite®, concentre sous vide le filtrat jusqu'à un volume de 5 ml, et ajoute de l'acétonitrile jusqu'à cristallisation. On obtient 1,7 g du produit attendu après essorage puis séchage.

PREPARATION 3.8

Chlorhydrate de 2-méthyl-1-(morpholin-4-yl)-2-(pipéridin-4-yl)propan-1-one.

(VII), HCl : X = 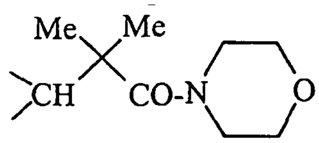

A) Chlorhydrate de 2-(1-benzylpipéridin-4-yl)-2-méthyl-1-(morpholin-4-yl)propan-1-one.

On chauffe à 80°C pendant 3 heures un mélange de 1 g du composé obtenu à l'étape C de la Préparation 3.6 et 1,2 ml de chlorure de thionyle dans 20 ml de 1,2-dichloroéthane. On concentre sous vide le mélange réactionnel, dissout le chlorure d'acide ainsi obtenu dans 20 ml de DCM, ajoute cette solution à un mélange de 0,7 g de morpholine, 1,6 ml de triéthylamine dans 20 ml de DCM préalablement refroidi à 0°C et laisse 24 heures sous agitation à TA. On concentre sous vide le mélange réactionnel, extrait le résidu à l'éther, lave la phase organique par une solution de NaOH 1N, à l'eau, sèche sur $MgSO_4$ et évapore sous vide le solvant. On dissout le produit obtenu dans l'acétone, acidifie à pH = 1 par ajout d'éther chlorhydrique, essore le précipité formé et le sèche. On obtient 0,7 g du produit attendu.

B) Chlorhydrate de 2-méthyl-1- (morpholin-4-yl) -2-(pipéridin-4-yl)propan-1-one.

On laisse 4 heures sous agitation à TA un mélange de 0,7 g du composé obtenu à l'étape précédente, 0,7 g de formiate d'ammonium et 0,2 g de palladium sur charbon à 10 % dans 10 ml de MeOH. On filtre le catalyseur sur Célite® et concentre sous vide le filtrat. On dissout le résidu dans l'acétonitrile, ajoute de l'éther, essore le précipité formé et le sèche. On obtient 0,46 g du produit attendu, F = 225°C.

EXEMPLE 1

Chlorhydrate de 3- [2- [4- (1-carbamoyl-1-méthyléthyl) pipéridin-1-yl]éthyl]-3-(3,4-dichlorophényl)-1-[2-(3,5-diméthylphényl)acétyl]pipéridine, monohydrate, isomère (-).

(I), HCl : X = $H_2NCO\text{-}C(CH_3)_2\text{-}\underset{|}{C}H\text{-}$, $R_1$ = Me ; Ar = 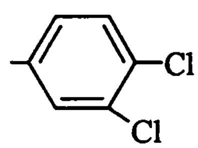

A) 3-(3,4-Dichlorophényl)-3-(2-hydroxyéthyl)-1-[2-(3,5-diméthylphényl)acétyl] pipéridine, isomère unique.

A un mélange de 2,0 g d'acide 3,5-diméthylphénylacétique dans 100 ml de DCM, on ajoute à TA 2,3 ml de triéthylamine, puis 3 g du composé obtenu à la Préparation 1 et 5,3 g de BOP, et laisse 1 heure sous agitation à TA. On concentre sous vide le mélange réactionnel, extrait le résidu à l'éther, lave la phase organique à l'eau, par une solution d'HCl 2N, à l'eau, par une solution à 10 % de NaOH dans l'eau, sèche sur $Na_2SO_4$, filtre et concentre sous vide le filtrat. On chromatographie le résidu sur gel de silice H en éluant au DCM, puis par le mélange DCM/MeOH (98/2 ; v/v). On obtient 3,9 g du produit attendu qui est utilisé tel quel à l'étape suivante.

B) 3-(3,4-Dichlorophényl)-3-(formylméthyl)-1-[2-(3,5-diméthylphényl)acétyl] pipéridine, isomère unique.

On refroidit à -70°C, sous atmosphère d'azote, une solution de 0,25 ml de chlorure d'oxalyle dans 3 ml de DCM, ajoute goutte à goutte une solution de 0,35 ml de DMSO dans 3 ml de DCM, puis une solution de 0,5 g du composé obtenu à l'étape précédente dans 5 ml de DCM et laisse 15 minutes sous agitation à -50°C. On ajoute ensuite 0,9 ml de triéthylamine et laisse sous agitation en laissant remonter la température à TA. On lave le mélange réactionnel à l'eau, par une solution d'HCl 1N, par une solution à 10 % de $NaHCO_3$ sèche la phase organique sur $Na_2SO_4$, filtre et concentre sous vide le filtrat. On obtient 0,5 g du produit attendu que l'on utilise tel quel à l'étape suivante.

C) Chlorhydrate de 3- [2- [4- (1-carbamoyl-1-méthyléthyl) pipéridin-1-yl]éthyl]-3-(3,4-dichlorophényl)-1-[2-(3,5-diméthylphényl)acétyl]pipéridine, monohydrate, isomère (-).

A une solution de 0,24 g du composé obtenu à la Préparation 3.1 (base libre) dans 3 ml de MeOH, on ajoute à TA et sous atmosphère d'azote, 0,08 ml d'acide acétique puis une solution de 0,5 g du composé obtenu à l'étape précédente dans 5 ml de MeOH. Après 5 minutes, on ajoute 0,08 g de cyanoborohydrure de sodium et laisse une nuit sous agitation à TA. On verse le mélange réactionnel sur une solution à 10 % de $NaHCO_3$ dans l'eau, extrait à l'éther, lave la phase organique à l'eau, sèche sur $Na_2SO_4$, filtre et concentre sous vide le filtrat. On chromatographie le résidu sur gel de silice H en éluant au DCM, puis par le gradient du mélange DCM/MeOH de (99/1 : v/v) à (90/10 ; v/v). On dissout le produit obtenu dans du DCM, acidifie jusqu'à pH = 1 par ajout d'éther chlorhydrique et concentre sous vide. On obtient 0,5 g du produit attendu après trituration dans l'éther, essorage et séchage sous vide.

$\alpha_D^{20}$ = -27, 7° (c = 1 ; MeOH).

RMN $^1H$ : δ (ppm) : 0,7 à 1,2 : se : 6H ; 1,2 à 2,4 : m : 16H ; 2,5 à 4,8 : m : 12H ; 6,5 à 8,0 : m : 8H ; 10,2 : se : 1H.

EXEMPLE 2

Dichlorhydratede3-[2-[4-(1-carbamoyl-1-méthyléthyl)pipérazin-1-yl]éthyl]-3-(3,4-dichlorophényl)-1-[2-(3,5-diméthylphényl)acétyl]pipéridine, 2,7 $H_2O$, isomère (-).

(I), 2HCl : X = $H_2NCO\text{-}C(CH_3)_2\text{-}N\text{-}$ ; $R_1$ = Me ; Ar = 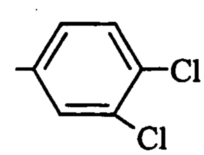

A une solution de 0,5 g du composé obtenu à l' étape B de l'Exemple 1 dans 20 ml de DCM, on ajoute, à TA et sous atmosphère d'azote, 0,23 g du composé obtenu à la Préparation 3.2 (base libre) puis 0,1 ml d'acide acétique et laisse 30 minutes sous agitation à TA. On ajoute ensuite 0,55 g de triacétoxyborohydrure de sodium et laisse une nuit sous agitation à TA. On ajoute au mélange réactionnel une solution à 10 % de $Na_2CO_3$ dans l'eau et laisse 15 minutes sous agitation à TA. On extrait le mélange réactionnel au DCM, lave la phase organique par une solution à 10 % de $Na_2CO_3$ dans l'eau, sèche sur $Na_2SO_4$, filtre et concentre sous vide le filtrat. On chromatographie le résidu sur gel de silice en éluant au DCM, puis par le gradient du mélange DCM/MeOH de (99/1 ; v/v) à (95/5 ; v/v). On dissout le produit obtenu dans du DCM, acidifie à pH = 1 par ajout d'éther chlorhydrique, essore le précipité formé, le lave à l'éther et le sèche sous vide. On obtient 0,4 g du produit attendu.

$\alpha_D^{20}$ = -37° (c = 1 ; MeOH) .

RMN $^1H$ : δ (ppm) : 0,6 à 2,3 : m : 18H ; 2,3 à 4,7 : m : 16H ; 6,4 à 8,0 : m : 8H.

EXEMPLE 3

Dichlorhydrate de 3-[2-[4-(1-N,N-diméthylcarbamoyl-1-méthyléthyl)pipérazin-1-yl]éthyl]-3-(3,4-dichlorophényl) -1-[2-(3,5-diméthylphényl)acétyl] pipéridine, 1,25 $H_2O$ isomère (-).

(I), 2HCl : X = $(CH_3)_2NCO\text{-}C(CH_3)_2\text{-}N\text{-}$ ; $R_1$ = Me ; Ar = 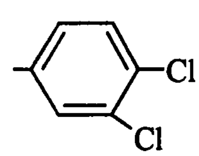

Dans 20 ml de MeOH, on ajoute à TA, 0,6 g du composé obtenu à l'étape B de l'Exemple 1, 0,3 g du composé obtenu à la Préparation 3.4, 0,1 ml d'acide acétique puis 0,12 g de cyanoborohydrure de sodium et laisse une nuit sous agitation à TA. On ajoute au mélange réactionnel une solution à 10 % de $Na_2CO_3$ dans l'eau et laisse 15 minutes sous agitation. On extrait le mélange à l'AcOEt, lave la phase organique par une solution à 10 % de $Na_2CO_3$ dans l'eau, à l'eau, par une solution saturée de NaCl, sèche sur $Na_2SO_4$ et évapore sous vide le solvant. On chromatographie le résidu sur gel de silice en éluant dans du DCM, puis par le gradient du mélange DCM/MeOH de (99/1 ; v/v) à (95/5 ; v/v). On dissout le produit obtenu dans du DCM, acidifie jusqu'à pH = 1 par ajout d'éther chlorhydrique, essore le précipité formé, le lave à l'éther et le sèche sous vide. On obtient 0,4 g du produit attendu.

$\alpha_D^{20}$ = - 28, 4° (c = 1 ; MeOH) .

RMN $^1H$ : δ (ppm) : 0,7 à 2,3 : m : 18H ; 2,35 à 4,7 : m : 22H ; 6,5 à 7,8 : m : 6H ; 10,3 : s : 1H.

EXEMPLE 4

Chlorhydrate de 3- [2- [4- (1-carbamoyl-1-méthyléthyl) pipéridin-1-yl]éthyl]-3-(3,4-dichlorophényl)-1-[2-(3,5-dichlorophényl)acétyl]pipéridine, sesquihydrate, isomère (-).

(I), HCl : X = $H_2NCO-C(CH_3)_2-CH-$ ; $R_1$ = Cl ; Ar = 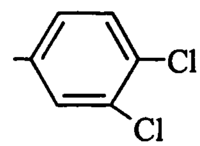

A) 3- (3, 4-Dichlorophényl) -3- (2-hydroxyéthyl) -1- [2- (3,5-dichlorophényl)acétyl] pipéridine, isomère unique.

Dans 150 ml de DCM on ajoute, à TA, 4,75 g du composé obtenu à la Préparation 1, 3,55 g du composé obtenu à la Préparation 2.1, 3,6 ml de triéthylamine puis 8,4 g de BOP et laisse 2 heures sous agitation à TA. On concentre sous vide le mélange réactionnel, extrait le résidu à l'AcOEt, lave la phase organique par une solution d'HCl 1N, à l'eau, par une solution de NaOH 1N, à l'eau, par une solution saturée de NaCl, sèche sur $Na_2SO_4$ et évapore sous vide le solvant. On obtient 8 g du produit attendu que l'on utilise tel quel.

B) 3-(3,4-Dichlorophényl)-3-(formylméthyl)-1-[2-(3,5-dichlorophényl)acétyl] pipéridine, isomère unique.

On prépare ce composé selon le mode opératoire décrit à l'étape B de l'Exemple 1 à partir de 0,25 ml de chlorure d'oxalyle dans 6 ml de DCM, 0,38 ml de DMSO dans 3 ml de DCM, 1 g du composé obtenu à l'étape précédente dans 6 ml de DCM puis 1,5 ml de triéthylamine. On obtient 1,0 g du produit attendu que l'on utilise tel quel.

C) Chlorhydrate de 3- [2- [4- (1-carbamoyl-1-méthyléthyl)pipéridin-1-yl]éthyl]-3-(3,4-dichlorophényl) -1- [2- (3, 5-dichlorophényl)acétyl]pipéridine, sesquihydrate, isomère (-) .

On prépare ce composé selon la mode opératoire décrit à l'étape C de l'Exemple 1 à partir de 0,25 g du composé obtenu à la Préparation 3.1 (base libre) dans 3 ml de MeOH, 0,08 ml d'acide acétique, 0,5 g du composé obtenu à l'étape précédente dans 5 ml de MeOH puis 0,08 g de cyanoborohydrure de sodium. On obtient 0,52 g du produit attendu.

$\alpha_D^{20}$ = - 0,6 ° (c = 1 ; MeOH ) .

EXEMPLE 5

Chlorhydrate de 3-[2-[4-(1-carbamoyl-1-méthyléthyl) pipéridin-1-yl]éthyl]-3-(3,4-dichlorophényl)-1-[2-[3,5-bis(trifluorométhyl)phényl]acétyl]pipéridine, monohydrate, isomère (+).

(I), HCl : X = $H_2NCO-C(CH_3)_2-CH-$ ; $R_1 = CF_3$ ; Ar = 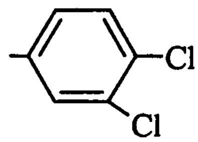

A) 3- (3, 4-Dichlorophényl) -3- (2-hydroxyéthyl) -1- [2- [3, 5-bis(trifluorométhyl)phényl] acétyl]pipéridine, isomère unique.

Dans 50 ml de DCM, on ajoute à TA, 1,2 g du composé obtenu à la Préparation 1, 1,2 g d'acide 3,5-bis (trifluorométhyl)phénylacétique, 1,7 ml de triéthylamine puis 2,16 g de BOP et laisse 15 minutes sous agitation. On concentre sous vide le mélange réactionnel, reprend le résidu par une solution d'HCl 1N, extrait à l'éther, lave la phase organique par une solution d'HCl 1N, à l'eau, par une solution de NaOH 1N, à l'eau, sèche sur $Na_2SO_4$ et évapore sous vide le solvant. On obtient 2,1 g du produit attendu que l'on utilise tel quel.

B) 3-(3,4-Dichlorophényl)-3-(formylméthyl)-1-[2-[3,5-bis(trifluorométhyl)phényl] acétyl]pipéridine, isomère unique.

On refroidit à -78°C 20 ml de DCM, ajoute, sous atmosphère d'azote, 1,5 g du composé obtenu à l'étape précédente, 0,45 ml de DMSO puis 0,3 ml de chlorure d'oxalyle et laisse 30 minutes sous agitation à -78°C.

On ajoute ensuite 2 ml de triéthylamine et laisse sous agitation en laissant remonter la température à TA. On ajoute au mélange réactionnel une solution d'HCl 1N, extrait au DCM, lave la phase organique par une solution d'HCl 1N, à l'eau, par une solution à 10 % de $Na_2CO_3$ dans l'eau, sèche sur $Na_2SO_4$ et évapore sous vide le solvant. On obtient 1,5 g du produit attendu que l'on utilise tel quel.

C) Chlorhydrate de 3- [2- [4- (1-carbamoyl-1-méthyléthyl)pipéridin-1-yl]éthyl]-3-(3,4-dichlorophényl)-1-[2-[3,5-bis(trifluorométhyl) phényl]acétyl]pipéridine, monohydrate, isomère (+).

On porte à reflux pendant 3 heures un mélange de 0,35 g du composé obtenu à la Préparation 3.1 et 0,4 g de $K_2CO_3$ dans 10 ml d'acétonitrile. On filtre un insoluble et concentre sous vide le filtrat. On dissout le produit de la Préparation 3.1 sous forme de base libre ainsi obtenu dans 3 ml de MeOH, ajoute 0,08 ml d'acide acétique puis une solution de 0,5 g du composé obtenu à l'étape précédente dans 5 ml de MeOH et laisse 5 minutes sous agitation à TA. On ajoute ensuite 0,08 g de cyanoborohydrure de sodium et laisse une nuit sous agitation à TA. On verse le mélange réactionnel sur une solution à 10 % de $NaHCO_3$ dans l'eau, extrait à l'éther, lave la phase organique à l'eau, sèche sur $Na_2SO_4$ et évapore sous vide le solvant. On chromatographie le résidu sur gel de silice H en éluant au DCM puis par le gradient du mélange DCM/MeOH de (99/1 ; v/v) à (90/10 ; v/v). On dissout le produit obtenu dans du DCM, acidifie à pH = 1 par ajout d'éther chlorhydrique et essore le précipité formé. On obtient 0,54 g du produit obtenu après séchage sous vide.

$\alpha_D^{20}$ = +28,2° (c = 1 ; MeOH).

RMN $^1H$ : $\delta$ (ppm) : 0,6 à 2, 2 : m : 16H ; 2,3 à 4, 2 : m : 12H ; 6,6 à 8,0 : m : 8H ; 10,3 : s : 1H.

EXEMPLE 6

Chlorhydrate de 3-[2-[4-(1-N,N-diméthylcarbamoyl-1-méthyléthyl)pipéridin-1-yl] éthyl]-3-(3,4-dichlorophényl)-1-[2-(3,5-diméthylphényl)acétyl] pipéridine, hémihydrate, isomère (-).

(I), HCl : X = $(CH_3)_2NCO-C(CH_3)_2-CH-$ ; $R_1$ = Me ; Ar = 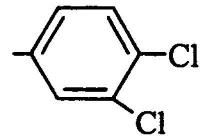

On porte à reflux pendant 3 heures un mélange de 0,35 g du composé obtenu à la Préparation 3.6 et 0,4 g de $K_2CO_3$ dans 10 ml d'acétonitrile. On filtre un insoluble et concentre sous vide le filtrat. On dissout le produit de la Préparation 3.6 sous forme de base libre ainsi obtenu dans 3 ml de MeOH, ajoute 0,1 ml d'acide acétique puis une solution de 0,6 g du composé obtenu à l'étape B de l'Exemple 1 dans 5 ml de MeOH et laisse 5 minutes sous agitation à TA. On ajoute ensuite 0,1 g de cyanoborohydrure de sodium et laisse une nuit sous agitation à TA. On verse le mélange réactionnel sur une solution à 10 % de $NaHCO_3$ dans l'eau, extrait à l'éther, lave la phase organique à l'eau, sèche sur $MgSO_4$ et évapore sous vide le solvant. On chromatographie le résidu sur gel de silice H en éluant au DCM puis par le gradient du mélange DCM/MeOH de (99/1 ; v/v) à (90/10 ; v/v). On dissout le produit obtenu dans du DCM, acidifie à pH = 1 par ajout d'éther chlorhydrique et concentre sous vide les solvants. On obtient 0,68 g du produit attendu après trituration dans l'éther, essorage et séchage, F = 202°C.

$\alpha_D^{20}$ = -27,1° (c = 1 ; MeOH) .

RMN $^1H$ : δ (ppm) : 0,6 à 2,5 : m : 23H ; 2,5 à 4,6 : m : 18H ; 6,4 à 7,8 : m : 6H ; 10,1 : s : 1H.

EXEMPLE 7

Chlorhydrate de 3- [2- [4- (1-carbamoyl-1-méthyléthyl) pipéridin-1-yl]éthyl]-3-(3,4-dichlorophényl)-1-[2-(3,5-diéthylphényl)acétyl]pipéridine, hémihydrate, isomère (-).

(I), HCl : X = $H_2NCO-C(CH_3)_2-CH-$ ; $R_1$ = Et ; Ar = 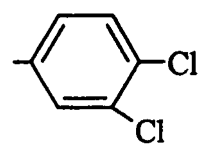

A) 3-(3,4-Dichlorophényl)-3-(2-hydroxyéthyl)-1-[2-(3,5-diéthylphényl)acétyl] pipéridine, isomère unique.

A un mélange de 1,64 g du composé obtenu à la Préparation 1 dans 30 ml de DCM, on ajoute à TA 1,15 g d'acide 3,5-diéthylphénylacétique, puis 3 ml de triéthylamine et 3,2 g de BOP et laisse 2 heures sous agitation à TA. On concentre sous vide le mélange réactionnel, reprend le résidu par une solution d'HCl 1N, extrait à l'éther, lave la phase organique par une solution d'HCl 1N, à l'eau, par une solution de NaOH 1N, à l'eau, par une solution saturée de NaCl, sèche sur $Na_2SO_4$ et évapore sous vide le solvant. On chromatographie le résidu sur gel de silice en éluant par le gradient du mélange DCM/MeOH de (99/1 ; v/v) à (95/5 ; v/v). On obtient 1,1 g du produit attendu que l'on utilise tel quel.

B) 3-(3,4-Dichlorophényl)-3-(formylméthyl)-1-[2-(3,5-diéthylphényl)acétyl] pipéridine, isomère unique.

On refroidit à -78°C, sous atmosphère d'azote, une solution de 0,5 g du composé obtenu à l'étape précédente dans 10 ml de DCM, ajoute 0,23 ml de DMSO puis 0,16 ml de chlorure d'oxalyle et laisse 30 minutes sous agitation à -78°C. On ajoute ensuite 0,95 ml de triéthylamine et laisse sous agitation en laissant remonter la température à TA. On ajoute une solution d'HCl 1N au mélange réactionnel, extrait au DCM, lave la phase organique par une solution d'HCl 1N, à l'eau, par une solution à 10 % de $Na_2CO_3$, sèche sur $Na_2SO_4$ et évapore sous vide le solvant. On obtient 0,5 g du produit attendu que l'on utilise tel quel.

C) Chlorhydrate de 3- [2- [4- (1-carbamoyl-1-méthyléthyl)pipéridin-1-yl] éthyl]-3-(3,4-dichloro phényl)-1-[2-(3,5-diéthylphényl)acétyl]pipéridine, hémihydrate, isomère (-).

A une solution de 0,23 g du composé obtenu à la Préparation 3.1 (base libre) dans 3 ml de MeOH, on ajoute à TA et sous atmosphère d'azote, 0,08 ml d'acide acétique puis une solution de 0,5 g du composé obtenu à l'étape précédente dans 5 ml de MeOH. Après 5 minutes, on ajoute 0,08 g de cyanoborohydrure de sodium et laisse une nuit sous agitation à TA. On verse le mélange réactionnel sur une solution à 10 % de $NaHCO_3$ dans l'eau, extrait à l'éther, lave la phase organique à l'eau, sèche sur $MgSO_4$ et évapore sous vide le solvant. On chromatographie le résidu sur gel de silice H en éluant au DCM puis par le gradient du mélange DCM/MeOH de (99/1 ; v/v) à (93/7 ; v/v). On dissout le produit obtenu dans du DCM, acidifie à pH = 1 par ajout d'éther chlorhydrique et concentre sous vide les solvants. On obtient 0,51 g du produit attendu après trituration dans l'éther, essorage et séchage.

$\alpha_D^{20}$ = -30,5° (c = 1 ; MeOH) .

RMN $^1H$ . δ (ppm) : 0,5 à 2, 2 : m : 23H ; 2,2 à 4, 65 : m : 16H ; 6, 4 à 7,8 : m : 8H ; 9,85 : s : 1H.

En procédant selon les modes opératoires décrits dans les Exemples précédents, on prépare les composés selon l'invention rassemblés dans le Tableau I ci-après.

TABLEAU I

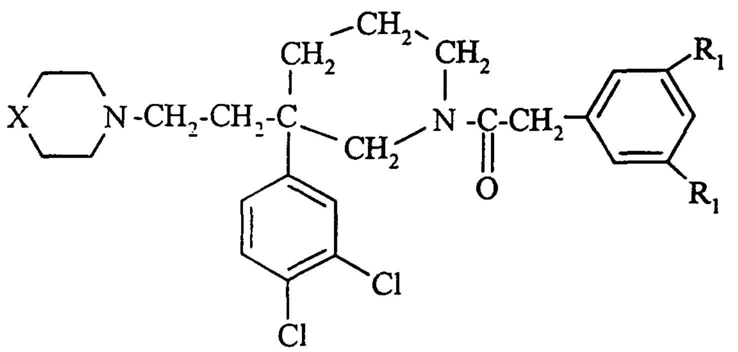

(I)

| Exemples | X | $R_1$ | Sel, hydrate RMN $\alpha_D^{20}$ (c = 1 ; MeOH) |
|---|---|---|---|
| 8<br>(a) | 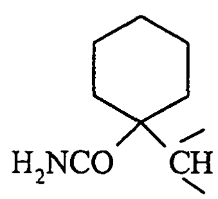 | Me | HCl<br>RMN<br>-23,3° |
| 9<br>(b) | 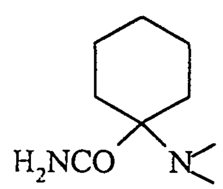 | Me | 2HCl, 1,5 $H_2O$<br>RMN<br>-26,6° |
| 10<br>(c) | 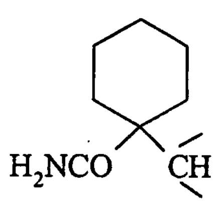 | Cl | HCl, 0,5 $H_2O$<br>RMN<br>-0,4° |

(suite)

| Exemples | X | $R_1$ | Sel, hydrate RMN $\alpha_D^{20}$ (c = 1 ; MeOH) |
|---|---|---|---|
| 11 (d) | 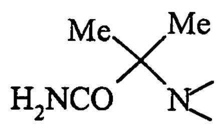 | Cl | 2HCl, 0,55 $H_2O$ RMN +32° |
| 12 (e) | 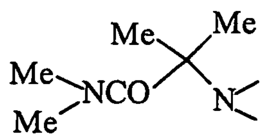 | Cl | 2HCl, 1,25 $H_2O$ RMN +2,4° |
| 13 (f) | 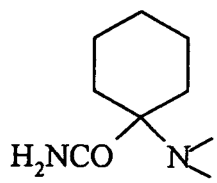 | Cl | 2HCl, 1,8 $H_2O$ RMN +28,4° |
| 14 (g) | 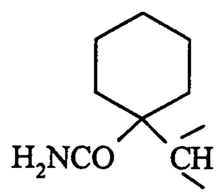 | $CF_3$ | HCl, 1,5 $H_2O$ RMN +25,7° |
| 15 (h) | 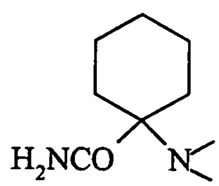 | $CF_3$ | 2HCl, 1,75 $H_2O$ RMN +25,2° |
| 16 (i) | 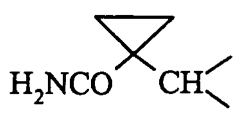 | Me | HCl, 1,6 $H_2O$ RMN -24,2° |
| 17 (j) | 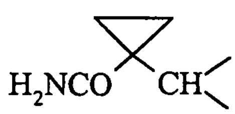 | $CF_3$ | HCl, 1,45 $H_2O$ RMN + 28,2° |
| 18 (k) | 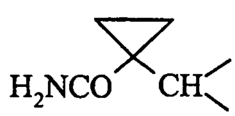 | Cl | HCl RMN + 37,2° |
| 19 (l) | 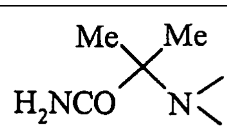 | Et | 2 HCl, 0,65 $H_2O$ RMN - 32,8 |

(suite)

| Exemples | X | $R_1$ | Sel, hydrate RMN $\alpha_D^{20}$ (c = 1 ; MeOH) |
|---|---|---|---|
| 20 (m) | 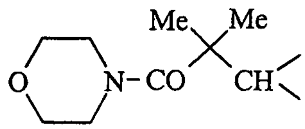 | Me | HCl<br>RMN<br>isomère unique |

On prépare ce composé selon le mode opératoire décrit à l'étape C de l'Exemple 1 à partir du composé obtenu à l'étape B de l'Exemple 1 et du composé obtenu à la Préparation 3.5 sous forme de base libre.
On prépare ce composé selon le mode opératoire décrit à l'Exemple 3 à partir du composé obtenu à l'étape B de l'Exemple 1 et du composé obtenu à la Préparation 3.3 sous forme de base libre.
On prépare ce composé selon le mode opératoire décrit à l'étape C de l'Exemple 4 à partir du composé obtenu à l'étape B de l'Exemple 4 et du composé obtenu à la Préparation 3.5 sous forme de base libre.
On prépare ce composé selon le mode opératoire décrit à l'Exemple 3 à partir du composé obtenu à l'étape B de l'Exemple 4 et du composé obtenu à la Préparation 3.2 sous forme de base libre.
On prépare ce composé selon le mode opératoire décrit à l'Exemple 3 à partir du composé obtenu à l'étape B de l'Exemple 4 et du composé obtenu à la Préparation 3.4.
On prépare ce composé selon le mode opératoire décrit à l'Exemple 3 à partir du composé obtenu à l' étape B de l'Exemple 4 et du composé obtenu à la Préparation 3.3 sous forme de base libre.
On prépare ce composé selon le mode opératoire décrit à l'étape C de l'Exemple 5 à partir du composé obtenu à l'étape B de l'Exemple 5 et du composé obtenu à la Préparation 3.5.
On prépare ce composé selon le mode opératoire décrit à l'Exemple 3 à partir du composé obtenu à l'étape B de l'Exemple 5 et du composé obtenu à la Préparation 3.3 sous forme de base libre.
On prépare ce composé selon le mode opératoire décrit à l'Exemple 2 à partir du composé obtenu à l'étape B de l'Exemple 1 et du composé obtenu à la Préparation 3.7 sous forme de base libre.
On prépare ce composé selon le mode opératoire décrit à l'étape C de l'Exemple 5 à partir du composé obtenu à l'étape B de l'Exemple 5 et du composé obtenu à la Préparation 3.7.
On prépare ce composé selon le mode opératoire décrit à l'étape C de l'Exemple 4 à partir du composé obtenu à l'étape B de l'Exemple 4 et du composé obtenu à la Préparation 3.7 sous forme de base libre.
On prépare ce composé selon le mode opératoire décrit à l'étape C de l'Exemple 7 à partir du composé obtenu à l'étape B de l'Exemple 7 et du composé obtenu à la Préparation 3.2 sous forme de base libre.
On prépare ce composé selon le mode opératoire décrit à l'étape C de l'Exemple 1 à partir du composé obtenu à l'étape B de l'Exemple 1 et du composé obtenu à la Préparation 3.8 sous forme de base libre.
Exemple 8 : RMN $^1H$ : δ (ppm) : 0,7 à 2,2 : m : 27H ; 2,3 à 4,6 : m : 14H ; 6,4 à 7,7 : m : 8H ; 10,1 : s : 1H.
Exemple 9 : RMN $^1H$ : δ (ppm) : 0,6 à 2,35 : m : 22H ; 2,4 à 4,6 : m : 14H ; 6,4 à 8,2 : m : 8H.
Exemple 10 : RMN $^1H$ : δ (ppm) : 0,7 à 2,25 : m : 21H ; 2,3 à 4,4 : m : 12H ; 6,7 à 7,8 : m : 8H ; 10,1 : s : 1H.
Exemple 11 : RMN $^1H$ : δ (ppm) : 0,6 à 2,2 : m : 12H ; 2,3 à 4,4 : m : 16H ; 6,8 à 8,0 : m : 8H.
Exemple 12 : RMN $^1H$ : δ (ppm) : 0,8 à 2,3 : m : 12H ; 2,35 à 4,4 : m : 22H ; 7,0 à 7,9 : m : 6H ; 10,6 : s : 1H.
Exemple 13 : RMN $^1H$ : δ (ppm) : 0,9 à 2,3 : m : 16H ; 2,35 à 4,5 : m : 16H ; 7,0 à 7,9 : m : 8H.
Exemple 14 : RMN $^1H$ : δ (ppm) : 0,9 à 2,3 : m : 21H ; 2,4 à 4,3 : m : 12H ; 6,8 à 8,1 : m : 8H ; 10,0 : s : 1H.
Exemple 15 : RMN $^1H$ : δ (ppm) : 1,0 à 2,4 : m : 16H ; 2,5 à 4,5 : m : 16H ; 6, 9 à 8,1 : m : 8H ; 11,0 : se : 1H.
Exemple 16 : RMN $^1H$ : δ (ppm) : 0,4 à 2,3 : m : 20H ; 2,4 à 4,6 : m : 13H ; 6,5 à 7,7 : m : 8H ; 9,6 : s : 1H.
Exemple 17 : RMN $^1H$ : δ (ppm) : 0,4 à 2,2 : m : 14H ; 2,3 à 4,4 : m : 13H ; 6,5 à 7,8 : m : 8H ; 9,9 : s : 1H.
Exemple 18 : RMN $^1H$ : δ (ppm) : 0,4 à 2,2 : m : 14H ; 2,3 à 4,4 : m : 13H ; 6,6 à 7,8 : m : 8H ; 9,9 : s : 1H.
Exemple 19 : RMN $^1H$ : δ (ppm) : 0,6 à 2,6 : m : 22H ; 2,6 à 4,8 : m : 16H ; 6,5 à 8,0 : m : 10H.
Exemple 20 : RMN $^1H$ : δ (ppm) : 0,7 à 2,25 : m : 22H ; 2,3 à 4,6 : m : 21H ; 6,4 à 7,7 : m : 6H ; 10,4 : s : 1H.

EXEMPLE 21

Chlorhydrate de 3-[2-[4-(1-carbamoyl-1-méthyléthyl) pipéridin-1-yl]éthyl]-3-(3,4-diméthylphényl)-1-[2-(3,5-dichlorophényl)acétyl]pipéridine.

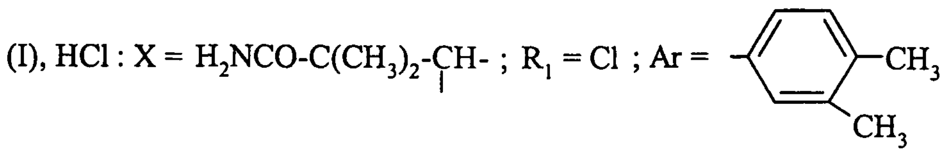

A)1-[2-(3,5-Dichlorophényl)acétyl]-3-(3,4-diméthylphényl)-3-[2-(tétrahydropyran-2-yloxy)éthyl]pipéridine.

On laisse 2 heures sous agitation à TA un mélange de 3 g du composé obtenu à la Préparation 1.2, 1,3 g du composé obtenu à la Préparation 2.1, 3,2 ml de triéthylamine et 4,8 g de BOP dans 100 ml de DCM. On concentre sous vide le mélange réactionnel, reprend le résidu par une solution d'HCl 1N, extrait à l'AcOEt, lave la phase organique à l'eau, par une solution de NaOH 1N, par une solution saturée de NaCl, sèche sur $Na_2SO_4$ et évapore sous vide le solvant. On obtient 4,5 g du produit attendu.

B)1-[2-(3,5-Dichlorophényl)acétyl]-3-(3,4-diméthylphényl)-3-(2-hydroxyéthyl) pipéridine.

On laisse 2 heures sous agitation à TA un mélange de 4,5 g du composé obtenu à l'étape précédente et 2 ml d'une solution d'HCl concentré dans 10 ml de MeOH. On concentre sous vide le mélange réactionnel, reprend le résidu dans le MeOH et évapore sous vide le solvant. On chromatographie le résidu sur gel de silice en éluant au DCM puis par le gradient du mélange DCM/MeOH de (99/1 ; v/v) à (95/5 ; v/v). On obtient 3 g du produit attendu.

C) 1-[2-(3,5-Dichlorophényl)acétyl]-3-(formylméthyl)-3-(3,4-diméthylphényl) pipéridine.

On refroidit à - 78°C 10 ml de DCM, ajoute, sous atmosphère d'azote, 0,5 g du composé obtenu à l'étape précédente, 0,18 ml de DMSO puis 0,13 ml de chlorure d'oxalyle et laisse 30 minutes sous agitation à - 78°C. On ajoute ensuite 0,75 ml de triéthylamine et laisse sous agitation en laissant remonter la température à TA. On ajoute au mélange réactionnel une solution d'HCl 1N, extrait au DCM, lave la phase organique à l'eau, par une solution à 10 % de $Na_2CO_3$, sèche sur $Na_2SO_4$ et évapore sous vide le solvant. On obtient 0,5 g du produit attendu.

D) Chlorhydrate de 3- [2-[4-(1-carbamoyl-1-méthyléthyl)pipéridin-1-yl]éthyl]-3-(3,4-diméthylphényl)-1-[2-(3,5-dichlorophényl)acétyl]pipéridine.

On laisse une nuit sous agitation à TA, un mélange de 0,5 g du composé obtenu à l'étape précédente, 0,35 g du composé obtenu à la Préparation 3.1 (base libre), 0,1 ml d'acide acétique et 0,15 g de cyanoborohydrure de sodium dans 30 ml de MeOH. On ajoute une solution à 10 % de $Na_2CO_3$ au mélange réactionnel, laisse 15 minutes sous agitation, extrait à l'AcOEt, lave la phase organique à l'eau, par une solution saturée de NaCl, sèche sur $Na_2SO_4$ et évapore sous vide le solvant. On chromatographie le résidu sur gel de silice en éluant au DCM puis par le gradient du mélange DCM/MeOH (99/1 ; v/v) à (95/5 ; v/v). On dissout le produit obtenu dans du DCM, acidifie à pH = 1 par ajout d'éther chlorhydrique et essore le précipité formé. On obtient 0,35 g du produit attendu.
RMN $^1H$ : δ (ppm) : 0,8 à 2,3 : m : 22H ; 2,3 à 4,0 : m : 13H ; 6,5 à 7,6 : m : 8H ; 9,5 : s : 1H.

EXEMPLE 22

Dichlorhydratede3-[2-[4-(1-carbamoyl-1-méthyléthyl)pipérazin-1-yl]éthyl]-3-(3,4-diméthylphényl)-1-[2-(3,5-dichlorophényl)acétyl]pipéridine, 1 $H_2O$.

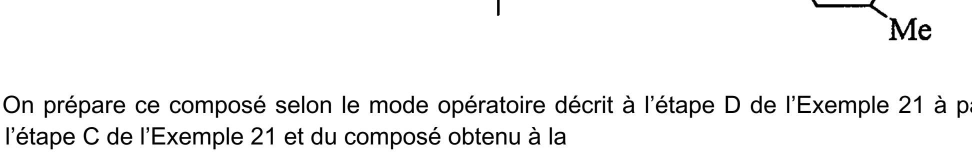

On prépare ce composé selon le mode opératoire décrit à l'étape D de l'Exemple 21 à partir du composé obtenu à l'étape C de l'Exemple 21 et du composé obtenu à la
Préparation 3.2 (base libre).

RMN $^1H$ : δ (ppm) : 1,4 : 1s : 6H ; 2,2 : 2s : 6H ; 1,3 à 4,0 : m : 26H ;7,0 à 8,0 : m : 6H.

**Revendications**

1. Composé de formule :

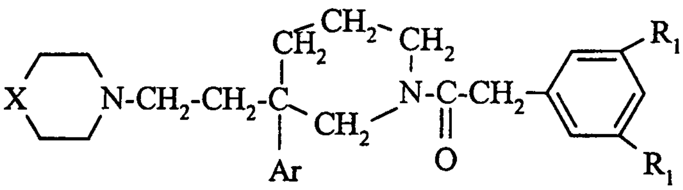

(I)

dans laquelle .

- X représente un groupe

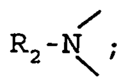

un groupe

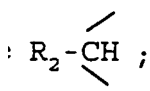

- Ar représente un phényle monosubstitué ou disubstitué par un atome d'halogène ; un $(C_1-C_3)$alkyle ;
- $R_1$ représente un atome de chlore, un atome de brome, un $(C_1-C_3)$alkyle ou un trifluorométhyle ;
- $R_2$ représente un groupe $-CR_3R_4CONR_5R_6$ ;
- $R_3$ et $R_4$ représentent le même radical choisi parmi un méthyle, un éthyle, un n-propyle ou un n-butyle ;
- ou bien $R_3$ et $R_4$ ensemble avec l'atome de carbone auquel ils sont liés constituent un $(C_3-C_6)$cycloalkyle ;
- $R_5$ et $R_6$ représentent chacun indépendamment un hydrogène ; un $(C_1-C_3)$alkyle;
- ou bien $R_5$ et $R_6$ ensemble avec l'atome d'azote auquel ils sont liés constituent un radical hétérocyclique choisi parmi l'azétidin-1-yle, la pyrrolidin-1-yle, la pipéridin-1-yle, la morpholin-4-yle, la thiomorpholin-4-yle ou la perhydroazépin-1-yle ;

et ses sels avec des acides minéraux ou organiques, ses solvats et/ou ses hydrates.

2. Composé selon la revendication 1 dans lequel Ar représente un 3,4-dichlorophényle ou un 3,4-diméthylphényle.

3. Composé selon la revendication 1 dans lequel les substituants $R_1$ représentent un atome de chlore, un méthyle, un éthyle ou un trifluorométhyle.

4. Composé selon la revendication 1 dans lequel X représente un groupe

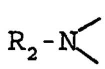

dans lequel $R_2$ représente un groupe $-CR_3R_4CONR_5R_6$.

5. Composé selon la revendication 4 dans lequel $R_3$ et $R_4$ représentent chacun un méthyle ou bien, ensemble avec l'atome de carbone auquel ils sont liés, constituent un cyclohexyle.

**6.** Composé selon la revendication 1 dans lequel X représente un groupe

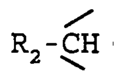

dans lequel $R_2$ représente un groupe $-CR_3R_4CONR_5R_6$.

**7.** Composé selon la revendication 6 dans lequel $R_3$ et $R_4$ représentent chacun un méthyle ou bien, ensemble avec l'atome de carbone auquel ils sont liés, constituent un cyclohexyle ou un cyclopropyle.

**8.** Composé selon la revendication 4 ou la revendication 6 dans lequel $R_5$ et $R_6$ représentent chacun l'hydrogène ou un méthyle.

**9.** Composé selon la revendication 1 de formule :

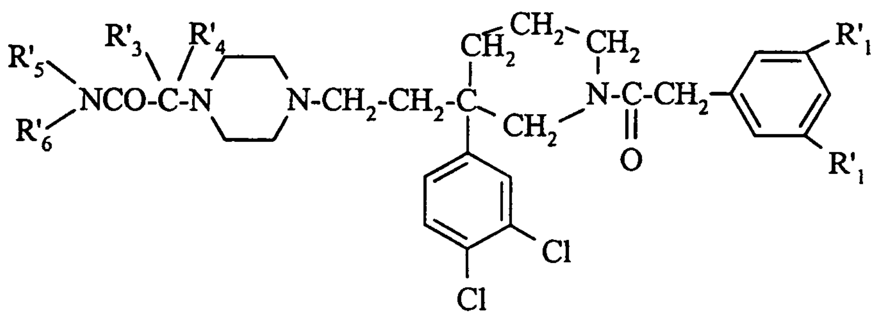

(I')

dans laquelle :

- $R'_1$ représente un atome de chlore, un méthyle, un éthyle ou un trifluorométhyle ;
- $R'_3$ et $R'_4$ représentent chacun un méthyle ou bien, ensemble avec l'atome de carbone auquel ils sont liés constituent un cyclohexyle ;
- $R'_5$ et $R'_6$ représentent chacun l'hydrogène ou un méthyle ; et ses sels avec des acides minéraux ou organiques, ses solvats et/ou ses hydrates.

**10.** Composé selon la revendication 1 de formule :

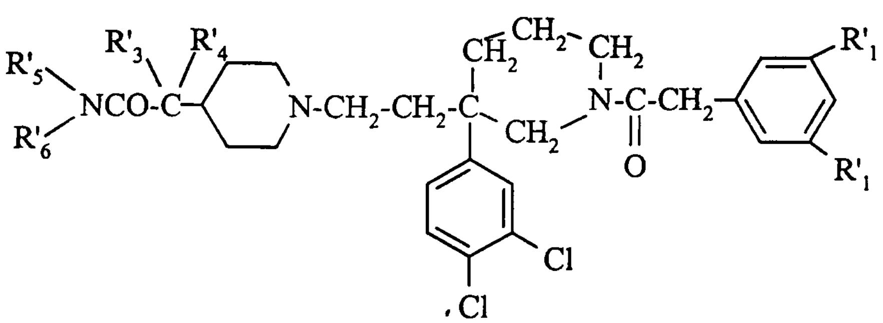

(I")

dans laquelle :

- $R'_1$ représente un atome de chlore, un méthyle, un éthyle ou un trifluorométhyle ;
- $R'_3$ et $R'_4$ représentent chacun un méthyle ou bien, ensemble avec l'atome de carbone auquel ils sont liés, constituent un cyclohexyle ou un cyclopropyle ;
- $R'_5$ et $R'_6$ représentent chacun l'hydrogène ou un méthyle ; et ses sels avec des acides minéraux ou organiques, ses solvats et/ou ses hydrates.

**11.** Composé selon l'une quelconque des revendications 1 à 10 de formule (I), (I'), (I") sous forme optiquement pure.

**12.** La 3-[2-[4-(1-carbamoyl-1-méthyléthyl)pipéridin-1-yl]éthyl]-3-(3,4-dichlorophényl)-1-[2-(3,5-diméthylphényl)acétyl] pipéridine, isomère (-), ses sels, ses solvats et/ou ses hydrates.

**13.** La 3- [2- [4- (1-N,N-diméthylcarbamoyl-1-méthyléthyl) pipéridin-1-yl]éthyl]-3-(3,4-dichlorophényl)-1-[2-(3,5-diméthylphényl)acétyl]pipéridine, isomère (-), ses sels, ses solvats et/ou ses hydrates

**14.** La 3- [2- [4- (1-carbamoyl-1-méthyléthyl) pipéridin-1-yl]éthyl]-3-(3,4-dichlorophényl)-1-[2-(3,5-diéthylphényl)acétyl] pipéridine, isomère (-), ses sels, ses solvats et/ou ses hydrates.

**15.** La 3- [2- [4- (1-carbamoyl-1-méthyléthyl) pipéridin-1-yl]éthyl]-3-(3,4-dichlorophényl)-1-[2-[3,5-bis(trifluorométhyl) phényl]acétyl]pipéridine, isomère (+), ses sels, ses solvats et/ou ses hydrates.

**16.** Procédé pour la préparation des composés de formule (I) selon la revendication 1, de leurs sels, leurs solvats et/ou leurs hydrates, **caractérisé en ce que** :

1a) on traite un composé de formule :

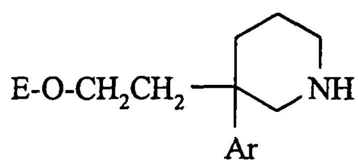

(II)

dans laquelle Ar est tel que défini pour un composé de formule (I) dans la revendication 1 et E représente l'hydrogène ou un groupe O-protecteur, avec un dérivé fonctionnel d'un acide de formule :

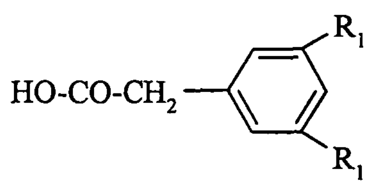

(III)

dans laquelle $R_1$ est tel que défini pour un composé de formule (I) dans la revendication 1, pour obtenir un composé de formule :

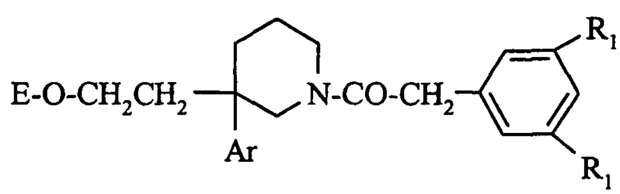

(IV) ;

2a) éventuellement, lorsque E représente un groupe protecteur, on l'élimine par action d'un acide ou d'une base, pour obtenir l'alcool de formule :

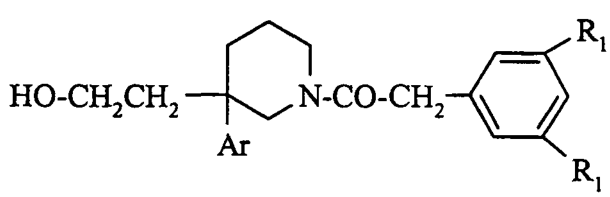

(IV, E = H) ;

3a) on traite l'alcool obtenu à l'étape 1a) ou à l'étape 2a) de formule (IV, E = H) avec un composé de formule :

$$Y\text{-}SO_2\text{-}Cl \qquad (V)$$

dans laquelle Y représente un groupe méthyle, phényle, tolyle, trifluorométhyle, pour obtenir un composé de formule :

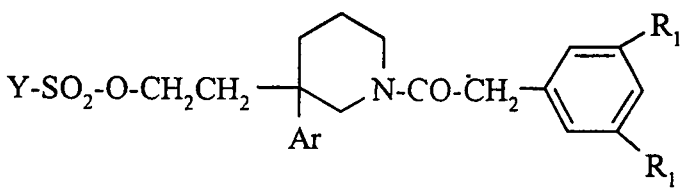

(VI) ;

4a) on fait réagir le composé de formule (VI) avec un composé de formule :

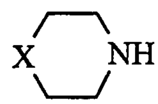

(VII)

dans laquelle X est tel que défini pour un composé de formule (I) dans la revendication 1;
5a) et, éventuellement, on transforme le composé ainsi obtenu en l'un de ses sels avec un acide minéral ou organique.

**17.** Procédé pour la préparation des composés de formule (I) selon la revendication 1, de leurs sels, leurs solvats et/ou leurs hydrates, **caractérisé en ce que** :

1b) on traite le composé de formule :

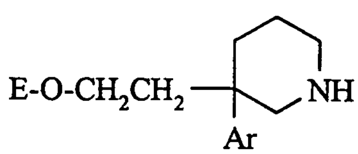

(II)

dans laquelle Ar est tel que défini pour un composé de formule (I) dans la revendication 1 et E représente l'hydrogène ou un groupe O-protecteur, avec un dérivé fonctionnel d'un acide de formule :

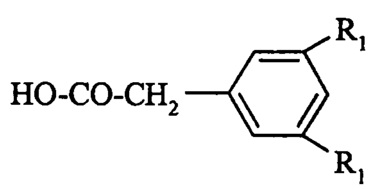

(III)

dans laquelle $R_1$ est tel que défini pour un composé de formule (I) dans la revendication 1, pour obtenir un composé de formule :

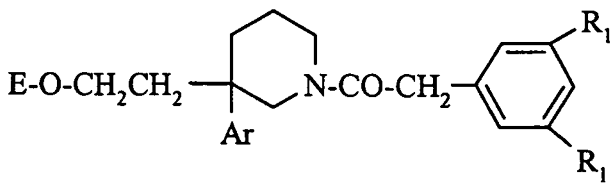

(IV) ;

éventuellement, lorsque E représente un groupe protecteur, on l'élimine par action d'un acide ou d'une base, pour obtenir l'alcool de formule :

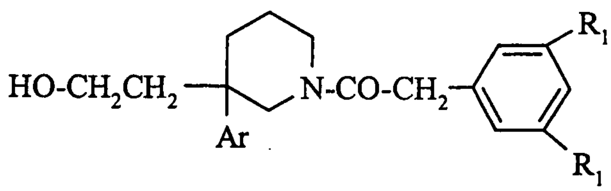

(IV, E = H) ;

2b) on oxyde le composé de formule (IV, E = H) ainsi obtenu pour préparer un composé de formule :

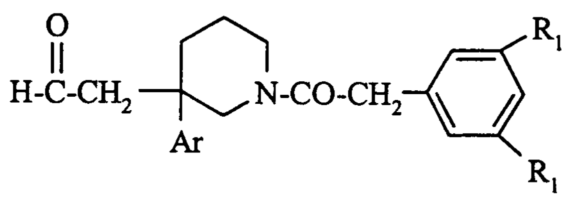

(VIII)

3b) on fait réagir le composé de formule (VIII) avec un composé de formule :

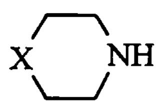

(VII)

dans laquelle X est tel que défini pour un composé de formule (I) dans la revendication 1, en présence d'un acide, puis on réduit le sel d'imminium formé intermédiairement au moyen d'un agent réducteur ;

4b) et, éventuellement, on transforme-le composé ainsi obtenu en un de ses sels avec un acide minéral ou organique.

**18.** Procédé stéréospécifique pour la préparation des composés de formule (I) selon la revendication 1, ayant la configuration (S), de leurs sels, de leurs solvats et/ou hydrates, **caractérisé en ce que** :

1d) on traite l'isomère (S) d'un composé de formule :

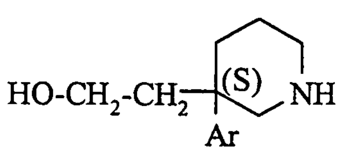

(II*, E=H)

dans laquelle Ar est tel que défini pour un composé de formule (I) dans la revendication 1, avec un dérivé fonctionnel de l'acide de formule :

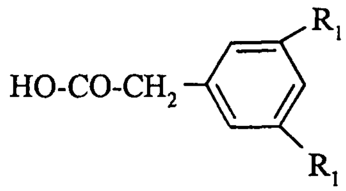

(III)

dans laquelle $R_1$ est tel que défini pour un composé de formule (I) dans la revendication 1, pour obtenir un composé de formule :

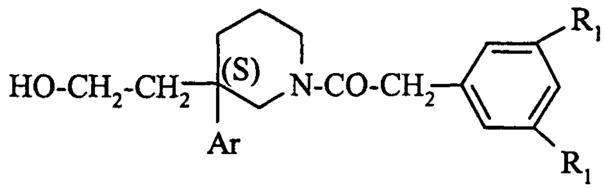

(IV*, E=H) ;

2d) on oxyde le composé de formule (IV*) pour obtenir un composé de formule:

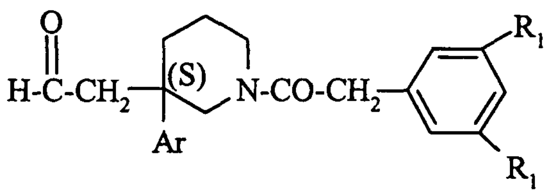

(VIII*) ;

3d) on fait réagir le composé de formule (VIII*) avec un composé de formule :

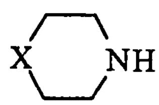

(VII) ;

dans laquelle X est tel que défini pour un composé de formule (I) dans la revendication 1,
en présence d'un acide, puis on réduit le sel d'iminium formé intermédiairement au moyen d'un agent réducteur ;
4d) et, éventuellement on transforme le composé ainsi obtenu en l'un de ses sels avec un acide minéral ou organique.

**19.** Procédé pour la préparation des composés de formule (I) selon la revendication 1, de leurs sels, leurs solvats et/ou hydrates, **caractérisé en ce que** :

on fait réagir un composé de formule :

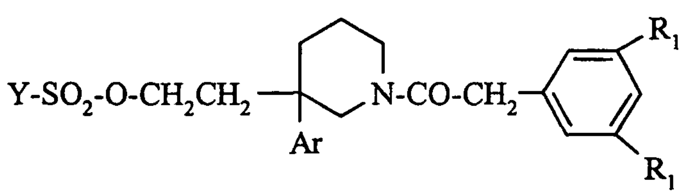

(VI) ;

dans laquelle :

- Ar représente un phényle monosubstitué ou disubstitué par un atome d'halogène ; un ($C_1$-$C_3$)alkyle ;

- Y représente un groupe méthyle, phényle, tolyle ou trifluorométhyle ;
- $R_1$ représente un atome de chlore, un atome de brome, un $(C_1-C_3)$alkyle ou un trifluorométhyle ;

avec un composé de formule :

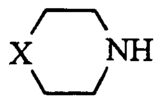 (VII)

dans laquelle X est tel que défini pour un composé de formule (I) dans la revendication 1, et, éventuellement, on transforme le composé ainsi obtenu en un de ses sels avec un acide minéral ou organique.

**20.** Procédé pour la préparation des composés de formule (I) selon la revendication 1, de leurs sels, leurs solvats et/ou leurs hydrates, **caractérisé en ce que** :

on fait réagir le composé de formule :

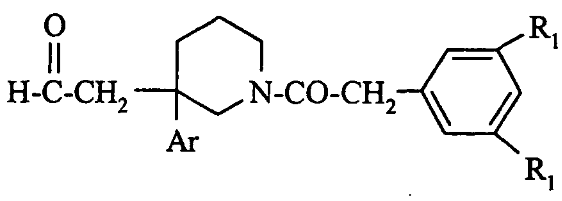 (VIII)

dans laquelle Ar et $R_1$ sont tels que définis pour un composé de formule (I) dans la revendication 1, avec un composé de formule :

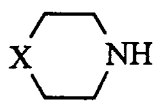 (VII)

dans laquelle X est tel que défini pour un composé de formule (I) dans la revendication 1, en présence d'un acide, puis on réduit le sel d'imminium formé intermédiairement au moyen d'un agent réducteur, et, éventuellement, on transforme le composé ainsi obtenu en un de ses sels avec un acide minéral ou organique.

**21.** Procédé stéréospécifique pour la préparation des composés de formule (I) selon la revendication 1, ayant la configuration (S), de leurs sels, leurs solvats et/ou leurs hydrates, **caractérisé en ce que** :

on fait réagir le composé de formule :

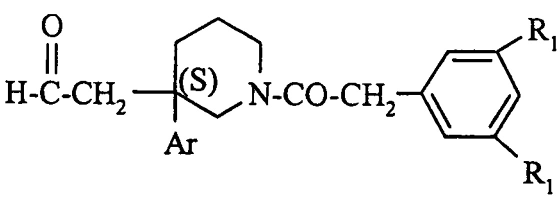 (VIII*)

dans laquelle Ar et $R_1$ sont tels que définis pour un composé de formule (I) dans la revendication 1, avec un composé de formule :

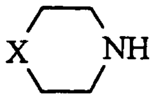 (VII)

dans laquelle X est tel que défini pour un composé de formule (I) dans la revendication 1, en présence d'un acide, puis on réduit le sel d'imminium formé intermédiairement au moyen d'un agent réducteur, et, éventuellement, on transforme le composé ainsi obtenu en un de ses sels avec un acide minéral ou organique.

**22.** Composé de formule :

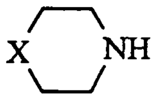 (VII)

dans laquelle :

- X représente un groupe

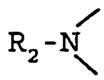 ;

un groupe

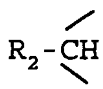 ;

- $R_2$ représente un groupe $-CR_3R_4CONR_5R_6$ ;
- $R_3$ et $R_4$ représentent le même radical choisi parmi un méthyle, un éthyle, un n-propyle ou un n-butyle ;
- ou bien $R_3$ et $R_4$ ensemble avec l'atome de carbone auquel ils sont liés constituent un $(C_3\text{-}C_6)$cycloalkyle ;
- $R_5$ et $R_6$ représentent chacun indépendamment un hydrogène ; un $(C_1\text{-}C_3)$alkyle
- ou bien $R_5$ et $R_6$ ensemble avec l'atome d'azote auquel ils sont liés constituent un radical hétérocyclique choisi parmi l'azétidin-1-yle, la pyrrolidin-1-yle, la pipéridin-1-yle, la morpholin-4-yle, la thiomorpholin-4-yle ou la perhydroazépin-1-yle ;

et ses sels avec des acides minéraux ou organiques.

**23.** Composition pharmaceutique comprenant en tant que principe actif un composé selon l'une quelconque des revendications 1 à 15 ou l'un de leurs sels, solvats et/ou hydrates pharmaceutiquement acceptables.

**24.** Composition pharmaceutique selon la revendication 23, contenant de 0,1 à 1000 mg de principe actif, sous forme d'unité de dosage, dans laquelle le principe actif est mélangée à au moins un excipient pharmaceutique.

**25.** Utilisation d'un composé selon l'une quelconque des revendications 1 à 15 ou de l'un de ses sels, solvats et/ou hydrates pharmaceutiquement acceptables pour la préparation de médicaments destinés à traiter toute pathologie où la Substance P et les récepteurs $NK_1$ humains sont impliqués.

**26.** Utilisation selon la revendication 25, pour la préparation de médicaments destinés à traiter les pathologies du système respiratoire, gastro-intestinal, urinaire, immunitaire, cardiovasculaire, du système nerveux central ainsi que la douleur, la migraine, les inflammations, les nausées et les vomissements, les maladies de la peau.

**27.** Utilisation selon la revendication 26 pour la préparation de médicaments destinés à traiter la bronchite chronique obstructive, l'asthme, l'incontinence urinaire, le syndrome du colon irritable, la maladie de Crohn, les colites ulcératives, la dépression, l'anxiété.

## Claims

1. Compound of formula:

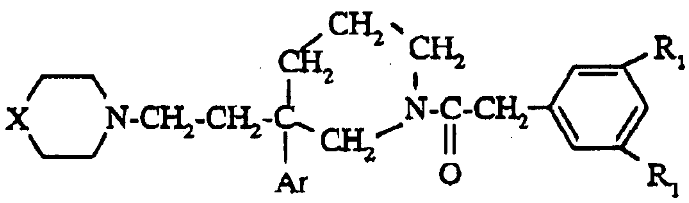

(I)

in which:

- X represents a group

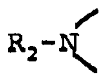

;

a group

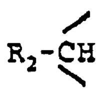

;

- Ar represents a phenyl monosubstituted or disubstituted with a halogen atom; a $(C_1-C_3)$alkyl;
- $R_1$ represents a chlorine atom, a bromine atom, a $(C_1-C_3)$ alkyl or a trifluoromethyl;
- $R_2$ represents a group $-CR_3R_4CONR_5R_6$;
- $R_3$ and $R_4$ represent the same radical chosen from a methyl, an ethyl, an n-propyl or an n-butyl;
- or alternatively $R_3$ and $R_4$, together with the carbon atom to which they are attached, constitute a $(C_3-C_6)$ cycloalkyl;
- $R_5$ and $R_6$ each independently represent a hydrogen; a $(C_1-C_3)$alkyl;
- or alternatively $R_5$ and $R_6$, together with the nitrogen atom to which they are attached, constitute a heterocyclic radical chosen from 1-azetidinyl, 1-pyrrolidinyl, 1-piperidyl, 4-morpholinyl, 4-thiomorpholinyl or perhydro-1-azepinyl;

and the salts thereof with inorganic or organic acids, and the solvates and/or hydrates thereof.

2. Compound according to Claim 1, in which Ar represents a 3,4-dichlorophenyl or a 3,4-dimethylphenyl.

3. Compound according to Claim 1, in which the substituents $R_1$ represent a chlorine atom, a methyl, an ethyl or a trifluoromethyl.

4. Compound according to Claim 1, in which X represents a group

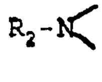

in which $R_2$ represents a group $-CR_3R_4CONR_5R_6$.

**5.** Compound according to Claim 4, in which $R_3$ and $R_4$ each represent a methyl or, together with the carbon atom to which they are attached, constitute a cyclohexyl.

**6.** Compound according to Claim 1, in which X represents a group

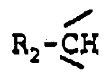

in which $R_2$ represents a group $-CR_3R_4CONR_5R_6$.

**7.** Compound according to Claim 6, in which $R_3$ and $R_4$ each represent a methyl or, together with the carbon atom to which they are attached, constitute a cyclohexyl or a cyclopropyl.

**8.** Compound according to Claim 4 or Claim 6, in which $R_5$ and $R_6$ each represent hydrogen or a methyl.

**9.** Compound according to Claim 1, of formula:

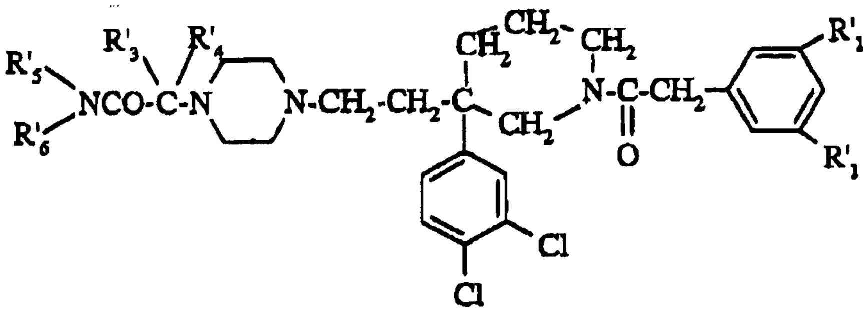

(I')

in which:

- $R'_1$ represents a chlorine atom, a methyl, an ethyl or a trifluoromethyl;
- $R'_3$ and $R'_4$ each represent a methyl or alternatively, together with the carbon atom to which they are attached, constitute a cyclohexyl;
- $R'_5$ and $R'_6$ each represent hydrogen or a methyl; and the salts thereof with inorganic or organic acids, and the solvates and/or hydrates thereof.

**10.** Compound according to Claim 1, of formula:

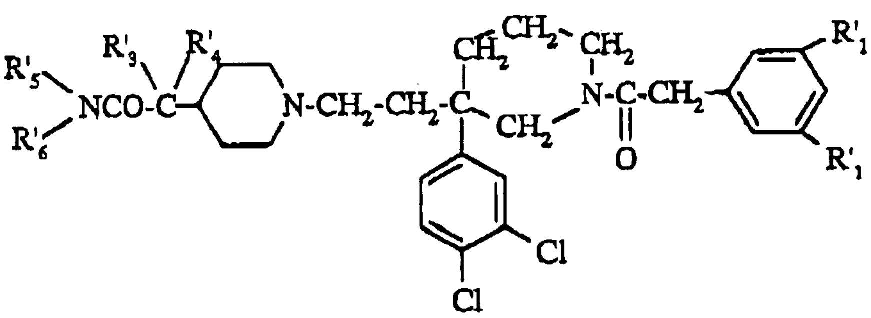

(I")

in which:

- $R'_1$ represents a chlorine atom, a methyl, an ethyl or a trifluoromethyl;
- $R'_3$ and $R'_4$ each represent a methyl or alternatively, together with the carbon atom to which they are attached, constitute a cyclohexyl or cyclopropyl;

- $R'_5$ and $R'_6$ each represent hydrogen or a methyl; and the salts thereof with inorganic or organic acids, and the solvates and/or hydrates thereof.

**11.** Compound according to any one of Claims 1 to 10, of formula (I), (I') or (I" ), in optically pure form.

**12.** 3- [2- [4- (1-Carbamoyl-1-methylethyl)-1-piperidyl] ethyl] -3- (3, 4-dichlorophenyl) -1- [2-(3, 5-dimethylphenyl) acetyl] piperidine, (-) isomer, the salts thereof and the solvates and/or hydrates thereof.

**13.** 3-[2-[4-(1-N,N-dimethylcarbamoyl-1-methylethyl)-1-piperidyl]ethyl]-3-(3,4-dichlorophenyl)-1- [2-(3,5-dimethylphenyl)acetyl]piperidine, (-) isomer, the salts thereof and the solvates and/or hydrates thereof.

**14.** 3-[2-[4-(1-Carbamoyl-1-methylethyl)-1-piperidyl] ethyl] -3- (3, 4-dichlorophenyl) -1- [2-(3,5-diethylphenyl)acetyl]piperidine, (-) isomer, the salts thereof and the solvates and/or hydrates thereof.

**15.** 3- [2- [4- (1-Carbamoyl-1-methylethyl) - 1-piperidyl] ethyl] -3- (3, 4-dichlorophenyl) -1- [2-[3, 5-bis(trifluoromethyl) phenyl] acetyl] piperidine, (+) isomer, the salts thereof and the solvates and/or hydrates thereof.

**16.** Process for preparing the compounds of formula (I) according to Claim 1, the salts thereof and the solvates and/or hydrates thereof, **characterized in that**:

1a) a compound of formula:

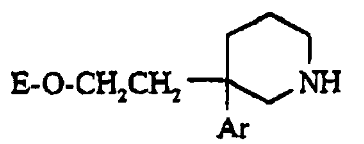

(II)

in which Ar is as defined for a compound of formula (I) in Claim 1 and E represents hydrogen or an O-protecting group, is treated with a functional derivative of an acid of formula:

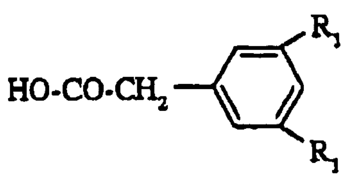

(III)

in which $R_1$ is as defined for a compound of formula (I) in Claim 1, to give a compound of formula:

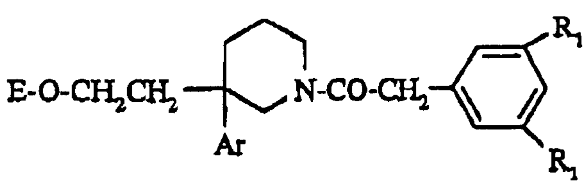

(IV) ;

2a) optionally, when E represents a protecting group, it is removed by the action of an acid or a base, to give the alcohol of formula:

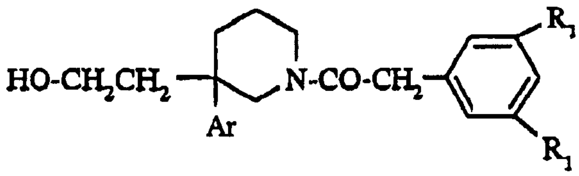

(IV, E = H) ;

3a) the alcohol obtained in step 1a) or in step 2a) of formula (IV, E = H) is treated with a compound of formula:

$Y\text{-}SO_2\text{-}Cl$ (V)

in which Y represents a methyl, phenyl, tolyl or trifluoromethyl group, to give a compound of formula:

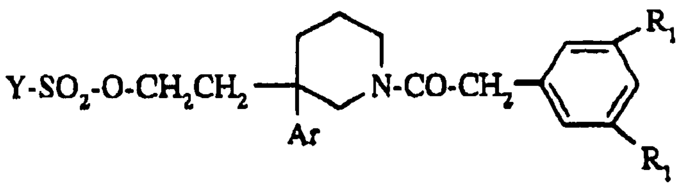

(VI) ;

4a) the compound of formula (VI) is reacted with a compound of formula:

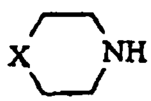

(VII)

in which X is as defined for a compound of formula (I) in Claim 1;
5a) and, optionally, the compound thus obtained is converted into one of the salts thereof with an inorganic or organic acid.

**17.** Process for preparing the compounds of formula (I) according to Claim 1, the salts thereof and the solvates and/or hydrates thereof, **characterized in that**:

1b) the compound of formula:

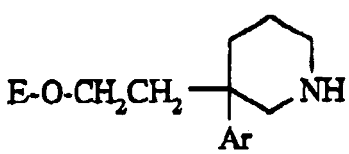

(II)

in which Ar is as defined for a compound of formula (I) in Claim 1 and E represents hydrogen or an O-protecting group, is treated with a functional derivative of an acid of formula:

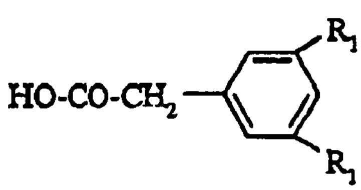

(III)

in which $R_1$ is as defined for a compound of formula (I) in Claim 1, to give a compound of formula:

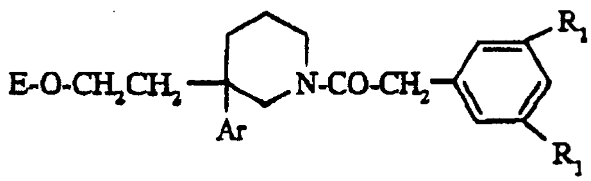 (IV) ;

optionally, when E represents a protecting group, it is removed by the action of an acid or a base, to give the alcohol of formula:

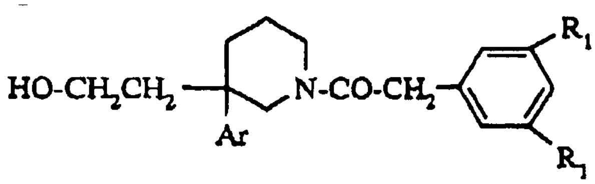 (IV, E = H) ;

2b) the compound of formula (IV, E = H) thus obtained is oxidized in order to prepare a compound of formula:

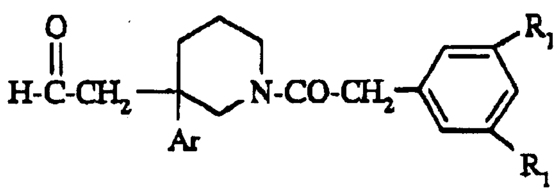 (VIII)

3b) the compound of formula (VIII) is reacted with a compound of formula:

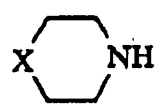 (VII)

in which X is as defined for a compound of formula (I) in Claim 1, in the presence of an acid, followed by reduction of the intermediate iminium salt formed by means of a reducing agent;
4b) and, optionally, the compound thus obtained is converted into one of the salts thereof with an inorganic or organic acid.

**18.** Stereospecific process for preparing the compounds of formula (I) according to Claim 1 having the (S) configuration, the salts thereof and the solvates and/or hydrates thereof, **characterized in that**:

1d) the (S) isomer of a compound of formula:

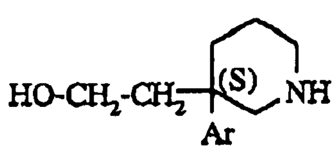 (II*, E=H)

in which Ar is as defined for a compound of formula (I) in Claim 1, is treated with a functional derivative of the acid of formula:

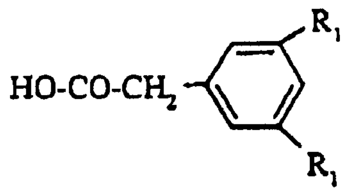 (III)

in which $R_1$ is as defined for a compound of formula (I) in Claim 1, to give a compound of formula:

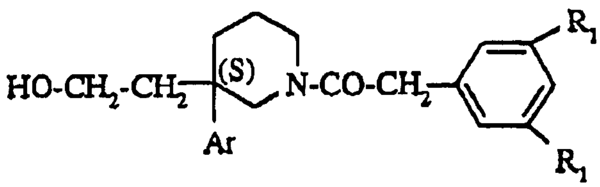 (IV*, E=H) ;

2d) the compound of formula (IV*) is oxidized to give a compound of formula:

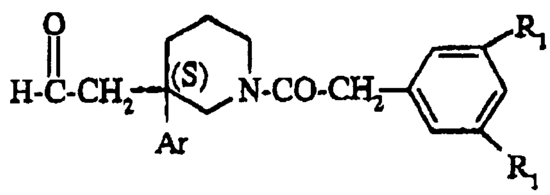 (VIII*) ;

3d) the compound of formula (VIII*) is reacted with a compound of formula:

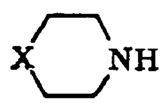 (VII) ;

in which X is as defined for a compound of formula (I) in Claim 1, in the presence of an acid, followed by reduction of the intermediate iminium salt formed by means of a reducing agent;
4d) and, optionally, the compound thus obtained is converted into one of the salts thereof with an inorganic or organic acid.

**19.** Process for preparing the compounds of formula (I) according to Claim 1, the salts thereof and the solvates and/or hydrates thereof, **characterized in that**:

a compound of formula:

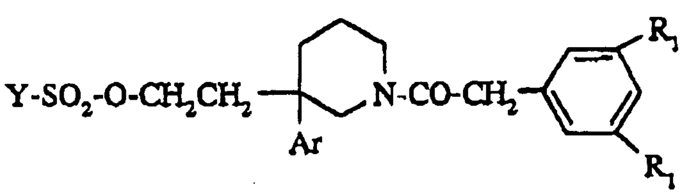 (VI) ;

in which:

- Ar represents a phenyl monosubstituted or disubstituted with a halogen atom; a $(C_1-C_3)$alkyl;

- Y represents a methyl, phenyl, tolyl or trifluoromethyl group;
- $R_1$ represents a chlorine atom, a bromine atom, a $(C_1-C_3)$alkyl or a trifluoromethyl;

is reacted with a compound of formula:

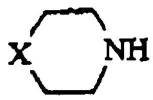
(VII)

in which X is as defined for a compound of formula (I) in Claim 1, and, optionally, the compound thus obtained is converted into one of the salts thereof with an inorganic or organic acid.

**20.** Process for preparing the compounds of formula (I) according to Claim 1, the salts thereof and the solvates and/or hydrates thereof, **characterized in that**:

the compound of formula:

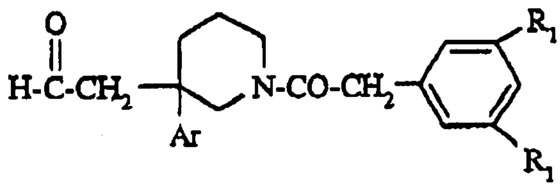
(VIII)

in which Ar and $R_1$ are as defined for a compound of formula (I) in Claim 1, is reacted with a compound of formula:

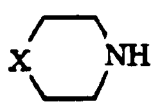
(VII)

in which X is as defined for a compound of formula (I) in Claim 1, in the presence of an acid, followed by reduction of the intermediate iminium salt formed by means of a reducing agent, and, optionally, the compound thus obtained is converted into one of the salts thereof with an inorganic or organic acid.

**21.** Stereospecific process for preparing the compounds of formula (I) according to Claim 1, having the (S) configuration, the salts thereof and the solvates and/or hydrates thereof, **characterized in that**:

the compound of formula:

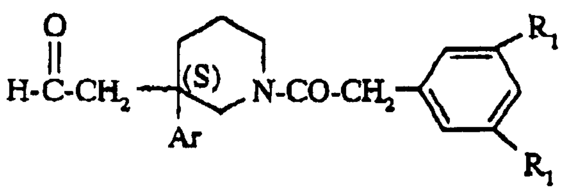
(VIII*)

in which Ar and $R_1$ are as defined for a compound of formula (I) in Claim 1, is reacted with a compound of formula:

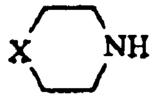 (VII)

in which X is as defined for a compound of formula (I) in Claim 1, in the presence of an acid, followed by reduction of the intermediate iminium salt formed by means of a reducing agent, and, optionally, the compound thus obtained is converted into one of the salts thereof with an inorganic or organic acid.

**22.** Compound of formula:

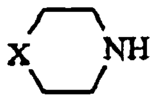 (VII)

in which:

- X represents a group

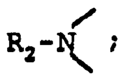

of a group

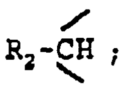

- $R_2$ represents a group $-CR_3R_4CONR_5R_6$;
- $R_3$ and $R_4$ represent the same radical chosen from a methyl, an ethyl, an n-propyl or an n-butyl;
- or $R_3$ and $R_4$, together with the carbon atom to which they are attached, constitute a ($C_3$-$C_6$) cycloalkyl;
- $R_5$ and $R_6$ each independently represent a hydrogen; a ($C_1$-$C_3$) alkyl ;
- or alternatively $R_5$ and $R_6$, together with the nitrogen atom to which they are attached, constitute a heterocyclic radical chosen from 1-azetidinyl, 1-pyrrolidinyl, 1-piperidyl, 4-morpholinyl, 4-thiomorpholinyl or perhydro-1-azepinyl;

and the salts thereof with inorganic or organic acids.

**23.** Pharmaceutical composition comprising, as active principle, a compound according to any one of Claims 1 to 15, or one of the pharmaceutically acceptable salts, solvates and/or hydrates thereof.

**24.** Pharmaceutical composition according to Claim 23, containing from 0.1 to 1000 mg of active principle, in unit dosage form, in which the active principle is mixed with at least one pharmaceutical excipient.

**25.** Use of a compound according to any one of Claims 1 to 15 or of one of the pharmaceutically acceptable salts, solvates and/or hydrates thereof, for the preparation of medicinal products intended for treating any pathology in which substance P and the human $NK_1$ receptors are involved.

**26.** Use according to Claim 25, for the preparation of medicinal products intended for treating pathologies of the respiratory, gastrointestinal, urinary, immune or cardiovascular system or the central nervous system, as well as for pain, migraine, inflammations, nausea and vomiting, and skin diseases.

**27.** Use according to Claim 26, for the preparation of medicinal products intended for treating obstructive chronic bronchitis, asthma, urinary incontinence, irritable bowel syndrome, Crohn=s disease, ulcerative colitis, depression and anxiety.

## Patentansprüche

1. Verbindung der Formel:

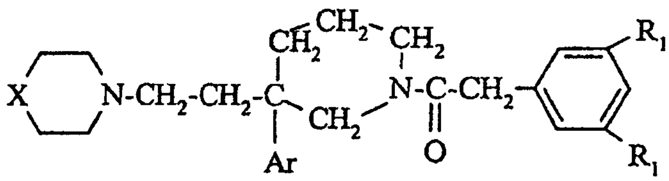

(I)

worin:

- X für eine Gruppe

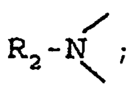

oder eine Gruppe

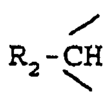

steht;
- Ar für mono- oder dihalogensubstituiertes Phenyl oder $(C_1\text{-}C_3)$ -Alkyl steht;
- $R_1$ für ein Chloratom, ein Bromatom, $(C_1\text{-}C_3)$ -Alkyl oder Trifluormethyl steht;
- $R_2$ für eine Gruppe $-CR_3R_4CONR_5R_6$ steht;
- $R_3$ und $R_4$ für den gleichen Rest, ausgewählt unter Methyl, Ethyl, n-Propyl oder n-Butyl, stehen;
- oder $R_3$ und $R_4$ gemeinsam mit dem Kohlenstoffatom, an das sie gebunden sind, $(C_3\text{-}C_6)$-Cycloalkyl bilden;
- $R_5$ und $R_6$ jeweils unabhängig voneinander für Wasserstoff oder $(C_1\text{-}C_3)$ -Alkyl stehen;
- oder $R_5$ und $R_6$ gemeinsam mit dem Stickstoffatom, an das sie gebunden sind, einen heterocyclischen Rest, ausgewählt unter Azetidin-1-yl, Pyrrolidin-1-yl, Piperidin-1-yl, Morpholin-4-yl, Thiomorpholin-4-yl oder Perhydroazepin-1-yl, bilden;

und Salze davon mit anorganischen oder organischen Säuren, Solvate davon und/oder Hydrate davon.

2. Verbindung nach Anspruch 1, worin Ar für 3,4-Dichlorphenyl oder 3,4-Dimethylphenyl steht.

3. Verbindung nach Anspruch 1, worin die Substituenten $R_1$ für ein Chloratom, Methyl, Ethyl oder Trifluormethyl stehen.

4. Verbindung nach Anspruch 1, worin X für eine Gruppe

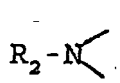

steht, worin $R_2$ für eine Gruppe $-CR_3R_4CONR_5R_6$ steht.

**5.** Verbindung nach Anspruch 4, worin $R_3$ und $R_4$ jeweils für Methyl stehen oder gemeinsam mit dem Kohlenstoffatom, an das sie gebunden sind, Cyclohexyl bilden.

**6.** Verbindung nach Anspruch 1, worin X für eine Gruppe

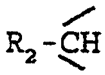

steht, worin $R_2$ für eine Gruppe $-CR_3R_4CONR_5R_6$ steht.

**7.** Verbindung nach Anspruch 6, worin $R_3$ und $R_4$ jeweils für Methyl stehen oder gemeinsam mit dem Kohlenstoffatom, an das sie gebunden sind, Cyclohexyl oder Cyclopropyl bilden.

**8.** Verbindung nach Anspruch 4 oder Anspruch 6, worin $R_5$ und $R_6$ jeweils für Wasserstoff oder Methyl stehen.

**9.** Verbindung nach Anspruch 1 der Formel:

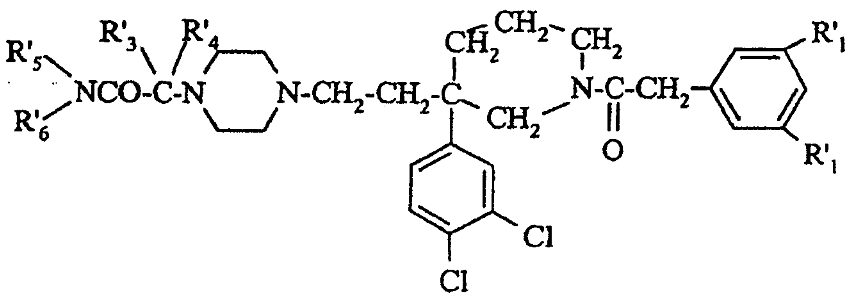

(I')

worin:

- $R'_1$ für ein Chloratom, Methyl, Ethyl oder Trifluormethyl steht;
- $R'_3$ und $R'_4$ jeweils für Methyl stehen oder gemeinsam mit dem Kohlenstoffatom, an das sie gebunden sind, Cyclohexyl bilden;
- $R'_5$ und $R'_6$ jeweils für Wasserstoff oder Methyl stehen;

und Salze davon mit anorganischen oder organischen Säuren, Solvate davon und/oder Hydrate davon.

**10.** Verbindung nach Anspruch 1 der Formel:

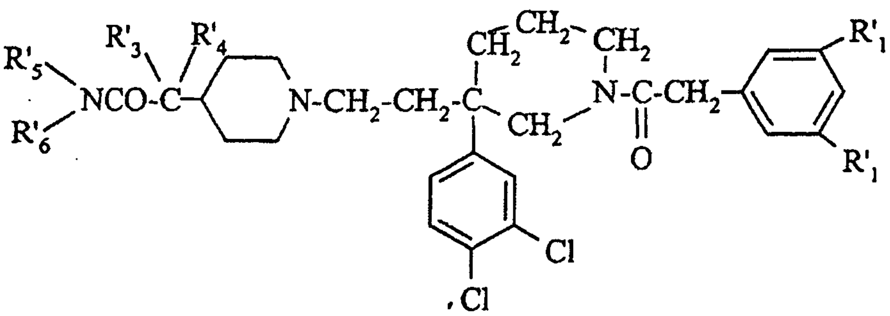

(I'')

worin:

- $R'_1$ für ein Chloratom, Methyl, Ethyl oder Trifluormethyl steht;

- $R'_3$ und $R'_4$ jeweils für Methyl stehen oder gemeinsam mit dem Kohlenstoffatom, an das sie gebunden sind, Cyclohexyl oder Cyclopropyl bilden;
- $R'_5$ und $R'_6$ jeweils für Wasserstoff oder Methyl stehen;

und Salze davon mit anorganischen oder organischen Säuren, Solvate davon und/oder Hydrate davon.

**11.** Verbindung nach einem der Ansprüche 1 bis 10 der Formel (I), (I') oder (I") in optisch reiner Form.

**12.** 3- [2- [4-(1-Carbamoyl-1-methylethyl) piperidin-1-yl] ethyl] -3- (3, 4-dichlorphenyl) -1- [2- (3, 5-dimethylphenyl)acetyl] piperidin, (-)-Isomer, Salze davon, Solvate davon und/oder Hydrate davon.

**13.** 3-[2-[4-(1-N,N-Dimethylcarbamoyl-1-methylethyl)-piperidin-1-yl]ethyl]-3-(3,4-dichlorphenyl)-1-[2-(3,5-dimethylphenyl)acetyl]piperidin, (-)-Isomer, Salze davon, Solvate davon und/oder Hydrate davon.

**14.** 3- [2- [4-(1-Carbamoyl-1-methylethyl)piperidin-1-yl] ethyl] -3- (3, 4-dichlorphenyl) -1- [2- (3, 5-diethylphenyl)acetyl] piperidin, (-)-Isomer, Salze davon, Solvate davon und/oder Hydrate davon.

**15.** 3- [2- [4-(1-Carbamoyl-1-methylethyl)piperidin-1-yl] ethyl] -3- (3, 4-dichlorphenyl) -1- [2- [3, 5-bis-(trifluormethyl) phenyl] acetyl]piperidin, (+)-Isomer, Salze davon, Solvate davon und/oder Hydrate davon.

**16.** Verfahren zur Herstellung der Verbindungen der Formel (I) nach Anspruch 1, Salzen davon, Solvaten davon und/oder Hydraten davon, **dadurch gekennzeichnet, daß** man:

1a) eine Verbindung der Formel:

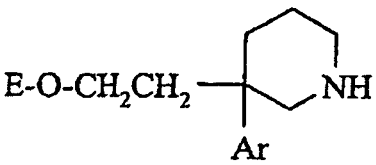

(II)

worin Ar die für eine Verbindung der Formel (I) in Anspruch 1 angegebene Bedeutung besitzt und E für Wasserstoff oder eine O-Schutzgruppe steht, mit einem funktionellen Derivat einer Säure der Formel:

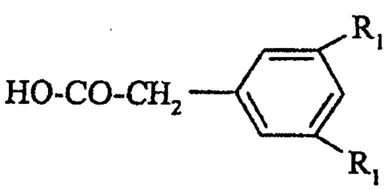

(III)

worin $R_1$ die für eine Verbindung der Formel (I) in Anspruch 1 angegebene Bedeutung besitzt, behandelt, wobei man eine Verbindung der Formel:

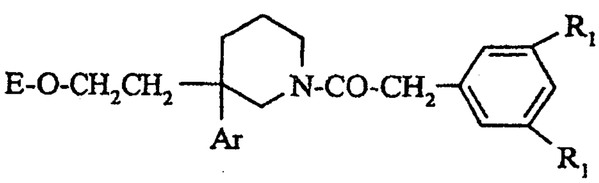

(IV)

erhält;

2a) gegebenenfalls, wenn E für eine Schutzgruppe steht, diese durch Einwirkung einer Säure oder Base abspaltet, wobei man den Alkohol der Formel:

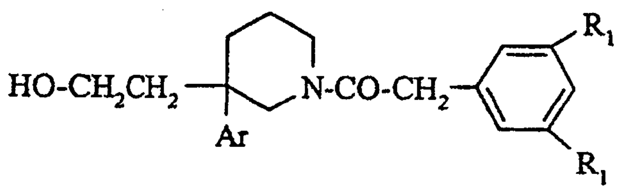
(IV, E = H)

erhält;

3a) den in Schritt 1a) bzw. Schritt 2a) erhaltenen Alkohol der Formel (IV, E = H) mit einer Verbindung der Formel:

$Y\text{-}SO_2\text{-}Cl$ (V)

worin Y für eine Methyl-, Phenyl-, Tolyl- oder Trifluormethylgruppe steht, behandelt, wobei man eine Verbindung der Formel:

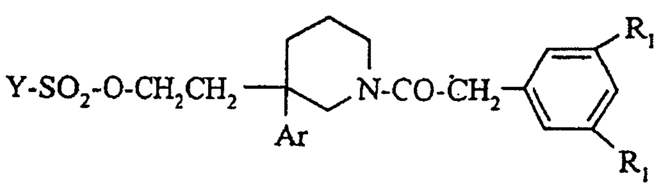
(VI)

erhält;

4a) die Verbindung der Formel (VI) mit einer Verbindung der Formel:

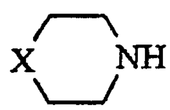
(VII)

worin X die für eine Verbindung der Formel (I) in Anspruch 1 angegebene Bedeutung besitzt, umsetzt;

5a) und gegebenenfalls die so erhaltene Verbindung in eines ihrer Salze mit einer anorganischen oder organischen Säure umwandelt.

**17.** Verfahren zur Herstellung der Verbindungen der Formel (I) nach Anspruch 1, Salzen davon, Solvaten davon und/oder Hydraten davon, **dadurch gekennzeichnet, daß** man:

1b) eine Verbindung der Formel:

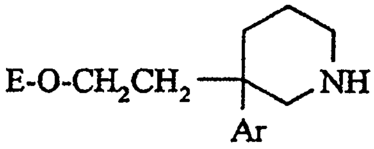
(II)

worin Ar die für eine Verbindung der Formel (I) in Anspruch 1 angegebene Bedeutung besitzt und E für Wasserstoff oder eine O-Schutzgruppe steht, mit einem funktionellen Derivat einer Säure der Formel:

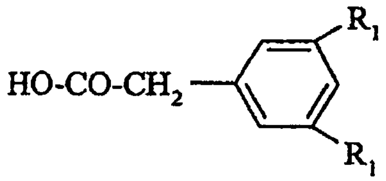 (III)

worin $R_1$ die für eine Verbindung der Formel (I) in Anspruch 1 angegebene Bedeutung besitzt, behandelt, wobei man eine Verbindung der Formel:

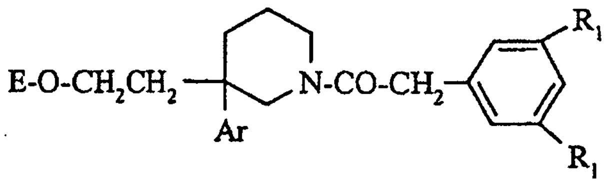 (IV)

erhält;
gegebenenfalls, wenn E für eine Schutzgruppe steht, diese durch Einwirkung einer Säure oder Base abspaltet, wobei man den Alkohol der Formel:

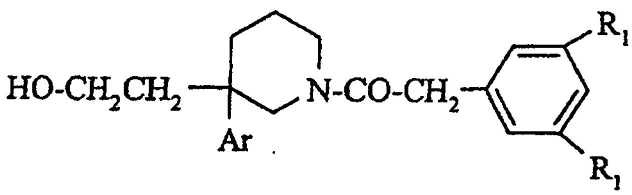 (IV, E = H)

erhält;
2b) die so erhaltene Verbindung der Formel (IV, E = H) zu einer Verbindung der Formel:

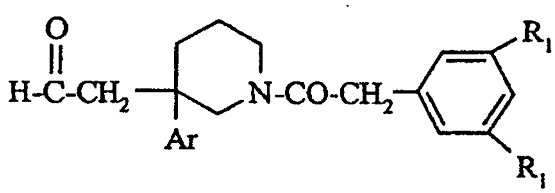 (VIII)

oxidiert;
3b) die Verbindung der Formel (VIII) in Gegenwart einer Säure mit einer Verbindung der Formel:

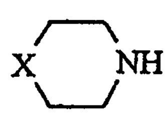 (VII)

worin X die für eine Verbindung der Formel (I) in Anspruch 1 angegebene Bedeutung besitzt, umsetzt und dann das gebildete Iminiumsalz-Zwischenprodukt mit einem Reduktionsmittel reduziert;
4b) und gegebenenfalls die so erhaltene Verbindung in eines ihrer Salze mit einer anorganischen oder organischen Säure umwandelt.

**18.** Stereospezifisches Verfahren zur Herstellung der Verbindungen der Formel (I) nach Anspruch 1 mit (S)-Konfiguration, Salzen davon, Solvaten davon und/oder Hydraten davon, **dadurch gekennzeichnet, daß** man:

1d) das (S)-Isomer einer Verbindung der Formel:

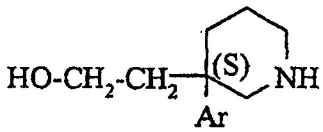 (II*, E=H)

worin Ar die für eine Verbindung der Formel (I) in Anspruch 1 angegebene Bedeutung besitzt, mit einem funktionellen Derivat der Säure der Formel:

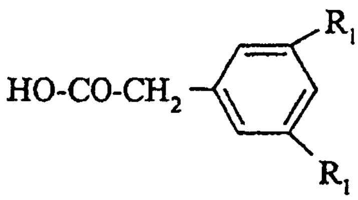 (III)

worin $R_1$ die für eine Verbindung der Formel (I) in Anspruch 1 angegebene Bedeutung besitzt, behandelt, wobei man eine Verbindung der Formel:

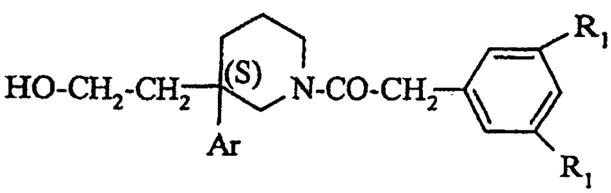 (IV*, E=H)

erhält;
2d) die so erhaltene Verbindung der Formel (IV*) zu einer Verbindung der Formel:

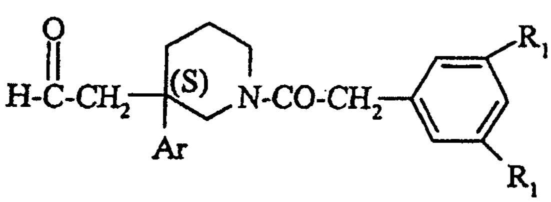 (VIII*)

oxidiert;
3d) die Verbindung der Formel (VIII*) in Gegenwart einer Säure mit einer Verbindung der Formel:

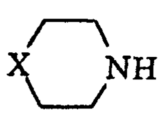 (VII)

worin X die für eine Verbindung der Formel (I) in Anspruch 1 angegebene Bedeutung besitzt, umsetzt und dann das gebildete Iminiumsalz-Zwischenprodukt mit einem Reduktionsmittel reduziert;
4d) und gegebenenfalls die so erhaltene Verbindung in eines ihrer Salze mit einer anorganischen oder organischen Säure umwandelt.

**19.** Verfahren zur Herstellung der Verbindungen der Formel (I) nach Anspruch 1, Salzen davon, Solvaten davon und/oder Hydraten davon, **dadurch gekennzeichnet, daß** man:

eine Verbindung der Formel:

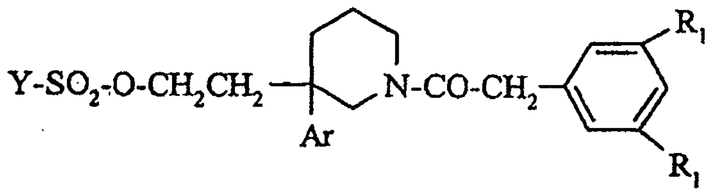

(VI)

worin:

- Ar für mono- oder dihalogensubstituiertes Phenyl oder ($C_1$-$C_3$)-Alkyl steht;
- Y für eine Methyl-, Phenyl-, Tolyl- oder Trifluormethylgruppe steht;
- $R_1$ für ein Chloratom, ein Bromatom, ($C_1$-$C_3$) -Alkyl oder Trifluormethyl steht;

mit einer Verbindung der Formel:

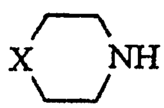

(VII)

worin X die für eine Verbindung der Formel (I) in Anspruch 1 angegebene Bedeutung besitzt, umsetzt und gegebenenfalls die so erhaltene Verbindung in eines ihrer Salze mit einer anorganischen oder organischen Säure umwandelt.

**20.** Verfahren zur Herstellung der Verbindungen der Formel (I) nach Anspruch 1, Salzen davon, Solvaten davon und/oder Hydraten davon, **dadurch gekennzeichnet, daß** man:

eine Verbindung der Formel:

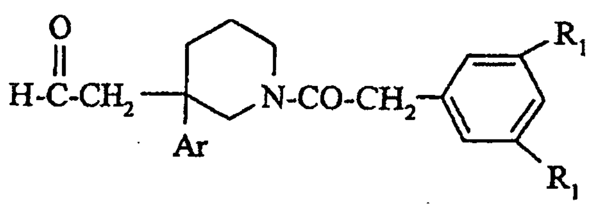

(VIII)

worin Ar und $R_1$ die für eine Verbindung der Formel (I) in Anspruch 1 angegebene Bedeutung besitzen, in Gegenwart einer Säure mit einer Verbindung der Formel:

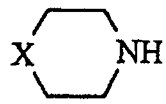 (VII)

worin X die für eine Verbindung der Formel (I) in Anspruch 1 angegebene Bedeutung besitzt, umsetzt und dann das gebildete Iminiumsalz-Zwischenprodukt mit einem Reduktionsmittel reduziert und gegebenenfalls die so erhaltene Verbindung in eines ihrer Salze mit einer anorganischen oder organischen Säure umwandelt.

**21.** Stereospezifisches Verfahren zur Herstellung der Verbindungen der Formel (I) nach Anspruch 1 mit (S)-Konfiguration, Salzen davon, Solvaten davon und/oder Hydraten davon, **dadurch gekennzeichnet, daß** man:

eine Verbindung der Formel:

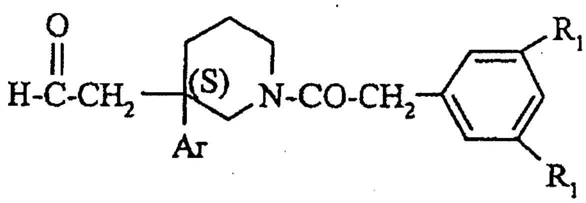 (VIII*)

worin Ar und $R_1$ die für eine Verbindung der Formel (I) in Anspruch 1 angegebene Bedeutung besitzen, in Gegenwart einer Säure mit einer Verbindung der Formel:

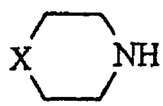 (VII)

worin X die für eine Verbindung der Formel (I) in Anspruch 1 angegebene Bedeutung besitzt, umsetzt und dann das gebildete Iminiumsalz-Zwischenprodukt mit einem Reduktionsmittel reduziert und gegebenenfalls die so erhaltene Verbindung in eines ihrer Salze mit einer anorganischen oder organischen Säure umwandelt.

**22.** Verbindung der Formel:

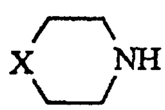 (VII)

worin:

- X für eine Gruppe

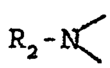

oder eine Gruppe

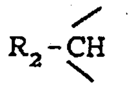

steht;
- $R_2$ für eine Gruppe $-CR_3R_4CONR_5R_6$ steht;
- $R_3$ und $R_4$ für den gleichen Rest, ausgewählt unter Methyl, Ethyl, n-Propyl oder n-Butyl, stehen;
- oder $R_3$ und $R_4$ gemeinsam mit dem Kohlenstoffatom, an das sie gebunden sind, $(C_3-C_6)$-Cycloalkyl bilden;
- $R_5$ und $R_6$ jeweils unabhängig voneinander für Wasserstoff oder $(C_1-C_3)$ -Alkyl stehen;
- oder $R_5$ und $R_6$ gemeinsam mit dem Stickstoffatom, an das sie gebunden sind, einen heterocyclischen Rest, ausgewählt unter Azetidin-1-yl, Pyrrolidin-1-yl, Piperidin-1-yl, Morpholin-4-yl, Thiomorpholin-4-yl oder Perhydroazepin-1-yl, bilden; und Salze davon mit anorganischen oder organischen Säuren.

**23.** Pharmazeutische Zusammensetzung, enthaltend als Wirkstoff eine Verbindung nach einem der Ansprüche 1 bis 15 oder eines ihrer pharmazeutisch unbedenklichen Salze, Solvate und/oder Hydrate.

**24.** Pharmazeutische Zusammensetzung nach Anspruch 23, mit 0,1 bis 1000 mg Wirkstoff in Dosierungseinheitsform, in der der Wirkstoff mit mindestens einem pharmazeutischen Hilfsstoff vermischt ist.

**25.** Verwendung einer Verbindung nach einem der Ansprüche 1 bis 15 oder eines ihrer pharmazeutisch unbedenklichen Salze, Solvate und/oder Hydrate zur Herstellung von Arzneimitteln zur Behandlung von Pathologien, an der Substanz P und humane $NK_1$-Rezeptoren beteiligt sind.

**26.** Verwendung nach Anspruch 25 zur Herstellung von Arzneimitteln zur Behandlung von Pathologien des Atmungssystems, Magen-Darm-Systems, Harnsystems, Immunsystems, Herz-Kreislauf-Systems und Zentralnervensystems sowie Schmerzen, Migräne, Entzündungen, Übelkeit und Erbrechen und Hauterkrankungen.

**27.** Verwendung nach Anspruch 26 zur Herstellung von Arzneimitteln zur Behandlung von chronisch obstruktiver Bronchitis, Asthma, Harninkontinzenz, Reizkolon, Morbus Crohn, Colitis ulcerosa, Depression und Angst.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- EP 0512901 A
- EP 0714891 A
- EP 0591040 A
- EP O714891 A
- EP 0625509 A
- EP 0612716 A


### Littérature non-brevet citée dans la description


- **L. URBAN et al.** *TINS,* 1994, vol. 17, 432-438
- **L. SEGUIN et al.** *Pain,* 1995, vol. 61, 325-343
- **S. H. BUCK.** The Tachykinin Receptors. Humana Press, 1994
- **P. HOLZER ; U. HOLZER-PETSCHE.** *Phamacol. Ther.,* 1997, vol. 73, 173-217 219263
- **J. MIZRAHI et al.** *Pharmacology,* 1982, vol. 25, 39-50
- **C. ADVENIER et al.** *Eur. Respir. J.,* 1997, vol. 10, 1892-1906
- **C. ADVENIER ; X. EMONDS-ALT.** *Pulmonary Pharmacol.,* 1996, vol. 9, 329-333
- **C. A. MAGGI.** *Progress in Neurobiology,* 1995, vol. 45, 1-98, Progress in Neurobiology
- **C. A. MAGGI et al.** *J. Autonomic Pharmacol.,* 1993, vol. 13, 23-93
- **M. OTSUKA ; K. YOSHIOKA.** *Physiol. Rev.,* 1993, vol. 73, 229-308
- **D. REGOLI et al.** *Pharmacol. Rev.,* 1994, vol. 46, 551-599
- *J. Am. Chem. Soc.,* 1941, vol. 63, 3280-3282
- *J. Med. Chem.,* 1973, vol. 16, 684-687
- *J. Org. Chem.,* 1968, vol. 33, 4288
- *J. Org. Chem.,* 1971, vol. 36 (1), 193-196
- *Eur. J. Med. Chem.,* 1990, vol. 25, 609-615
- **D. G. PAYAN et al.** *J. Immunol.,* 1984, vol. 133, 3260-3265
- **Y. TAKEDA et al.** *J. Neurochem.,* 1992, vol. 59, 740-745
- **BUELL et al.** *FEBS Letters,* 1992, vol. 299, 90-95
- *Eur. J. Pharmacol.,* 1993, vol. 250, 403-413
- *Life Sci.,* 1995, vol. 56, PL 27-32
- **R. STEINBERG et al.** *J. Neurochemistry,* 1995, vol. 65, 2543-2548
- **X. EMONDS-ALT et al.** *European Journal of Pharmacology,* 1993, vol. 250, 403-413
- **X. EMONDS-ALT et al.** *Eur. J. Pharmacol.,* 1993, vol. 250, 403-413